# EUROPEAN PATENT SPECIFICATION

(11) **EP 3 277 836 B1**
(45) Date of publication and mention of the grant of the patent: **27.02.2019**
(21) Application number: 16713460.0
(22) Date of filing: 01.04.2016
(51) Int. Cl.: C12Q 1/68

(54) **METHOD OF TREATMENT USING GENETIC PREDICTORS OF A RESPONSE TO TREATMENT WITH SSR-125543**
BEHANDLUNGSVERFAHREN UNTER VERWENDUNG VON GENETISCHEN PRÄDIKTOREN EINER REAKTION AUF EINE BEHANDLUNG MIT SSR-125543
MÉTHODE DE TRAITEMENT UTILISANT LES PRÉDICTEURS GÉNÉTIQUES D'UNE RÉPONSE À UN TRAITEMENT AVEC SSR-125543

(30) Priority: 02.04.2015 US 201562141881 P
(43) Date of publication of application: 07.02.2018
(73) Proprietor: HMNC Value GmbH, 80539 Munich (DE)
(72) Inventor: HOLSBOER, Florian, 80805 München (DE)
(74) Representative: Weickmann & Weickmann PartmbB
(86) International application number: PCT/EP2016/057230
(87) International publication number: WO 2016/156576

(56) References cited:
- WO-A1-2014/202541
- WO-A2-2013/160315
- WO-A2-2013/160317

### SEQUENCE LISTING

<110> HMNC Value GmbH
<120> METHOD OF TREATMENT USING GENETIC PREDICTORS OF A RESPONSE TO
   TREATMENT WITH CRHR1 ANTAGONISTS
<130> HMN15730PCT2
<150> US 62/141,881
   <151> 2015 04 02
<160> 548
<170> BiSSAP 1.2
<210> 1
   <211> 121
   <212> DNA
   <213> Homo sapiens
<220>
   <221> source
   <222> 1..121
   <223> /mol_type="unassigned DNA" /organism="Homo sapiens"
<220>
   <221> variation
   <222> 61
   <223> /allele="[A/G]"
<400> 1
   atgagtctcc aggactctat ggcttccttc atgtcatcgt ccactctgcc aagggattta 60
   agcaatcagc cagtaagtgc cctggccagg acgaggttgg gtgggccatt gtggattctg 120
   c 121
<210> 2
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 2
   atgaagatct acgacagaga tgaattgagg ggacaaatgt cagagctcac agacgactgt 60
   atctctgttc aggaccgctt ccacctcact gaaattcact ccctcaatgt gctggagggc 120
   a 121
<210> 3
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 3
   aacagcagga tcagaagcct atttttaatg tcattccacc aattcccgtt ggttccgaaa 60
   attggaatag gtgccaagga tctggagatg acaacttgac ttctctgggg actctgaact 120
   t 121
<210> 4
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 4
   ccaaagaatc attaactcct ggtagagaag aaaaccccag cagtgtgctt gctcaggaga 60
   agggagatgt gatggacttc tataaaaccc taagaggagg agctactgtg aaggtttctg 120
   c 121
<210> 5
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 5
   atatgtttga caatttttat ttttagctag tcatcaaagc tcttacaagt cagaatttca 60
   aacttgacca ggactatagt ttatttactg gagtgctagg agagaatgca aaagtgatgg 120
   t 121
<210> 6
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 6
   ttgatcatgc attcccaata ttcgtatatg tatttataaa ttacataatg ggcagggtgc 60
   aatggctcac acctgtaatc ccagcactgg gggaagctga ggtgggtgga tcacctgaag 120
   t 121
<210> 7
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 7
   cactggatat tctaaggagt gttttgaact aatcttgttc ccttgaagtt cctggagttt 60
   attagcagat gtaagtagta tggagtaagt tcatacctct caaaaagcac tataatttag 120
   g 121
<210> 8
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 8
   cgacgatgta taccagaata tttgttcaga ttaatatttt ccttattctg gcttattaaa     60
   atagtaacgc ctgttcttat taaggttgat tttgcctgta attagaagtc atgggcaact    120
   t                                                                    121
<210> 9
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 9
   aaacactcct tagaatatcc agtggaaagc actgggactg attttcattc gttgagcatt     60
   acccaaggta gtgtgcccta aaaagaagtg taccttatga acagaatagt agaaactatc    120
   c                                                                    121
<210> 10
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 10
   gtgcatgtgg gatttattct tctgactcag gaaagcaatt tgatgaagtg acatgtttct 60
   actaaacagc acacatcaag acacgttatg ctgcttctgt ttatcccacc tactggaagg 120
   a 121
<210> 11
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/T]"
<400> 11
   catataatat cttaatttaa caagtaaatg cagatgcctt aagattccct atcttggagt 60
   agttggctga caccttctcc agaaagcata gttaacctgc tgcatgacaa agggcaagtt 120
   a 121
<210> 12
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 12
   catcaggcag ccctccagct gaatgatttt tgtctgtgcc tggcccagtc cctgagtcca 60
   aagtggtttt taggattcac atcggttaca ggaccgggcc atggtctgcc cacctgaagc 120
   t 121
<210> 13
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 13
   taaaaaatat gtgaaaatgc attttccccc tattccttct ggaaagcaac attagggtcc     60
   agcagttctg tctggaagga gggagatgca ggagcagcat cctggcttat gaccgcgtgg    120
   c                                                                    121
<210> 14
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 14
   cagtatttaa gatgaaagga gatcagattt ggtttcggag aacagagcag atgtcgtggc     60
   atcagtaatc actagggtgt ccctttaagg atatgaggac tgtggtgagc agggatggct    120
   a                                                                    121
<210> 15
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 15
   ccggagccag ctcggcactg gaaccggcgt cctctggtgg cagagagaga gcgctactgg 60
   agattttcgg accgaatcgg cacgctcgtc agatccaagc aggcgggact ggcctggagc 120
   a 121
<210> 16
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 16
   agaggcaact gtgtctcggg aaggtaaagt gaacatctca gggtcatgta agtcggaagc 60
   aacacagcgg tgacttacac tcagatcctt actctccaga gttagtgctc ttaaccagta 120
   g 121
<210> 17
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[C/G]"
<400> 17
   ttcacagcat caagcagtcc acagcagtct gagctggcag gtcatggagc agcccccaaa 60
   cagctgtggc tggggggatg acggccaggc tccctgacca ccctgcctgt ggaggtgacc 120
   t 121
<210> 18
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 18
   cttctagttg tatttattgt taaaatgaca tcataatatt acaggaaatc cccccagcct     60
   accctcaccc tgctgtgatt ttactgatca ttatctcccc ctgttcttta ctcaggtgta    120
   t                                                                    121
<210> 19
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 19
   tgcatgaact tgggtcccag agggtcctat tatgaaagct ggatcaattg caatgggaaa     60
   agggctaacg ttattgtacc tagaatgctg aagtggtcaa ctacctaaat aaagattaca    120
   c                                                                    121
<210> 20
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 20
   aatattagaa gtttcctttg tctcccctta ttttgtcacc aggagtaaaa attaacttta 60
   agaaaaggaa atttgctggg gtcaccttgt accttgtcct ggctttgttc tcgggtgctg 120
   g 121
<210> 21
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 21
   aaggaaagaa ataacagtaa aaattcaata aaattgaaac aaatatataa gaaaatcaac 60
   aaaactaaaa gtgtttttaa aaagattaat gcagttgata agcttctagc aagactattc 120
   a 121
<210> 22
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 22
   tctgagggcg ggaaccaggt ctcctttacc ctgggatgca tgggagctca gaaatgtgga 60
   atgaagtcgt taatttacac agcacctacc gtgcacctgg agaaggtgag aacatggctg 120
   g 121
<210> 23
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 23
   caagcaacct gcctcctgct agacaattag ctttatccat gagttaccaa agagggagcc 60
   aaaacccagg gaagctgaaa gagctgttga ttgtcaccct gtgagttggt gatagaaaga 120
   t 121
<210> 24
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 24
   gattgtaccc accaaaatct ataaacaata aggaactgtg gttgtttgct gcaaataact 60
   atgataaacc acactgtttg tatcacatgt attagcccat tgtgacattg tcaattgacc 120
   a 121
<210> 25
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 25
   gtagggtatt gagtgagggg ttgttatctt cagtcaatcc atcaattaat ttgtattaga 60
   acattctgtg tgccagtcac agtacatgcc ctcatcattc ccaactcttg aggagcttag 120
   t 121
<210> 26
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/T]"
<400> 26
   attttgcagt tatctcagaa tattaactag aatatatggc tcatgagagc aggccctgtg 60
   actgccttcc tcactctcat gtcactaggg actagcacat agtaggcact caagaaacgt 120
   t 121
<210> 27
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 27
   gcgcttagtt tcctgccaca gggagacagt aagaaaggtg acgtcaatct gagatgagag 60
   agagagcaaa acagttcttt tgaccacctt gaccccgacc ttgaaataag gtggaactaa 120
   t 121
<210> 28
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 28
   acgggctttg tgcgaagttc gtcggcgctg gtgtccacag caagtgaagt gggttcagtt     60
   agtcccaggt tccaatggtt gaggcggaaa ggcaaaggta taaataccct tgatagcctt    120
   t                                                                    121
<210> 29
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 29
   ggcctctgct tcccaccaag gtgctggggg aagtgggctg ctgtggaccc ccaccccgga     60
   acactctgct ttctgcagga tcctcatgct ccccaaggac ccagagaggc tggggtgggg    120
   g                                                                    121
<210> 30
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 30
   cacaacattg atgctctctg aacactatga cctctgatta tttatcaacc tccaagagct 60
   atcactgtca ctggggacag agagcagaca aaataaaaca cctgggagtg gggtgcagaa 120
   g 121
<210> 31
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[C/G]"
<400> 31
   tgggctgggc tcccctcttc tgtgagagcc aaacagagcc cttcctgagt cccatccatt 60
   cgcagggtcc tactgttgtc cgccccctcg ttcccactgc cagctctggg ggagctgggc 120
   t 121
<210> 32
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 32
   cttgcctcta tctgggtcct tttcattgct ctacaaagaa tcctttcttc ctcccaggcc 60
   acactaagta ataacaactg gggacttttc tcacgccaac ttctgagccg cttcaagtgt 120
   c 121
<210> 33
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 33
   aggcccggcc catcaatgcc cacgctacac gaggcatact agacagtcgc tgcctaagcc     60
   aaagtcagat caccgatatt cttccaggaa aaggctcctc ttgccccctt tcccacaaga    120
   a                                                                    121
<210> 34
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 34
   tacttttgaa gtttctgtca aagaatgtca gagaatatat agttttgtgt ggctatctct     60
   atttttctta tatattatcc ctgttaatgc agggcatact gttactcttg aatgttttaa    120
   c                                                                    121
<210> 35
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 35
   tctgtgaacg tgttggcact aactgaaaat gaatgtttgc tacattatag tccattaggt 60
   atggtcatag ttgccagtgg tgagcagaat cctcccagga gtaataaatt catcagtata 120
   c 121
<210> 36
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 36
   gcaatcaata catcatgatg taatgtagtc atatagacta ggacacttag attagccccc 60
   atgacgcaag gcgtgttctg agtaacagtc tcaaattaag tggagacttt gtgatcactg 120
   c 121
<210> 37
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 37
   gttttttaaa ggttttagta ttgcaatgtg gaatccaaaa ctgttatcaa tgaacttttg 60
   attgttacat tgaaatatgt cagtctatct tgcactttga atgtatcttt tacccatgca 120
   t 121
<210> 38
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 38
   aagagcaaag ctccgtctta aaaaaaaaaa aaaaaaaaaa aagaacacag cctcccacct 60
   aatatttcct gacacggggc ctcaggatgg cactaacggt tccctcaccc agggaggtag 120
   a 121
<210> 39
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 39
   tctgttccct aaaattttga tgtacccaga aaagcatatt gtaaaaaatg ttcagatggt 60
   aagagtttta ctttctaata aagcatacag atttgtgatg gggagttcag ttcatgggca 120
   g 121
<210> 40
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 40
   gaaagtggtg agggacaggc ctggacagtg tccactgggc agagagagcc gattccgtgc 60
   agctcctggt gctgatgtgc agcgtctggg gatcccgtcg tctgttttac tctggggata 120
   t 121
<210> 41
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 41
   ttaatgtcag cacactaata ttcaaacatc cttgacctca tctcatataa ataaatccaa 60
   atgcaaatat cagtcagtca atatatgttg tatgtctagc tcccacacaa tttttatagc 120
   a 121
<210> 42
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 42
   cctgcactat ccaagattct ggatgtcttt aaggtaacaa gtgtccatgt tgttccttga 60
   aagcctggag aaatctggtg tgggaaatgt aggactcttg gtgtgggggg actgttcaag 120
   a 121
<210> 43
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 43
   tagcaatgtt cctctgtctg cacttaagcc ataagaactc tttttccttg taagcccatc 60
   agtactcaat gaaatgcctg cagagatttg gtgcatagct attttcgctt ctgctgagaa 120
   c 121
<210> 44
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 44
   ctccttcatc ctcccacaga gccttggcat gctcatagaa ttcctgaaag tgaacacaag 60
   aaagtttaga gaaaggcaag agcttgaact aatcaacaac actgtcattc aaaccctgag 120
   a 121
<210> 45
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 45
   tggatctgaa gatcttagcc aaggcaggaa agcacacgat caggtaacct ccagattcac     60
   agccctggtg ccccggttct cctgggaact ggtcctgaga tcttggacaa atccctggtt    120
   c                                                                    121
<210> 46
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 46
   aaaacatttt ttactcgcat taactctttc aatttcacaa caaatctaag aaaaatgcaa     60
   aaacaggaaa attaaaacaa atggaaacat taaaagtatc catcaatata tacaaatatt    120
   g                                                                    121
<210> 47
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 47
   taaccaaatt agtctaaaac actatcatct cctcctggat tactgcaaca gactccttct     60
   atgcttgccc ccttcggcct attcacacag tttctatagt gatcctttca aaatttcaga    120
   t                                                                    121
<210> 48
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 48
   gctttggggc tgtttttcct ggaaaaacga ctgccttcta aggccaaagg tcagtttaaa 60
   aagggctgct ggaccgccaa accccaccga acagggaata tttagggcag aaataaggac 120
   t 121
<210> 49
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 49
   taatagagca cagtatccaa gagagtagga tcttaataac cccctgaaaa agcaagcaga 60
   aatggctttc taaaagcagg agaacaagag aaatacttcc aacacgcatg gtggtcagat 120
   t 121
<210> 50
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 50
   tgcgactcaa gcccaccctg cctggcctgc accaggtgga accccatgcg cttgcctagc 60
   aaggaccaga caccgagggg ccgttggttc taggacggcg agggtcagaa ggagaggcct 120
   g 121
<210> 51
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 51
   aatcccaata aaagctctaa taccacctaa aaccatttct gttctctacc tctgtcatta 60
   atgcttaaat gaaacaaggc tgaaaatcaa ataatgcaga aatgtgcctt cgtcaataag 120
   t 121
<210> 52
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 52
   atgacacagc acaggttcta tatctttaga tggtaaatta aaaattcctg gctgaatttg 60
   attgattgtc atttttaaaa attgttaaag acttgtaaga gggaagaata ggccagacat 120
   t 121
<210> 53
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 53
   aatgatcctg ggtaacaaat gtgatatgaa tgacaaaaga caagtgtcaa aagaaagagg     60
   agagaaggta aatgtgaatg gaatggataa aggttggaat ctactcacat taagcatttc    120
   t                                                                    121
<210> 54
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 54
   aaaataataa tttctcacgc tcacttcggc agcacatata ctaaaataat aatttctccc     60
   agttctttat tattagcctc caaagagtat acctgcagca gctttaaaca acatgccact    120
   c                                                                    121
<210> 55
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 55
   tgctatttta cattgggtga tcaggaaatg ctcctaggag gaggtggtat ttgagcaggg 60
   atatgggtga agtaagagag gtcatgcaag ggctagaaga agactactcc aggcagagga 120
   g 121
<210> 56
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 56
   aaaatctggc aagatagcct atacaacatg gtgagaccct gtctctacaa aaattaaaaa 60
   atatatatat agccgaacat agtggctata ttggagtgca gcaggtggaa ggagtgcttg 120
   a 121
<210> 57
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 57
   agttctccag aggacctgcc tacctccaga cggctcactc actcccactc attctgtata 60
   aactgctagt gaagcctttc tgacacagca cacccaccac attactctct aattcatgga 120
   c 121
<210> 58
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 58
   ttaagacctc agaaaactct tgttaaaatg gaaatctatt ccctaaaaga gattatacac     60
   atatccatta tggacattca catgctagca gtgattcatt gatcaaatta gttgtcactt    120
   t                                                                    121
<210> 59
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 59
   gtttatttgt tgattttatc tgtggaagat cagtccaacg tttaaagtgg ggtgttgaag     60
   actccagcta ctattttatt aggggcttat ctctatgttt acctctaata atattttctt    120
   t                                                                    121
<210> 60
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 60
   tttgctaaga agtgttaatt ctctaagagg aaaatgtcat ttctccaaaa caaaacttta 60
   agcaggtgat tttttttaaa agccctgtca ggttgacaag tgctataaga taataaacct 120
   t 121
<210> 61
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 61
   tccaaatcac gtaacaaggt tacctccaga aaaaaaggct attgctgaac agaggctttc 60
   atttttactt ttattcccca gaattttttg aatgctttag aacattgatt ctcaaaccgt 120
   a 121
<210> 62
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 62
   gagggaagct ggctctcttt gaatggaaat ttaaccagaa gttaaaataa attccattca 60
   atcgtataga atagttttgt tccttttcac ttaaaaatat ttttctctct tttatgtgcc 120
   t 121
<210> 63
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 63
   ctcgaggagg ggctcacacc gagatcaatc catgatgaca gcacttcatg gcccgtctca     60
   aacacacagg cccactccct ggtctggccc aggctggggt gctcagggcc tctgtgttgt    120
   t                                                                    121
<210> 64
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 64
   aaaatgatgg gataatctaa ttcatctaac ttgctttaca aatgaggaaa ctgataatcc     60
   aaaagattta atctcatagg aaccaggtga cagagcagga aataggccac tggtctcctg    120
   c                                                                    121
<210> 65
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 65
   catctgcaga tttaacgatt tcattgaaaa aaaaatcctc cagatcaggt attttagaat 60
   attaaataac accaatcctg aggcccgtct gtaaccactc aaagggtcca ccttgcccac 120
   t 121
<210> 66
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 66
   cttggggatt ccactgggct atgtgtccat ttatttattc attcaataaa tatttactga 60
   atgtccacca ggccctatag ataccatggg aaacagacag tggcccctgt tctcaagtgg 120
   c 121
<210> 67
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 67
   gaaggactaa aggggtcaag atacaaggag tcaccaaaga atgcagaaga gacaagttca 60
   agaagactac cacatacgta ttggttaccc agagagaacc tgaaaacagc agcaccattg 120
   g 121
<210> 68
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 68
   tggttcaggt ggctctggat aaggtcagtg aggcttagtt caaaccaacc tgatttataa 60
   acataagaac attctactac taattcttgt taatattggt cttagaaaag gaaatttctg 120
   a 121
<210> 69
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 69
   tcaggaggtc tgttgctaat cccaaccagc atgatttacg ggaagtaaat catctatgac 60
   atgcccaaag agaataaaag tacatacagg atgcttctac ttagggcttt tttggtagag 120
   a 121
<210> 70
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 70
   atcctgatca gcctgtctca caaacattgg gttctataga cgctcctaga ttgcattttc 60
   atttaagctg agccttgatg gtctgctgga atatggtagg ctacacttta cacacacaag 120
   g 121
<210> 71
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 71
   tccttttgtt cccagtgcct tgacagggta tggggggacc tgcatgacta gcattaaatg 60
   aaggactggg ctttgccaga atgaagaaat cctctgagaa tgtgcagtag agcaaaacaa 120
   g 121
<210> 72
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 72
   tggacattta taaccaggga tctgtgcgtt ttgctataat tcagaaagta gcagactact 60
   agacacgtgt catttggcaa gggattttaa gagcacatag tatacttaga ataatcatgc 120
   t 121
<210> 73
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 73
   gagagggagg aaaagtcggt tcgagaaccc aggtggaaaa tagattgagg gaagcaaaac 60
   aagatgttac aggaggaata tgggtgattg tcttttcctt ttatatttct gcatgttttg 120
   t 121
<210> 74
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 74
   taaaaaataa ttttaaagca gtgtggtctc aatcttagta gaagagtaga aagcaagata 60
   atttctactt ggaataaaca agtgcacagt ggaagtgatt aactcttact ctcaatgtta 120
   t 121
<210> 75
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 75
   aaatagaggg ctctggacat cttcagaggg tcccacttta gacttcactg atctcttttt 60
   aacatttttt atcaatacat aatatttgta cattttatgg ggtatttgtg atattttgct 120
   a 121
<210> 76
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 76
   cctaatctca ttcacaaata tatctgaata aaaatggtaa atccaaagac aacaacatca 60
   ataactatct tagctatatc ccttactgga aataataaat gtaaagtgtg aaagaatcaa 120
   t 121
<210> 77
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 77
   cctcgcctct ctcttctgat ttatctggct cttgcctctc cccctccatc aaaagaccac 60
   actatctctc tcctcttttc catttgaacg attttgccat tcatcaaact gattgctaat 120
   g 121
<210> 78
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 78
   cttcagagac ttcgtaatta aaggaacaga gtgagagaca tcatcaagtg gagagaaatc     60
   atagtttaaa ctgcattata aattttataa cagaattaaa gtagatttta aaagataaaa    120
   t                                                                    121
<210> 79
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 79
   ctggtatggg agccagggtt gaagtcactc acgggtcctc tccgagaact cgagtggtga     60
   aatggagagc cggggcctgc ccttgtccct gcagcaggac tggggaggag ggggtgcctg    120
   a                                                                    121
<210> 80
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 80
   gtctgtgtga cagagcaaga ccctgtctct taaaaaaaaa aaaagtgatg tagccatttc 60
   ataaagacag ttgggcaata actatcaaat ttaaaatgca tatcaccttt gcacttccag 120
   g 121
<210> 81
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 81
   accacacggt ccctcaggct gcttgttacc gtggaagctt cctgaactct ctccagaccc 60
   acagacctcc cttcttgggg gctgccgctg aggagcttct ggctagtgag ctctgaagca 120
   c 121
<210> 82
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 82
   aagggaaatg ttttcatttt tctcttccca acccaatccc ctctctctaa atcttggtat 60
   aggtgaggtg ctaacagaca gtgaaacaag aaagtggttg gagtcattcc aaaaggggaa 120
   c 121
<210> 83
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[C/G]"
<400> 83
   tatgagttct gtaagaacag gtactggggt caggcttttc accactgagt ccccctagaa     60
   cccagcatgg tgccttgaac acagaaggtg ttcagaaaac atatattgaa tgatgaacga    120
   a                                                                    121
<210> 84
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 84
   ggaagaccca tttgcttcct ttccccaatt ctaccaacac atttattgag cacttactat     60
   ataactggca attgagatga aagtacacat aacaaggtga acatgcaagg ggtccaccag    120
   t                                                                    121
<210> 85
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[C/G]"
<400> 85
   ggcatcttta gaccaaagaa tctactgcac ttcaaattct tcatgtgtgt aatgagaata 60
   ctgatggcac tacctttgta gtgtttggga atgatttggg gagatacttt tttgtgtaaa 120
   g 121
<210> 86
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 86
   tcctggactt tgccatgtcc ttctaagtga cccgagcact tccagtctca tttgggcagc 60
   atccttcccg aattccattc tgtacacttc aagcaaatta gttttagagc atagctctga 120
   t 121
<210> 87
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 87
   aaaaaaaaaa aaaaaaaaag aatagaatag aatagagagt ttggaaaaag atacacataa 60
   atatgcttga ttcattttgc taatgcaaag acaatttcat ggaaaagtga taacctttaa 120
   g 121
<210> 88
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 88
   ttgttgagtg ttggtgatgc tgatagttgg agatacccag acagataagg tatattgccc 60
   actttcaaaa cttggctgcg ttagttacat ccctatcgat gcaattttct tttctttttg 120
   a 121
<210> 89
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 89
   ttcttctaag actgctcttc ctggcttgca gatggccgcc ttcatgctgg gtctttacac 60
   aacctttttc actgtgctac cacattcctg aaggacacca gtcatattga ttagagtccc 120
   a 121
<210> 90
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 90
   cgattttgga gcagtagggg actggctgcc gagggggcat ctagattgag ataggtggga 60
   aggcaggaca agacccctaa gctcactgcc tcctcgattc cagtcgtcag actccataag 120
   t 121
<210> 91
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 91
   tacacttaca aaagaataga gaatcggcta taaatttgct gagtcagaac attatactgg 60
   acatccactt tctcacttct ttgtttttcc agaatgagca cttttgccaa tcccggtttg 120
   t 121
<210> 92
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 92
   caagaccggc ctgggcaaca tatcaaggac ccatctctac aaaattgaaa aaaaaaaaaa 60
   agggggaagc aggaaaaggt gatcatggtg gaccacacaa agctttagaa tgaattcttt 120
   t 121
<210> 93
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 93
   gactgaggaa gctccatttt ctttgaggta catcaacatc aataacagat caatggaccc     60
   acttaatgga gctcttaatt gagtagaaaa aaatatttaa gagttttgcc gctctacggc    120
   a                                                                    121
<210> 94
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 94
   tgcggcctgc agcctgacct catggcttag ctgtgcctcc tggacaccat ccctctctgc     60
   aatggcgtgt ggtcctgagt cactgacagc actgacccgc tcctctgagc accagccctg    120
   t                                                                    121
<210> 95
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 95
   ttatatggtg gtaaggtgtt ggggaggggg aggggaattg ttttttaatc tttatgatta 60
   aatctcagtt ttttttagtg ggtctgaatc cctgggctgt gactttcaga aatgagacaa 120
   g 121
<210> 96
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 96
   gaggcattat ttggtgagaa tcaccattta aaaatgcaaa atattgtgtc actggcttaa 60
   actgcagatt cctaggccag aggcaatcaa tacatcatga tgtaatgtag tcatatagac 120
   t 121
<210> 97
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 97
   cacccctgac ccactcatat gtctgttctc actcagaggt gaggccctgt gtcttcagcc 60
   atggtaaact caggacctct ggacaggcag gcccagggtg taggcaccat gacttttcct 120
   g 121
<210> 98
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 98
   ctgggggtta gggggacaga gaagtaacgt cacaagattt taagcttggg ccagatatgg 60
   aaaataattt aatcctagat cacattttac acatgaataa ctgagaacag aaagaagtga 120
   t 121
<210> 99
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 99
   atagggttga aagtagagca gaaagggcaa gcagagaact agacagagaa gacagatgac 60
   agaggagagg aggggaatga ctgccagggc caggtcccag gagagtggga aggtattatt 120
   a 121
<210> 100
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/T]"
<400> 100
   tatgtcattt gtaaaatttt aatcataagg tacaatttcc ttgaggcttc ttcacaatga     60
   acattgagcc catggtgata tccccagtct tcttgcccta gaggcagcca catatgctta    120
   t                                                                     121
<210> 101
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 101
   aaaaaaaaaa aaaaaaaaaa aaagaacaca gcctcccacc tcatatttcc tgacacgggg     60
   actcaggatg gcactaacgg ttccctcacc cagggaggta gaaggacttg gacacaagac    120
   g                                                                     121
<210> 102
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 102
   caacacactt aatcttgggg aatctgagtt tattagagga atgtagggag gaagcaggct     60
   acatgccctc ccagcttaga tttagattta gccagaagaa tgtctgcact tctttgctag    120
   a                                                                     121
<210> 103
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 103
   agcaatcctc ccacctcagc ttcccaaagt gctgagatta caggcgtgag ccactgcacc 60
   aggcccatct tcctttagac tgtcttgatg aagtcactag agcatatgat aaaaggagag 120
   a 121
<210> 104
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 104
   aaggaaataa agaacatgac agaaaaaccg tctatcattt taaagaatat atatatatat 60
   ataatcatta ggagaatatt catagaaata aaaacattaa aggtgtttct ggtgagatct 120
   c 121
<210> 105
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 105
   agaagtaatt tgagtatctt ttccttgttt ttctcttttg tccagcttat atttatccac 60
   aattttataa atctggctca gcaaagcatg ttggaaggga tctcatttta aacaattctg 120
   t 121
<210> 106
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 106
   ggtcgtcttc tagtacagta agggcaaagg gcactgcaat tgctattaaa ctgtaagaag 60
   aaggaaaaaa tggacagatt tcgtagccta gtccatcaaa atcattactt tgtagttgat 120
   a 121
<210> 107
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 107
   tctggaaggg atcccccgga actgggggaa tttccaggca catgaggctc tgtcaaccca 60
   accaggaaca tccgcccctg ccatctgctc cagacgtcat tgcagagtct gtgtgagagg 120
   a 121
<210> 108
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 108
   aaaaaaaagt atgaatgaaa gtagatttta agtatgccat gttagataaa taatacgtac     60
   atattttggc actaaatgaa taactgctgg aaaaatttat tttaagtggc ttttaaaatg    120
   c                                                                     121
<210> 109
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 109
   ttaaaattag tagttttaca taaaaatctg aatgtctggc ttttcttgga aaattggaaa     60
   atctggccat gccagacctc atttttaaat ggcaattgca tgtgcccctg caagcaggga    120
   t                                                                     121
<210> 110
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 110
   ataggtgtgt gtaccacagc tcccagctgc atgtacttta aaaatgtgtc taagccaggc 60
   atggtggctc acgcccgtaa tcccagcact ttgggaggcg gaggcgggtg gatcacctga 120
   g 121
<210> 111
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 111
   tgcaataatg tgcaaacaga aaaatcagaa cctgctcatg ctgccatatt aataggaacc 60
   atcagtcagc cagagaggga ctcacatatc agacttacat attactaaac tattttctgt 120
   g 121
<210> 112
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 112
   cagagcttgg tatctgagcc tggggccctt tgagccagct gtgttggggg aggtggaggc 60
   aggaagttgt aaggtttgag actttgagag ggagccttga gtgtgtagtt actaagggaa 120
   a 121
<210> 113
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[C/G]"
<400> 113
   gaaactggga taatacagcc atgcgctacc tactggcatt cccgtcagtg cgtacacgat     60
   catggtccca gactgcaatt tttttttttt ttttttgaga cagagtctca ctctgtcacc    120
   c                                                                    121
<210> 114
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 114
   tccttaactt aactgctttc ctcattggct tggtctccat agtgattcat tttgctgtaa     60
   aaagtagaca attatagaca attatgaaaa atatgaatac tgtggtctct gagtctgaat    120
   t                                                                    121
<210> 115
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 115
   aaattagtat actacctaac ctgggaaata aattaaaaac tgtgatttga ttttcataat     60
   aaagaataga aactatcctt taggtcattt ctaattacaa aaaaatttcc attcaaatca    120
   t                                                                    121
<210> 116
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 116
   atatgaatga aagaataaaa ctcatcttaa ttttcagaga cttatctaca tagaaaaaat     60
   aaagtatttt agaattaaca agattggaag attgctgaca taaaaatcaa tttttagtag    120
   t                                                                    121
<210> 117
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 117
   gggtggtgag gcctaagctg aacctgagag gtgaggaaaa cagaccaagc tgaccaaacc     60
   actccaggcc cttcctccac tcacagggat gctcctcccg tggtgccttc ctctagacac    120
   t                                                                    121
<210> 118
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 118
   tgcacaccat gcgaactgtg gagtatctca gtaagagtgt taggaggaat attttatagg     60
   acttgtgctt gtattaggtg attttgggga gtttaagaaa gcagagcttt ctcgattgga    120
   t                                                                    121
<210> 119
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 119
   tctaattgac ttttattagg gatttatgaa tcaggcagta ttccatctag gaaatgtcta     60
   aaaaggtgct ccaccccatt ggcagaacag ttgtttgaga tttgttgttg ttattttgct    120
   t                                                                    121
<210> 120
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 120
   aatgggaagc agcagggtgt gatgtggacc ctggattgta tgtattccct ctcttagggc 60
   atggctgctt tttatttgca gctttaccat tgccatgctg gaaaatcatc acatatttca 120
   a 121
<210> 121
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/T]"
<400> 121
   ggcagtccta aaggaaggtc accctgggat catctcacct ttgacgaggc tggccaggga 60
   agctgctgag aatgaaataa actcttctct cttttgcttg gagaaaagaa atcatgggta 120
   g 121
<210> 122
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 122
   ctgggctaga ggcaaaagca gagatgtgag ctgtaaattt gaatgaagga ccagatagaa 60
   agtagaaagt ggaaaatgga acctagagct ttggacaggg ctcaaaggaa aacaagcatt 120
   t 121
<210> 123
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 123
   ttgaaatatg attctatatt taataggaaa aggaaacagc agcctattaa aaatgtatca     60
   aaacaataac tttttattag tcctactaac atctgaactt ttatgttcct acctacaagt    120
   c                                                                    121
<210> 124
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 124
   ccactgtggg ggacggcaga ctgatgggaa cattggttga gtgaccacaa gtgctgttga     60
   aagttttttg gtaacagttt taagtgtttt ggttaagcta gacctgaaaa aaatggtata    120
   g                                                                    121
<210> 125
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 125
   ataaatttgt caaataaata aactttaaag aaatggccaa cttgggaagg acattaggcc 60
   atcagtttgt agtcttacgt caattcttga tctccaagca aaattagttt cagttctctg 120
   a 121
<210> 126
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 126
   agggagggga gaccaagggc tctgagcagc ccccaaagct ccttgtccct cagggtggct 60
   atgtggggag cggcctacct ctgagatctt ctggaactgg ttgttggact ggctgcactt 120
   c 121
<210> 127
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 127
   attagaatac tttactctac ttaattaatc aatcatattt agtttgactc accttcccag 60
   aaccttctag ttctttctta tctttcagtg cttgtccaga caacattttc atttcaacaa 120
   c 121
<210> 128
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 128
   ccagattcat gaagaaccct gtatcattga tatcacctag accaccacaa aacaaacata     60
   acatttatgt ctctttagtc tccattaaaa ataaacatgt aaaaatgaat caaactcatt    120
   t                                                                    121
<210> 129
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 129
   aatgaactta cattctacct gcctccctgt atattttgct ttggttctaa ttattgttaa     60
   atgaatcaca acatgtgata tacctctcag ttacttccaa ttgaatcaag agtttttctg    120
   a                                                                    121
<210> 130
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 130
   tgataaactg tgctccataa cacaaataat ttcattcttc ttcctttctt gccgagtagt 60
   aaaaaaaaga ggatggctgg tttatctcaa gtaatcagac atttaataat aatatagaaa 120
   a 121
<210> 131
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 131
   ccaacagctc atgagcaagg aggccaaaac cctgcgtgga cggtctgctt ccctgccctt 60
   accccccgac ctttattttt tttttgagac gaagtctcgc tctgtcacct aggctgaagt 120
   a 121
<210> 132
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 132
   agagactgcc cactcttgtt aacttcctgg gtttgttttg attccatcaa gggagtagca 60
   atgtctcata cttttgtctt ccccacgggg aagggcacat atttggcact caatacatgt 120
   a 121
<210> 133
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 133
   ctttctattc tatttttagc agcctatgga ttctaggagt gacccagctc cagggatagg     60
   acttgattaa tctaaattta gagaatggat ttagattaat ccaatcttgg taattccccg    120
   t                                                                    121
<210> 134
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 134
   cagaggctgg atgaagatgt acgcaagctc tttcctcctg agacccagtg agggaggcaa     60
   aggaggctcc ctagctaaag agggagctca aagttgcagc ctttcctcat gcaaggcaag    120
   g                                                                    121
<210> 135
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 135
   gctgtggtaa agcattaatg aagcacaggg cctatcacgc agtcaggctc agtataaggt 60
   aaggtgtttt ttttttaatc caggtaacat aagaagcacc tgttagcatg agttccatac 120
   a 121
<210> 136
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 136
   tggggtgggt catactcaaa ttgatacaca gcctttgtcc tgagtgtttg tcttccaaaa 60
   aaatctcttt gcttagagat ctcagaaaat atttgctgtg ttaggggcag attcctggat 120
   t 121
<210> 137
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 137
   ccttattcat agagtagtat tgcttaaaaa ctgctccaac cacttcttaa acctgaaacc 60
   atagacagaa acatctccta agactgataa atcctaagct ttatgctgtt agagactggg 120
   t 121
<210> 138
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 138
   ctgcttgtat tacctgaaca gttctttgtg ttttgattct attgtgttct gtgttgctga 60
   atagcagttg gtaagcaatg gcatgtcatc cttgtccctg attttggccg aatgaggaca 120
   t 121
<210> 139
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 139
   tcttacagta aatccccact tatcaaatct tcagatgtgt agagaaggaa taaggcaggg 60
   ataatggggg agtgggacag agagatgccc tttctggagt ttgcacaacg gttgcatgct 120
   g 121
<210> 140
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 140
   gtcttcgtga atctgcgtaa attgctgcat ctctcttggc ctcagttttc ttagccacac 60
   agacaggact gaactaaatg atctctaaag tacttctcaa gtctataatt ctatgattct 120
   c 121
<210> 141
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 141
   agaggattag aatgacttgc tcctcacaat ttccctgcgt ctgtaactgc acccatgtag 60
   acctcatcac ctagagcctt agcctcctaa ataatagtag ctggcactta ctgagaatgc 120
   t 121
<210> 142
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 142
   agtgattata ttttccatta tcccatatat atgtaatggt atgtaatttg tatcattctc 60
   atttcatagg agagttattt cattacacaa caagaatgcc atagggtggc atttctgaaa 120
   g 121
<210> 143
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 143
   gaggccgtgt gaatgcttgg gagaagcgcg ctttcggcca ggggtctgga atgcttgcac     60
   agggttcttc tctataaaca gtgcagacca gggcctcctg ggcaagcgca gggggtgggc    120
   g                                                                    121
<210> 144
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 144
   tattgtggca ataacttctg ctgaaaaact aacctgttct actgagaatt ctatcaatgt     60
   aaatagataa acagatgctg gattacacat atctgtacag aactttccta atgctctatc    120
   t                                                                    121
<210> 145
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 145
   tctaatggga agagagaaac aaggagagag agaaaaacaa acaggcaaat tggagaaaca 60
   atgcatacaa agtagacatt tccaccgtgc gctgcagttt tttccatcat tatttgtttg 120
   g 121
<210> 146
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 146
   acatctaagc ccactccagc cggcccccag aggtgggagg gtccgccacc tcccacagcg 60
   agcacctggg ttaccatagg tgcagttaca gcagaagcga ataatgagga gaatctccat 120
   g 121
<210> 147
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 147
   ccataatttg tattagcaca ttaaagaccc cgagaggttc tgcaaaagga aactagttgt 60
   accaacttgg tacaactcag catttccaaa atatttggtt acagagcact ttttgcatgt 120
   g 121
<210> 148
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 148
   tcagggtgga ttttgaaatt tccattatat gcaaagccca tgaaaggcta aatatcagtt 60
   aagaggggag aggagggtgg ctcctaggtc ctctaatggg caggaaagta tttaaaacaa 120
   c 121
<210> 149
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 149
   tgagattagt gtttgtaaat gcacactgtt gggggaaccc tcttcctagt ccttgtttcc 60
   atgtttccca ggaatgaaca ctagtggagc agcacttccc atttcccccc actctttact 120
   c 121
<210> 150
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 150
   taccacctct tcagcaccct ctcgcatccc cacccgtcca gcagcagcac aaaggggccc 60
   aaaggtgcag cattagggaa tctaatggcc tgaggaataa gttctcgccc actgtgactc 120
   c 121
<210> 151
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 151
   cccttcccaa gccatgggca aaaacagctc aggtagtaat gaaggtgtgg ctatagctga 60
   acaattggat ttaaatccca cagagccatg gtgctgggaa gaggggctgc cctggccagt 120
   c 121
<210> 152
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 152
   aagtggaatt acatcaaact aaattacatc atcagagtaa agagacaatt tacaaaaagg 60
   aaagaaatat ttaaaaacca cacatcggat aaggggctaa tttccaaaat atatgaggaa 120
   c 121
<210> 153
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 153
   ctccagcgcg cctgaggctc atgcatttgg ctaatgagct gcggtttctc ttcaggtcgg      60
   aatggatctt gaaggggacc gcaatggagg agcaaagaag aagaactttt ttaaactgaa     120
   c                                                                     121
<210> 154
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 154
   taagacaaag ctggctccag gcaaagaata ctaccagcaa caaagaggaa catttcagat      60
   aataaaagag acaattcatt gggtggatca caagctcagg agttcgagac cagcctggcc     120
   a                                                                     121
<210> 155
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/T]"
<400> 155
   tctgcctaga aggactagcc tgctgcttca tttcccccct cctctgcagc cgatttcaga 60
   aggctgcagc agagaaagcg agacccccac accttgtttg tgtgtaccct tccttccgca 120
   c 121
<210> 156
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 156
   taaaaagcaa atattagtaa cctggaaaac atacatggag gtatgttcat taacggcagt 60
   aaaaaaccaa accaaatttt agagatgagc ggtaccttag aagatttagt caggggaaaa 120
   g 121
<210> 157
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 157
   caggaacatc cagctgcctg catgactttt ctaagtgtct aaaaagcatc ttaaacttaa 60
   attcttgatt ccctctcctt tactccacga caaaaatcca gctcttccca ttgtcttctc 120
   t 121
<210> 158
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 158
   tttatgcttg aaaatcatag aaattgtgtc taaggatatg ctttgggata tttggacttc     60
   acttttgttt tagtttttag ttagctgttg agtttaaagt aatttagtgc tctgatattt    120
   g                                                                     121
<210> 159
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 159
   tgcgggtaca ccggcaggca ggaaaaccca ggcttctctc cacatggtgt ttacgtcgtg     60
   aggggagaga gactagggac gcacgagtag agaagatccc tttggtttat gttaagtgca    120
   g                                                                     121
<210> 160
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 160
   tgattcctag gctgcctgta ctagtgatag tgaggctcac taccatccac cacctaaatt 60
   agaaccgctt gatgacacag cacaggttct atatctttag atggtaaatt aaaaattcct 120
   g 121
<210> 161
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 161
   acaagtcggg gtgtagctta cgggagggaa gtcaaagtca ggcacgttca tcacactcag 60
   aatgtagtcc actctgaact ggttctcggg gttggccagc tccacggggg gcaccaggtt 120
   g 121
<210> 162
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 162
   gtagtagatg ctcattgtaa gattcaaaaa cattccagct tacaaaacat atccagctta 60
   aattttaact catggtcttt agcaagtata gattcctcaa gtgaaagggc attgaggcag 120
   a 121
<210> 163
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 163
   gcaatgctag aaatatgggg attaaaataa tgggaaaatc agttttagtg taatacaagg     60
   aaaaacatta aacatgaagc tgtccagcag tagaacaaat tgccttgcaa agagctgcaa    120
   a                                                                    121
<210> 164
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 164
   gcccaagatt ctatatttga acaagcttct gggtaatatt tatgacaggg aagtcttgag     60
   aaaatttgga ctataggtcg tcttttaagg ttcttgccaa ctctaagact gccatcccat    120
   a                                                                    121
<210> 165
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 165
   agctacctca gagtactttg tcttttaatg ggattataat agaatctcat gaccttgtta 60
   aacttaaata agtcaataaa tggaacattt caaacagtgc ctggttcaca gtggtattat 120
   c 121
<210> 166
   <211> 120
   <212> DNA
   <213> HOMO SAPIENS
<220>
   <221> source
   <222> 1..120
   <223> /mol_type="unassigned DNA" /organism="HOMO SAPIENS"
<220>
   <221> variation
   <222> 61
   <223> /allele="[-/CACTTACCTTCTTTGTGCCACAGTTTCCCTATCTAAAACACAAGGTTATCAGTTATCAACATCTCTTGGGATTGTGAGGACTAAAGTAATGCACATAAAG]"
<400> 166
   ctttgttaaa tgttttttct gcatctattg agctgatcat atgctttctc ttgctaatgt 60
   ggctttgtac agtgcctgtt acatggtata ctttcaacat tagtagtagt agatgttgta 120
<210> 167
   <211> 120
   <212> DNA
   <213> HOMO SAPIENS
<220>
   <221> source
   <222> 1..120
   <223> /mol_type="unassigned DNA" /organism="HOMO SAPIENS"
<220>
   <221> variation
   <222> 61
   <223> /allele="[-/AAATTACCCTGTTAGGTTTCAATGAAACACCTTTTCTCTTGTAACAAACATCTCCTCCAAGCTAGAATTTCAAAACAG]"
<400> 167
   tttttctaag tttatgtctt aacctaacaa taactcaaaa gagaaacaag tatctctcca 60
   tgttaccatc cactaggtaa taatttttat gctagcaaca aaacccaaaa tatgtgttca 120
<210> 168
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 168
   ttggttatga tttttttttc atttgaagta aatatccacc tttgtatcta attttgcatt     60
   aaaaaaaaaa tttttttttt ttactttaag ttgaatccct acaattgtat aaccttcagg    120
   t                                                                    121
<210> 169
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 169
   ccagctagtt ttatttttta atagtgttct tgcacatgag gagaaagact gaattcaatt     60
   acactattct ataactaatt ataagttata ataaaaatga aacaaaaaca tttcaactga    120
   t                                                                    121
<210> 170
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 170
   aagaaggtgg cgtgtcactt cgtttgactt cagctgggaa catgcatatc agtcgactca 60
   aattttttgc tattctgtgc ttatccacga atcgatagga aagcaagtgt ggatttgggg 120
   g 121
<210> 171
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 171
   aatgacacag atcgtcacac agttttaaga caaatgtttt tacctatttg acctagtctg 60
   acaatcccta tttgggcaaa aatcttcatt tgcaggtcat gattggaggc aggcacagaa 120
   a 121
<210> 172
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 172
   aattctttga tgtgctacaa acctgaaact ggtaagacaa gcacaaagca acgtgcaata 60
   aaaaaatcgt atctcaaggg aaaatactca aagaaagaaa agtggcagca cttatattag 120
   t 121
<210> 173
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 173
   caaaagtgat aaaagtaact ttcaaggcta gatcatgcaa gacaaggcaa catagcttct     60
   acctggttct atgaagacat ttgcctttgg ctccctgagc ctccatccaa gaagtcgaag    120
   t                                                                    121
<210> 174
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 174
   gaggtccagg ctgcagtgag ctatgatcac atgccttcac tccagcctgg gtgacagagc     60
   aagacactga ctcaaaacaa aatacataaa ttaatttgtt taattcatga ttagttacta    120
   t                                                                    121
<210> 175
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 175
   aatgtgggcc acatgtgacc aacaagataa ttatgaaacc tgactgctgg atatgctgat 60
   acagccaaaa aacatcaagg actgtgagtg agtttggagg tgggagcaga gaaaatttct 120
   g 121
<210> 176
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 176
   gcaatcagaa aggtcctctt taaatgtgag ttagatcatg ttactgctct gctcaaaata 60
   atgcagtggt tttccattgc acagagtggc agattgcatt ttccaaaaga caattgcaat 120
   g 121
<210> 177
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 177
   ctagcatgat ttattaatat tagcctttct tctctccccg tttatgcttt ggtgggtact 60
   agacagaaac cccacaaatt ttaagacagt tttaagagaa atagtaactg gttaaatatc 120
   c 121
<210> 178
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 178
   aggtcataca gcaaataact ggcattcctg gaacccaaat tccaggtgtc ttgttccaaa 60
   acccatgttc tttattctat tctgcctctg ccaaacaaaa cccaaaccaa aaatgtcttc 120
   t 121
<210> 179
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 179
   agtcaataaa cccaaatgat aatttaaaat tcaccctgat gatggttcca ataaatatat 60
   aaatagtgta gctctagttc ggtttcataa gaattgtgca gcaataattc tttctgtaat 120
   t 121
<210> 180
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 180
   tatgccacca tacccgctaa tttttgtatt taatagaaac agggtttcgc catgttgaca 60
   agctggactt gaactcctca cttgaactcc tcacgtcaag tgatctgcct gctttagcct 120
   c 121
<210> 181
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 181
   aaacaaacaa acaaacaaac tgaggtttag gtttaggtag ctggagttta taggcatggc 60
   acataggtca gagcctcaat tttctagcta aatgtcaatg tttcccactt attttattgc 120
   c 121
<210> 182
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 182
   gcttacagcc agggctacac agaagtgagc aaagctggtg aagatgggga tgggggagtg 60
   aagtgagttg acgctagaaa gggatgtagc aaatgtaact attattccag aatccaagtg 120
   t 121
<210> 183
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 183
   ctactagcta cataatgtga tgccatatta aactgtaatc acctttccac caaactaata 60
   aagacaacat gctaattttt gtattaagac acagtgcaat aacacacaat tgaatgatgc 120
   t 121
<210> 184
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 184
   tgtgtactcc caaaattcat atgttgatac ctaatctcca aagcaatagt attaagggtg 60
   agtgcctttg ggaggtgatt ggataatgag ggcagagctt tcatgtacag aattagtact 120
   c 121
<210> 185
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 185
   gaaacagagg cttagacagt ttacttatgt gcccaaggac acaaaatcag aaacaggtac 60
   aaggagcact tgaaccaaaa ccaatactgt cttgccatac caaacagtat ttatttattt 120
   a 121
<210> 186
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 186
   cctgtggccg ttggttttcc tgggtgggga agggtgctgg cctcattcac aacagcagat 60
   actcattcct ccagggtcag gctatggggc tcaacgtgat caggacagat ctgagccccg 120
   t 121
<210> 187
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 187
   actgggcttg tagcttaaat tattcacact ttactcatgt aatgatgaac agttttaggt 60
   acttataata tgtagaggct aactctctct ttctctcact ctgtctttcc ctctgtttgt 120
   c 121
<210> 188
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 188
   tcaacccaag gcagagagag ccctgtctca aaacagattt ctgagtgtgg cttctgtcca     60
   agcatgtgaa ttaacatgta acacaaaaga gaagaaagaa atgttaagga aattatacca    120
   g                                                                     121
<210> 189
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 189
   attttctctc tctcgtagct gagagagtca tgactatggc gtgttctctg tactctgagg      60
   acctgaaccc actcatgggt tactctggcc tttggtcagg tagttttgcc aactcgctat    120
   t                                                                    121
<210> 190
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 190
   gcagtactca ccatgggcct taaggtgaga ctcagagatg tgctggcttc aggtataacc 60
   aagcacattt gaaactatag cggctatggg gagagattcc ttctgcttga gaaaaggaga 120
   g 121
<210> 191
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[C/G]"
<400> 191
   gtgtatgctt tgtgaggata ggtagctttt cttactcact gttgttacca gtacctagaa 60
   ccaagcctga ccttattagg ttctttcaaa tatttgaaag atattttaaa atattcacat 120
   a 121
<210> 192
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 192
   tgatctcatt accttaattc ctttgcttat aaaatgagtt cattggtcag aagcaacgct 60
   atgtacaata ccaagaatat gaatatgtca tttacagaat gacaagctcg tcaatttcag 120
   t 121
<210> 193
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 193
   gcaaagaatc actggttacc attatctttg aaatggctcc tcataaaaca cagaaaataa     60
   acattaagac atgaaagcta caaggcccac aatgcgggaa ttttaacctt gaaaactgtc    120
   c                                                                    121
<210> 194
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 194
   tcagagttta ggattttggc cattctaaga gatgtgcagt agtaactcag tgttttattt     60
   acaattccct aatgacatat gatgttaagt atcttctaat atgctcattt gtcatctgca    120
   t                                                                    121
<210> 195
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/T]"
<400> 195
   ttttaatatt tgtttagata tgacatttat tcaaagttaa aagcaaacac nnnnagaatt 60
   atgaagaggt atctgtttaa catttcctca gtcaagttca gagtcttcag agacttcrta 120
   a 121
<210> 196
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 196
   gttttccttt aacattccat tatcctattg ttcattcttt ggagctgtga tttgtttaat 60
   atatttcagg cttcttaata aatcaagtca tgtaagttat tatttggatc atttcgaaac 120
   t 121
<210> 197
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 197
   gtggttctaa agcttcggtg aatattagaa tggcctcaag agctagtaaa aaacacagcc 60
   agcctggatt attcaagtag gctagggttt ggccttttat ttttataata ttccgaggtg 120
   a 121
<210> 198
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 198
   gttggtgaga aggcatatgg ggaaaaaata aggcaggaaa ggaagacgga aaatgctgtg     60
   agtagggtgg cattttaaat actgtggtca gggaagcctc accaaaaatg tgacatctga    120
   a                                                                    121
<210> 199
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 199
   ttgtctttta ttggttttat aaaggatcta agtgtttgga aaggtgtggg accatgtact     60
   attggagatt tcagtgtttg actatgagag aaggaaatgt tattttttgg gaatgttatt    120
   t                                                                    121
<210> 200
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 200
   gaagcatcac ctcttttatc atatgaagcc ttttcacaaa ggagggaatg atgattgact 60
   aagttttgtg tctattctat acactgtact gtcaaagcat gcagagcatg tattgcatat 120
   a 121
<210> 201
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 201
   ctctttgtgt taaggttgta tcatctacct gtagtcactg cagtcagctg aattttacca 60
   agagaatctg acagtcgttg cccagtcaaa ttagtttaga tccatctgta acaggttcct 120
   a 121
<210> 202
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 202
   ctgattcctg ctctgtagcc acacagatgc caacagctgg cacttgtcca agaaacatgt 60
   actcaaggtc aggtgcagtg gctcatgcct gtaatcctag gtttttggga agctgaggag 120
   g 121
<210> 203
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 203
   aatagagacc agaaaggatt atttgatgtt catttagcaa gcaacatagt aaaataattt 60
   attccactgt ttgtatgtat ccttgactgt ttctaacaag tgacccattc tttcttaata 120
   t 121
<210> 204
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 204
   aaaataaagc ccagatgcct ttccggctcc ccccacgggg ttgccctgat ggtttaagac 60
   aataacagat atgaaaatcc tctgtaaaca ggaaggcttc accactcttg gaactcaaga 120
   t 121
<210> 205
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 205
   tgtgacgatg ccttatgaca aacaactcta catctcagtg tcttacacca atgagctcat 60
   aagcctgcag gttggctgtg gtgactgctc ctggcttggc cccatgggtg tctcatccca 120
   g 121
<210> 206
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 206
   ttatgctctt tattctaagg aagtgccccc taaaacaaag ctcaggagcc tcaacccggc 60
   agggaagaca gtttcctcac gaggcaggca agcaacacca ggtggctctc tttcccaaga 120
   t 121
<210> 207
   <211> 100
   <212> DNA
   <213> HOMO SAPIENS
<220>
   <221> source
   <222> 1..100
   <223> /mol_type="unassigned DNA" /organism="HOMO SAPIENS"
<220>
   <221> variation
   <222> 40
   <223> /allele="[A/G]"
<400> 207
   ttactagtta tgcaatgcac tcgaatccag tttaagttca gcgctctcat ctgtaaaagt 60
   ggggcaagaa tttgcctttt gatgttggga gatcaagttc 100
<210> 208
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 208
   gccctataga taccatggga aacagacagt ggcccctgtt ctcaagtggc ttagactcta     60
   atgggaaaga catttatttt ttcttttttt ttttttttta gagacggagt ctcgctctgt    120
   c                                                                    121
<210> 209
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 209
   agtatctaag cttattggcc ctaagtaaat cttaggttag gtagagctca gttcccaggg     60
   acattcaaga ttcataaaga agtgatattt ttcccagcta aaatattttt cttcttacca    120
   g                                                                    121
<210> 210
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 210
   cttttatttt aaactttttc ttaagtaatt ttacaattgc tgaaaagtaa aagagcctca 60
   atgaattcct gaataccctt tacctgtttt cctgaatgtt cctaaaaata cctagcaatg 120
   a 121
<210> 211
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 211
   ctgagcacct tctatttgcc aacaactgtt ttaggcactg gggatatagt gataaacaga 60
   acaaccacaa atccctgtcc tctggaactc accgtcagag tgaggaaggc ctgagtcccc 120
   t 121
<210> 212
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 212
   gggtgtgatt tggttgctaa tttctcttca cttctgggaa accagcccct tataaatcaa 60
   actataggcc agagaggctg ccacatgctc ccaggctgtt tatttgaaga gagacttaca 120
   t 121
<210> 213
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 213
   aaataaaagc aacacagagc agtatgtaca ggacagcgtt agaatatacc agagaacaag     60
   aacacaatct acaatcattt ccagtgaatg caggatgtta aagagatgca taaaatcccc    120
   t                                                                    121
<210> 214
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 214
   gactgcccag agcacagcgt ggagaaggcg ctcggccccc gcccaggcag gcagagcacc     60
   atgatgggtt cacgatgccc tatgccaggg tcgtgggtga caggtgtgtt tgccatctct    120
   a                                                                    121
<210> 215
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/T]"
<400> 215
   gactaaaaag catagtattc tgttcttcag ggagttgtgg gttcggatct gtgcaaagat 60
   aggaggtagc tgaataaaca tagttgcaaa ttataacctc ccaaatgtgc cctgaggaca 120
   c 121
<210> 216
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 216
   aaataaacac gaagaacaaa gccccaccac cgtgctgtgc tgtttgtgtg gccccactgc 60
   atcgaggcca caggctagct gctagacgca tctagagttc cctgattcct aaaattattt 120
   a 121
<210> 217
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 217
   cattttaatt ttcaaattgc ttgattaaaa tggcaaacag tttgaaaatt gtatacctct 60
   atatcattca gttaaaaaac aataaagtga cattcttaaa aacatcaagg actttcccct 120
   c 121
<210> 218
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 218
   catttataca ggataatgga aaagggggtt tctcccgagt agagaactta aacagtgtga     60
   agcacagtgt gttccacact atagctgatg ggttggcctc aggggggatg ttcaggtata    120
   c                                                                     121
<210> 219
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 219
   taatatatga agggtgcatt attctaattt aatggattaa tcatactttt taaaaacagt     60
   attactaaat tctgtaataa catggtgatt ttatatacac atgactaggt gaaaggatat    120
   t                                                                     121
<210> 220
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 220
   gcaaggtggc actcttagga gttgaatcca gctctggtgt gtgggacagg caggaggaga 60
   agaagagagg gaggaaaagt cggttcgaga acccaggtgg aaaatagatt gagggaagca 120
   a 121
<210> 221
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 221
   gcaggcgccc cctttccccg ctccccaggc gcttcagcac cgcggacagc gcccatccga 60
   atcactgagg ccaaagccca gcacgtctaa ggcagtcccg taggaagacc ccgtgtgcac 120
   c 121
<210> 222
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 222
   aggaccattt tagaaatctg tgaaccacag tggtgaaaga aggaacacat tctctacaga 60
   aatgtatatt aagtgtctgt taacctggca ttgtcctccc caaccaaaac tatttctatt 120
   g 121
<210> 223
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 223
   agagcagggg aaagagagtg gaagtaccag gtgggcaaag tttacaattt taagtaggat      60
   agtcagggca gacctcatta aggagataac tttgagccaa gacgggatag agcagaagga     120
   a                                                                     121
<210> 224
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 224
   cttcttgcac atgattcttt ccatgacacc tagtgccctt ctccatctag agctacctct      60
   atatgtccac gttccttctc tctaagctca tgatagacct caggagaaag tcaggtaggc     120
   c                                                                     121
<210> 225
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 225
   cgtatggaac cttttggcat tggctttttc tactcagcat aatttcctgg agagtcatcc 60
   aaattgctgc atgtatggat agcttgttcc atttcattgc tgagttcatt tgcttttttt 120
   t 121
<210> 226
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 226
   gcaactttac ctgctaatga ctatatacac ccatttttct cattttttaa aaatatcatc 60
   acatattact ttaaaatgtc aagaactgct tcaacagcca ggcaatgatg gctggtatgc 120
   t 121
<210> 227
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/T]"
<400> 227
   aagcaaagca attgctacaa ggaggattat gggtgaaagt catggatgga ttatgagtta 60
   atcacacacc tagagaagca tgtaaaatgt gcaggtaaat tacacccatt cattcaggca 120
   g 121
<210> 228
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 228
   cccgcccacc tgagcacagt gtccatatag gaacatgagt gacagccctg cacatgggca 60
   agagcatcca aaccacactt caggcaaaac tacatttcag tgatgtccat ccttaggaaa 120
   a 121
<210> 229
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 229
   cttactttaa catccaaaaa taactaaaaa gtcctagaaa attaaacttt tccaaatttc 60
   aaaagtactt gtgctgtatg aattctactt catgtatcat acacaaacaa gttatgacaa 120
   a 121
<210> 230
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 230
   acatattcca gaaattctcc ataatttctg atccactctt acattcctct cctttccagc 60
   actattattg atctcttctt cttcttttga aaatctttgt tccctccatc tatcatttca 120
   g 121
<210> 231
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 231
   gcagggtcca cgggcttctg gacacctccc tacctgggcc ggcttcatcc tcctacgacc 60
   aacagtcgtg ttgatgacat gcacctgtcc cgggacttcc cccagccccc agccagctgc 120
   g 121
<210> 232
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 232
   aaaaataatg ttcctttcta aatatgctaa attatttcca taaaactcat aaacttttat 60
   acctagaaat ttatgaaaac ctattgacaa cttttatgcc tgaaaagatc tgaaagattg 120
   a 121
<210> 233
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 233
   ggccacaaag cccttgcaca ggcacagcta taatttttgt ctctcttctg ttggaaaggt     60
   acaaagttaa ctggagtgat gtgtgtaatt gatggtataa tggtaagcaa aaatcacaaa    120
   t                                                                    121
<210> 234
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 234
   gaagagagga gtgaggtgct atgtactttt aaacaacaag atatcatgag aactcactcc     60
   atatcacaag aagagcaccc tgggggatgg tattaaatca ttagaaacca cctcatgatc    120
   c                                                                    121
<210> 235
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 235
   aaactaggaa gaatattgaa ggtagccaga aaagaaaaaa aggcacattg catgcagagg 60
   aacaaagatg agaatcacag caaacttctc tttagaaaca atataagttg taagacaatg 120
   g 121
<210> 236
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 236
   ggctgagtga atgaccacca ctctgtggtt caccaaaaaa ccacatcagg ttttccccag 60
   acaccttggg acagtttgaa atgtccaaat agtaaagcaa tgaactgcca taaatgtagt 120
   t 121
<210> 237
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[C/G]"
<400> 237
   gttgagggga gcctggagaa gttggctggg acagatgaca ccacttggag accatattta 60
   cgctctcaga ctttatccaa gtgggactgt tgtttaaagg tttgaaaaaa catggcttat 120
   a 121
<210> 238
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 238
   cactcacagg tctcccctgg attgtgcaga accagacatt gctgcctttg cctaggcagg     60
   ataatagata tcatgagggc ttgggaagct tcggggggaa agttaggcta tctgcccacc    120
   c                                                                    121
<210> 239
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 239
   agtatagtcc tgagaaagtt ttggccaagc caatggagag ccccatagcc aaagctgccc     60
   attagaggaa tcccatatca agtagaattg gatgggtgag aattctcagg tggctgagag    120
   t                                                                    121
<210> 240
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 240
   caagaagaga ggataaccaa cacacaaatg agtaaataaa atgattgctg attgctatta 60
   atgctaacaa ggaaagagat cctgttccat gtgagtgaga tcatgcccat tgcttcatca 120
   t 121
<210> 241
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 241
   catggcacac agtcacagaa acatagcaag cccttgaaat caggctttct gactttgtct 60
   aatctcctgc tttagcaaag acatcaattc tccctccttt tatttaaatg gtggctgggt 120
   c 121
<210> 242
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 242
   cacctacaca cacctacaca catgcatgca cacacacatg gcctctctct ccaggcttct 60
   agagctcagg acaggtcaga tccatctctg tcgggcacaa cattgatgct ctctgaacac 120
   t 121
<210> 243
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 243
   ctgagagttt cgtacagacc tggtccaaaa attccaattt cataggtgtg gagttttcat     60
   acaagtactt caattgctac actcaaagag aaagatttaa cacctagaaa tctagctgtc    120
   t                                                                    121
<210> 244
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 244
   aataatgcat aaggtttttg taagaattag aattaataaa gtacttagac cataataact     60
   aattagtatt agttgttgtc tttgctatta ttttgatgtg gtggttgttt ggtttcacct    120
   g                                                                    121
<210> 245
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 245
   tccctgcttc ttactaatat tgtcactttg tctcttaata cagatatttt cttttgatca 60
   atgtttgtaa agtaacatat gtttctgacc tcttacttta aaacttacta tggccttgta 120
   a 121
<210> 246
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 246
   gccttgcaga ttatgtagca ggtcctgatg taacagaatt aagattgcag gtgggattgg 60
   agttgctaat cagctgactt tgagatggag aggtgatcct ggattatttt ggtggaccca 120
   t 121
<210> 247
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 247
   ccccctaggg agtagctgcg gcggcaccaa gagaggggtg gggggcgtgc tgcgcagagg 60
   aggacctcac aaagcggcct cagagtttcg caggtcctgc tgttctaggg aagggttaaa 120
   g 121
<210> 248
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/T]"
<400> 248
   gaccacttga acacatcctt ttaaatagat acctttttta aaatctatgg ttatgtaaca 60
   actgtgtccg agggatttaa gcagaaagcc ctgtgggttt ctcttttcaa aagacagacc 120
   t 121
<210> 249
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 249
   gtgtcctctc taaggatggg acccctactg tccatctcag gctcagcact gccttggggc 60
   aggccacttc tggcttcttt aggcctcgtt tccacgggag gggaagctgg gtccgatggt 120
   g 121
<210> 250
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 250
   tctgtcaccc cttctacatc ttagctcacc tgtcctcaca aataaacatc actcttgaat 60
   actacaatct cactttatta gattgtaaat ttttatgagg aaaaaggtcc tgagctatgg 120
   c 121
<210> 251
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 251
   acacagcatt aattaaaaat ggaagttttc cacttccttg ataatttggc tatctgaata 60
   aatttgtgaa tttgctaggt taagacctag ttcgtggtca catttcaaca aaacagcttg 120
   a 121
<210> 252
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 252
   acttatatct ctttctaaac actagcagcc cagaattctc aggccacttt tgggcattgt 60
   agcaacacaa taggtgcctc ctgtggaccc catgcctcca atcagagcag ggattaccgg 120
   c 121
<210> 253
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 253
   atggagggga cagagtttat ctttttcacg gtttgtatat atatattttt taatcttttg 60
   agagtcccag tttttgaagc attcacttgg ctgattcacc aattcataga ctggagtaga 120
   a 121
<210> 254
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 254
   tagtgtaagg tgaccggaaa aatctgatta aaggacaaat gttcagttca aaggtgtttc 60
   aagctgagaa tagcatctct atttactcct cacgatgttc atctcaggag gcactgtact 120
   t 121
<210> 255
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 255
   agagcttttg cagaacttgt tgatgaattg aatttatggc ctgggtgagg aaaaggaatt 60
   agtaatggca cttgggtttt tggtgtgaac aactagtgat taatcggagt tcccatttaa 120
   c 121
<210> 256
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 256
   ttctcagtgt tcaccaagtc tggttgtccc agtctcctat ctctgtctgt tcctctcctc 60
   atctgtcttt atgttagtta tggccctgaa tataaaacag ataaaggaag ggtctggttg 120
   a 121
<210> 257
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 257
   taatttctac atttctacca aaagtcactt catggcaatc taggcttttt ctatcacatg 60
   actcaaagtt ctccagcatc agcatctacc cattatgcaa ttccaactca tttccacatt 120
   t 121
<210> 258
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 258
   agtagaaaat aagcacaata attttagatg tttataagtt ctctgaaaac aatagagtat 60
   aatgatataa cacgtttagg tagtttggaa aattatagtc gagtcaatga ccttagattc 120
   a 121
<210> 259
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 259
   atatctgagt attaagaaaa attgaaaccc taagcatcaa tttcttagga acttctctga 60
   accattaagt tgttttaaaa ttactttcct ccatcagact cctaatcatc acctagtgat 120
   a 121
<210> 260
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 260
   tacatacaca catacacata catgcagata gatagataga tagataaaga tctccagtca 60
   ataacctaac tttacatcta agaaactggg aaaaaagcaa ataaaacccc aaagcagcag 120
   a 121
<210> 261
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 261
   ctcgcagcgc ggaactctga cgcaatccag ggccgaggaa aaatgattaa aacccaacaa 60
   actcgagtgc tggggtccac caagcgggcc gtcttggtta gaaggcccgc cccacacgtc 120
   t 121
<210> 262
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 262
   agatcacttt ttattgcaat atgcaattta ctggagagat gaactgctcc tgctgagatt 60
   attagtgtca ctgcatttta agcaggtaca acacttgaac tcactgcagt agcaacagga 120
   a 121
<210> 263
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 263
   gggggctcca ggcagaggga acagcttgtg caaaggccct ggggcaggcc aagggcagag 60
   aacttaaggt atggaaaaaa aaaaaaaaag gcatggaaag gaggccagca tggctaggag 120
   c 121
<210> 264
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 264
   gtcagatgtg ttgtggaaga ataattactc tattttgtga ttttataaag tgtattttct 60
   atattattat taaatgtctg attacttgag ataaaccagc catcctcttt ttttactact 120
   c 121
<210> 265
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 265
   taaatggtgg gtgctatttt gttgctgtta ggtctatttt cttcatctgt tatttcgcat 60
   aacagtaaaa cagatactca gatgacttat ataactttca ttagtttcat taggtggtgt 120
   c 121
<210> 266
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 266
   gaacgaatga gaagtgagga aacgcttagc gcaaaaggaa aaagagagaa agacatacag 60
   aaacaaggtt acgcggaggc cggcgaaaag cgattccccg ctcccccagg ccaagggccc 120
   c 121
<210> 267
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 267
   tacaaggtca ggctcaacgg aagtgaccgt cccacagtta tgcagcacta agtcaatggc 60
   acatttgctt gtgtgttggt tacatttgta actcaaagct gatgccttaa gaaggttagg 120
   g 121
<210> 268
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 268
   cctgaagtgt ctgcttagcc gcgcacgggg tatttatatc tcaggctttg gagaactatc     60
   aggtttgggg cccggctagg gcgtgcgtgt tcacgctggg acctgtcaca acctggtctt    120
   a                                                                    121
<210> 269
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 269
   atggatcttg aaggggaccg caatggagga gcaaagaaga agaacttttt taaactgaac     60
   aataaaaggt aactagcttg tttcattttc atagtttaca tagttgcgag atttgagtaa    120
   t                                                                    121
<210> 270
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 270
   gtttaaaaca acactgtgat ttcacaattt ctgtggatca tgaattaagg agcagcttag 60
   atggatggtc ctgggttgag tccttcctgt ggctgcagtc aagaagtcag ctggggctac 120
   a 121
<210> 271
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 271
   tggctactct aatttttcaa tggtaaacag accagagtta ttctaagaaa ttatgaaaag 60
   aaatccattt cgaagtctta aagcaaattt agagactgac aattgaaaat acatcttctt 120
   t 121
<210> 272
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 272
   gcttgaggcc aggatttcaa gacttgcctg agcaacataa tgagatgccc tctctcaaaa 60
   atttaattaa ttaatttaaa aagaaaatcc cagctactca ggaagctgag atgggaagat 120
   c 121
<210> 273
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/C]"
<400> 273
   aacttaaatc agcaagcaga aaacaaacaa cttcattaaa aatgagcaga ggacctgaac 60
   aaacacttct cagaagaaaa cattcttatg gccaacaaat acatgaaaaa agcctcatca 120
   c 121
<210> 274
   <211> 121
   <212> DNA
   <213> HOMO SAPIENS
<220>
   <221> source
   <222> 1..121
   <223> /mol_type="unassigned DNA" /organism="HOMO SAPIENS"
<220>
   <221> variation
   <222> 61
   <223> /allele="[A/G]"
<400> 274
   caagggcagc atgttgcttc atgagcggtt ctggacaggg atggtgggag atgttgctag 60
   agggaattgt ggccctgggc ttagaaacaa aggggcaaga aggtctcaga agctggggcc 120
   t 121
<210> 275
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 275
   aggactctat ggcttccttc atgtcatcgt ccactctgcc aagggattta 50
<210> 276
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 276
   acgacagaga tgaattgagg ggacaaatgt cagagctcac agacgactgt 50
<210> 277
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 277
   tcagaagcct atttttaatg tcattccacc aattcccgtt ggttccgaaa 50
<210> 278
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 278
   tcacagtagc tcctcctctt agggttttat agaagtccat cacatctccc 50
<210> 279
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 279
   ttgcattctc tcctagcact ccagtaaata aactatagtc ctggtcaagt 50
<210> 280
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 280
   attcccaata ttcgtatatg tatttataaa ttacataatg ggcagggtgc 50
<210> 281
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 281
   agtgcttttt gagaggtatg aacttactcc atactactta catctgctaa 50
<210> 282
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 282
   tgacttctaa ttacaggcaa aatcaacctt aataagaaca ggcgttacta 50
<210> 283
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 283
   tactattctg ttcataaggt acacttcttt ttagggcaca ctaccttggg 50
<210> 284
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 284
   aggtgggata aacagaagca gcataacgtg tcttgatgtg tgctgtttag 50
<210> 285
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 285
   ttgtcatgca gcaggttaac tatgctttct ggagaaggtg tcagccaact 50
<210> 286
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 286
   ccctccagct gaatgatttt tgtctgtgcc tggcccagtc cctgagtcca 50
<210> 287
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 287
   gtgaaaatgc attttccccc tattccttct ggaaagcaac attagggtcc 50
<210> 288
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 288
   tgctcaccac agtcctcata tccttaaagg gacaccctag tgattactga 50
<210> 289
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 289
   cagtcccgcc tgcttggatc tgacgagcgt gccgattcgg tccgaaaatc 50
<210> 290
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 290
   agagcactaa ctctggagag taaggatctg agtgtaagtc accgctgtgt 50
<210> 291
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 291
   aagcagtcca cagcagtctg agctggcagg tcatggagca gcccccaaac 50
<210> 292
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 292
   gtaaagaaca gggggagata atgatcagta aaatcacagc agggtgaggg 50
<210> 293
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 293
   tatttaggta gttgaccact tcagcattct aggtacaata acgttagccc 50
<210> 294
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 294
   agaacaaagc caggacaagg tacaaggtga ccccagcaaa tttccttttc 50
<210> 295
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 295
   tgctagaagc ttatcaactg cattaatctt tttaaaaaca cttttagttt 50
<210> 296
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 296
   tctcaccttc tccaggtgca cggtaggtgc tgtgtaaatt aacgacttca 50
<210> 297
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 297
   gcctcctgct agacaattag ctttatccat gagttaccaa agagggagcc 50
<210> 298
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 298
   accaaaatct ataaacaata aggaactgtg gttgtttgct gcaaataact 50
<210> 299
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 299
   tcaagagttg ggaatgatga gggcatgtac tgtgactggc acacagaatg 50
<210> 300
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 300
   agtgcctact atgtgctagt ccctagtgac atgagagtga ggaaggcagt 50
<210> 301
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 301
   ccttatttca aggtcggggt caaggtggtc aaaagaactg ttttgctctc 50
<210> 302
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 302
   aagggtattt atacctttgc ctttccgcct caaccattgg aacctgggac 50
<210> 303
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 303
   agcctctctg ggtccttggg gagcatgagg atcctgcaga aagcagagtg 50
<210> 304
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 304
   atgctctctg aacactatga cctctgatta tttatcaacc tccaagagct 50
<210> 305
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 305
   cccctcttct gtgagagcca aacagagccc ttcctgagtc ccatccattc 50
<210> 306
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 306
   tctgggtcct tttcattgct ctacaaagaa tcctttcttc ctcccaggcc 50
<210> 307
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 307
   catcaatgcc cacgctacac gaggcatact agacagtcgc tgcctaagcc 50
<210> 308
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 308
   tcaagagtaa cagtatgccc tgcattaaca gggataatat ataagaaaaa 50
<210> 309
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 309
   gaatttatta ctcctgggag gattctgctc accactggca actatgacca 50
<210> 310
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 310
   catcatgatg taatgtagtc atatagacta ggacacttag attagccccc 50
<210> 311
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 311
   ggttttagta ttgcaatgtg gaatccaaaa ctgttatcaa tgaacttttg 50
<210> 312
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 312
   tgggtgaggg aaccgttagt gccatcctga ggccccgtgt caggaaatat 50
<210> 313
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 313
   actgaactcc ccatcacaaa tctgtatgct ttattagaaa gtaaaactct 50
<210> 314
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 314
   agtaaaacag acgacgggat ccccagacgc tgcacatcag caccaggagc 50
<210> 315
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 315
   cacactaata ttcaaacatc cttgacctca tctcatataa ataaatccaa 50
<210> 316
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 316
   ccaagattct ggatgtcttt aaggtaacaa gtgtccatgt tgttccttga 50
<210> 317
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 317
   gaagcgaaaa tagctatgca ccaaatctct gcaggcattt cattgagtac 50
<210> 318
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 318
   tgaatgacag tgttgttgat tagttcaagc tcttgccttt ctctaaactt 50
<210> 319
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 319
   gatcttagcc aaggcaggaa agcacacgat caggtaacct ccagattcac 50
<210> 320
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 320
   ttactcgcat taactctttc aatttcacaa caaatctaag aaaaatgcaa 50
<210> 321
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 321
   agtctaaaac actatcatct cctcctggat tactgcaaca gactccttct 50
<210> 322
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 322
   tctgccctaa atattccctg ttcggtgggg tttggcggtc cagcagccct 50
<210> 323
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 323
   ccatgcgtgt tggaagtatt tctcttgttc tcctgctttt agaaagccat 50
<210> 324
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 324
   cttctgaccc tcgccgtcct agaaccaacg gcccctcggt gtctggtcct 50
<210> 325
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 325
   aaagctctaa taccacctaa aaccatttct gttctctacc tctgtcatta 50
<210> 326
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 326
   acaggttcta tatctttaga tggtaaatta aaaattcctg gctgaatttg 50
<210> 327
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 327
   aatgtgagta gattccaacc tttatccatt ccattcacat ttaccttctc 50
<210> 328
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 328
   ttgtttaaag ctgctgcagg tatactcttt ggaggctaat aataaagaac 50
<210> 329
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 329
   tggagtagtc ttcttctagc ccttgcatga cctctcttac ttcacccata 50
<210> 330
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 330
   cttccacctg ctgcactcca atatagccac tatgttcggc tatatatata 50
<210> 331
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 331
   tagagagtaa tgtggtgggt gtgctgtgtc agaaaggctt cactagcagt 50
<210> 332
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 332
   ctaatttgat caatgaatca ctgctagcat gtgaatgtcc ataatggata 50
<210> 333
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 333
   ttattagagg taaacataga gataagcccc taataaaata gtagctggag 50
<210> 334
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 334
   agtgttaatt ctctaagagg aaaatgtcat ttctccaaaa caaaacttta 50
<210> 335
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 335
   gtaacaaggt tacctccaga aaaaaaggct attgctgaac agaggctttc 50
<210> 336
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 336
   aagagagaaa aatattttta agtgaaaagg aacaaaacta ttctatacga 50
<210> 337
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 337
   ggctcacacc gagatcaatc catgatgaca gcacttcatg gcccgtctca 50
<210> 338
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 338
   gataatctaa ttcatctaac ttgctttaca aatgaggaaa ctgataatcc 50
<210> 339
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 339
   gtggaccctt tgagtggtta cagacgggcc tcaggattgg tgttatttaa 50
<210> 340
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 340
   aacaggggcc actgtctgtt tcccatggta tctatagggc ctggtggaca 50
<210> 341
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 341
   aggggtcaag atacaaggag tcaccaaaga atgcagaaga gacaagttca 50
<210> 342
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 342
   ccttttctaa gaccaatatt aacaagaatt agtagtagaa tgttcttatg 50
<210> 343
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 343
   tgttgctaat cccaaccagc atgatttacg ggaagtaaat catctatgac 50
<210> 344
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 344
   gcctgtctca caaacattgg gttctataga cgctcctaga ttgcattttc 50
<210> 345
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 345
   cccagtgcct tgacagggta tggggggacc tgcatgacta gcattaaatg 50
<210> 346
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 346
   taaccaggga tctgtgcgtt ttgctataat tcagaaagta gcagactact 50
<210> 347
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 347
   aaaagtcggt tcgagaaccc aggtggaaaa tagattgagg gaagcaaaac 50
<210> 348
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 348
   gagtaagagt taatcacttc cactgtgcac ttgtttattc caagtagaaa 50
<210> 349
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 349
   ctctggacat cttcagaggg tcccacttta gacttcactg atctcttttt 50
<210> 350
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 350
   tcacacttta catttattat ttccagtaag ggatatagct aagatagtta 50
<210> 351
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 351
   cagtttgatg aatggcaaaa tcgttcaaat ggaaaagagg agagagatag 50
<210> 352
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 352
   ttcgtaatta aaggaacaga gtgagagaca tcatcaagtg gagagaaatc 50
<210> 353
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 353
   agccagggtt gaagtcactc acgggtcctc tccgagaact cgagtggtga 50
<210> 354
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 354
   caaaggtgat atgcatttta aatttgatag ttattgccca actgtcttta 50
<210> 355
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 355
   ccctcaggct gcttgttacc gtggaagctt cctgaactct ctccagaccc 50
<210> 356
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 356
   ttttcatttt tctcttccca acccaatccc ctctctctaa atcttggtat 50
<210> 357
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 357
   ttcaatatat gttttctgaa caccttctgt gttcaaggca ccatgctggg 50
<210> 358
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 358
   cccttgcatg ttcaccttgt tatgtgtact ttcatctcaa ttgccagtta 50
<210> 359
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 359
   aaagtatctc cccaaatcat tcccaaacac tacaaaggta gtgccatcag 50
<210> 360
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 360
   tgctctaaaa ctaatttgct tgaagtgtac agaatggaat tcgggaagga 50
<210> 361
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 361
   atcacttttc catgaaattg tctttgcatt agcaaaatga atcaagcata 50
<210> 362
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 362
   ttggtgatgc tgatagttgg agatacccag acagataagg tatattgccc 50
<210> 363
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 363
   atcaatatga ctggtgtcct tcaggaatgt ggtagcacag tgaaaaaggt 50
<210> 364
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 364
   gcagtagggg actggctgcc gagggggcat ctagattgag ataggtggga 50
<210> 365
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 365
   attggcaaaa gtgctcattc tggaaaaaca aagaagtgag aaagtggatg 50
<210> 366
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 366
   attctaaagc tttgtgtggt ccaccatgat caccttttcc tgcttccccc 50
<210> 367
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 367
   gctccatttt ctttgaggta catcaacatc aataacagat caatggaccc 50
<210> 368
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 368
   agcctgacct catggcttag ctgtgcctcc tggacaccat ccctctctgc 50
<210> 369
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 369
   ttctgaaagt cacagcccag ggattcagac ccactaaaaa aaactgagat 50
<210> 370
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 370
   actacattac atcatgatgt attgattgcc tctggcctag gaatctgcag 50
<210> 371
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 371
   ccactcatat gtctgttctc actcagaggt gaggccctgt gtcttcagcc 50
<210> 372
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 372
   gggggacaga gaagtaacgt cacaagattt taagcttggg ccagatatgg 50
<210> 373
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 373
   aagtagagca gaaagggcaa gcagagaact agacagagaa gacagatgac 50
<210> 374
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 374
   tggctgcctc tagggcaaga agactgggga tatcaccatg ggctcaatgt 50
<210> 375
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 375
   ccaagtcctt ctacctccct gggtgaggga accgttagtg ccatcctgag 50
<210> 376
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 376
   aatcttgggg aatctgagtt tattagagga atgtagggag gaagcaggct 50
<210> 377
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 377
   tatcatatgc tctagtgact tcatcaagac agtctaaagg aagatgggcc 50
<210> 378
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 378
   cagaaacacc tttaatgttt ttatttctat gaatattctc ctaatgatta 50
<210> 379
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 379
   ttaaaatgag atcccttcca acatgctttg ctgagccaga tttataaaat 50
<210> 380
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 380
   tagtacagta agggcaaagg gcactgcaat tgctattaaa ctgtaagaag 50
<210> 381
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 381
   atcccccgga actgggggaa tttccaggca catgaggctc tgtcaaccca 50
<210> 382
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 382
   agccacttaa aataaatttt tccagcagtt attcatttag tgccaaaata 50
<210> 383
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 383
   gcaggggcac atgcaattgc catttaaaaa tgaggtctgg catggccaga 50
<210> 384
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 384
   gtaccacagc tcccagctgc atgtacttta aaaatgtgtc taagccaggc 50
<210> 385
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 385
   tgcaaacaga aaaatcagaa cctgctcatg ctgccatatt aataggaacc 50
<210> 386
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 386
   taactacaca ctcaaggctc cctctcaaag tctcaaacct tacaacttcc 50
<210> 387
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 387
   aatacagcca tgcgctacct actggcattc ccgtcagtgc gtacacgatc 50
<210> 388
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 388
   aactgctttc ctcattggct tggtctccat agtgattcat tttgctgtaa 50
<210> 389
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 389
   tggaaatttt tttgtaatta gaaatgacct aaaggatagt ttctattctt 50
<210> 390
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 390
   attgattttt atgtcagcaa tcttccaatc ttgttaattc taaaatactt 50
<210> 391
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 391
   gcctaagctg aacctgagag gtgaggaaaa cagaccaagc tgaccaaacc 50
<210> 392
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 392
   gcgaactgtg gagtatctca gtaagagtgt taggaggaat attttatagg 50
<210> 393
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 393
   acaacaacaa atctcaaaca actgttctgc caatggggtg gagcaccttt 50
<210> 394
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 394
   tgatgatttt ccagcatggc aatggtaaag ctgcaaataa aaagcagcca 50
<210> 395
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 395
   ttcttttctc caagcaaaag agagaagagt ttatttcatt ctcagcagct 50
<210> 396
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 396
   ggcaaaagca gagatgtgag ctgtaaattt gaatgaagga ccagatagaa 50
<210> 397
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 397
   taggaacata aaagttcaga tgttagtagg actaataaaa agttattgtt 50
<210> 398
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 398
   tttttcaggt ctagcttaac caaaacactt aaaactgtta ccaaaaaact 50
<210> 399
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 399
   caaataaata aactttaaag aaatggccaa cttgggaagg acattaggcc 50
<210> 400
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 400
   cagtccaaca accagttcca gaagatctca gaggtaggcc gctccccaca 50
<210> 401
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 401
   tgaaaatgtt gtctggacaa gcactgaaag ataagaaaga actagaaggt 50
<210> 402
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 402
   gattcatttt tacatgttta tttttaatgg agactaaaga gacataaatg 50
<210> 403
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 403
   tcttgattca attggaagta actgagaggt atatcacatg ttgtgattca 50
<210> 404
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 404
   tgctccataa cacaaataat ttcattcttc ttcctttctt gccgagtagt 50
<210> 405
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 405
   atgagcaagg aggccaaaac cctgcgtgga cggtctgctt ccctgccctt 50
<210> 406
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 406
   gagtgccaaa tatgtgccct tccccgtggg gaagacaaaa gtatgagaca 50
<210> 407
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 407
   tatttttagc agcctatgga ttctaggagt gacccagctc cagggatagg 50
<210> 408
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 408
   catgaggaaa ggctgcaact ttgagctccc tctttagcta gggagcctcc 50
<210> 409
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 409
   agcattaatg aagcacaggg cctatcacgc agtcaggctc agtataaggt 50
<210> 410
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 410
   catactcaaa ttgatacaca gcctttgtcc tgagtgtttg tcttccaaaa 50
<210> 411
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 411
   agagtagtat tgcttaaaaa ctgctccaac cacttcttaa acctgaaacc 50
<210> 412
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 412
   tcggccaaaa tcagggacaa ggatgacatg ccattgctta ccaactgcta 50
<210> 413
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 413
   ccgttgtgca aactccagaa agggcatctc tctgtcccac tcccccatta 50
<210> 414
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 414
   atctgcgtaa attgctgcat ctctcttggc ctcagttttc ttagccacac 50
<210> 415
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 415
   gtaagtgcca gctactatta tttaggaggc taaggctcta ggtgatgagg 50
<210> 416
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 416
   tgccacccta tggcattctt gttgtgtaat gaaataactc tcctatgaaa 50
<210> 417
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 417
   ctgcgcttgc ccaggaggcc ctggtctgca ctgtttatag agaagaaccc 50
<210> 418
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 418
   ttaggaaagt tctgtacaga tatgtgtaat ccagcatctg tttatctatt 50
<210> 419
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 419
   aatgatggaa aaaactgcag cgcacggtgg aaatgtctac tttgtatgca 50
<210> 420
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 420
   ctcctcatta ttcgcttctg ctgtaactgc acctatggta acccaggtgc 50
<210> 421
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 421
   aagtgctctg taaccaaata ttttggaaat gctgagttgt accaagttgg 50
<210> 422
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 422
   ttttgaaatt tccattatat gcaaagccca tgaaaggcta aatatcagtt 50
<210> 423
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 423
   gtttgtaaat gcacactgtt gggggaaccc tcttcctagt ccttgtttcc 50
<210> 424
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 424
   tgggcgagaa cttattcctc aggccattag attccctaat gctgcacctt 50
<210> 425
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 425
   gccatgggca aaaacagctc aggtagtaat gaaggtgtgg ctatagctga 50
<210> 426
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 426
   acatcaaact aaattacatc atcagagtaa agagacaatt tacaaaaagg 50
<210> 427
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 427
   aaaaagttct tcttctttgc tcctccattg cggtcccctt caagatccat 50
<210> 428
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 428
   ctggctccag gcaaagaata ctaccagcaa caaagaggaa catttcagat 50
<210> 429
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 429
   ggactagcct gctgcttcat ttcccccctc ctctgcagcc gatttcagaa 50
<210> 430
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 430
   atattagtaa cctggaaaac atacatggag gtatgttcat taacggcagt 50
<210> 431
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 431
   atgggaagag ctggattttt gtcgtggagt aaaggagagg gaatcaagaa 50
<210> 432
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 432
   aaaatcatag aaattgtgtc taaggatatg ctttgggata tttggacttc 50
<210> 433
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 433
   cataaaccaa agggatcttc tctactcgtg cgtccctagt ctctctcccc 50
<210> 434
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 434
   gctgcctgta ctagtgatag tgaggctcac taccatccac cacctaaatt 50
<210> 435
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 435
   gtgtagctta cgggagggaa gtcaaagtca ggcacgttca tcacactcag 50
<210> 436
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 436
   ctcattgtaa gattcaaaaa cattccagct tacaaaacat atccagctta 50
<210> 437
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 437
   tttgcaaggc aatttgttct actgctggac agcttcatgt ttaatgtttt 50
<210> 438
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 438
   ctatatttga acaagcttct gggtaatatt tatgacaggg aagtcttgag 50
<210> 439
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 439
   ctgtgaacca ggcactgttt gaaatgttcc atttattgac ttatttaagt 50
<210> 440
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 440
   actactacta atgttgaaag tataccatgt aacaggcact gtacaaagcc 50
<210> 441
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 441
   ttttgggttt tgttgctagc ataaaaatta ttacctagtg gatggtaaca 50
<210> 442
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 442
   tttttttttc atttgaagta aatatccacc tttgtatcta attttgcatt 50
<210> 443
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 443
   ttatttttta atagtgttct tgcacatgag gagaaagact gaattcaatt 50
<210> 444
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 444
   cgtgtcactt cgtttgactt cagctgggaa catgcatatc agtcgactca 50
<210> 445
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 445
   atcgtcacac agttttaaga caaatgtttt tacctatttg acctagtctg 50
<210> 446
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 446
   tgtgctacaa acctgaaact ggtaagacaa gcacaaagca acgtgcaata 50
<210> 447
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 447
   cttggatgga ggctcaggga gccaaaggca aatgtcttca tagaaccagg 50
<210> 448
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 448
   atcatgaatt aaacaaatta atttatgtat tttgttttga gtcagtgtct 50
<210> 449
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 449
   acatgtgacc aacaagataa ttatgaaacc tgactgctgg atatgctgat 50
<210> 450
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 450
   gtcttttgga aaatgcaatc tgccactctg tgcaatggaa aaccactgca 50
<210> 451
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 451
   ttattaatat tagcctttct tctctccccg tttatgcttt ggtgggtact 50
<210> 452
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 452
   tttggtttgg gttttgtttg gcagaggcag aatagaataa agaacatggg 50
<210> 453
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 453
   agaattattg ctgcacaatt cttatgaaac cgaactagag ctacactatt 50
<210> 454
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 454
   caggcagatc acttgacgtg aggagttcaa gtgaggagtt caagtccagc 50
<210> 455
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 455
   acaaacaaac tgaggtttag gtttaggtag ctggagttta taggcatggc 50
<210> 456
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 456
   tctggaataa tagttacatt tgctacatcc ctttctagcg tcaactcact 50
<210> 457
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 457
   cataatgtga tgccatatta aactgtaatc acctttccac caaactaata 50
<210> 458
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 458
   caaaattcat atgttgatac ctaatctcca aagcaatagt attaagggtg 50
<210> 459
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 459
   aatactgttt ggtatggcaa gacagtattg gttttggttc aagtgctcct 50
<210> 460
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 460
   ttggttttcc tgggtgggga agggtgctgg cctcattcac aacagcagat 50
<210> 461
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 461
   gggaaagaca gagtgagaga aagagagagt tagcctctac atattataag 50
<210> 462
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 462
   gcagagagag ccctgtctca aaacagattt ctgagtgtgg cttctgtcca 50
<210> 463
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 463
   tctcgtagct gagagagtca tgactatggc gtgttctctg tactctgagg 50
<210> 464
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 464
   ctcaagcaga aggaatctct ccccatagcc gctatagttt caaatgtgct 50
<210> 465
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 465
   gtgaggatag gtagcttttc ttactcactg ttgttaccag tacctagaac 50
<210> 466
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 466
   acgagcttgt cattctgtaa atgacatatt catattcttg gtattgtaca 50
<210> 467
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 467
   caaggttaaa attcccgcat tgtgggcctt gtagctttca tgtcttaatg 50
<210> 468
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 468
   ggattttggc cattctaaga gatgtgcagt agtaactcag tgttttattt 50
<210> 469
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 469
   ctgaagactc tgaacttgac tgaggaaatg ttaaacagat acctcttcat 50
<210> 470
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 470
   aacattccat tatcctattg ttcattcttt ggagctgtga tttgtttaat 50
<210> 471
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 471
   agcttcggtg aatattagaa tggcctcaag agctagtaaa aaacacagcc 50
<210> 472
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 472
   aggcatatgg ggaaaaaata aggcaggaaa ggaagacgga aaatgctgtg 50
<210> 473
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 473
   ttggttttat aaaggatcta agtgtttgga aaggtgtggg accatgtact 50
<210> 474
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 474
   acatgctctg catgctttga cagtacagtg tatagaatag acacaaaact 50
<210> 475
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 475
   taaggttgta tcatctacct gtagtcactg cagtcagctg aattttacca 50
<210> 476
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 476
   ctctgtagcc acacagatgc caacagctgg cacttgtcca agaaacatgt 50
<210> 477
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 477
   agaatgggtc acttgttaga aacagtcaag gatacataca aacagtggaa 50
<210> 478
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 478
   ccaagagtgg tgaagccttc ctgtttacag aggattttca tatctgttat 50
<210> 479
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 479
   acacccatgg ggccaagcca ggagcagtca ccacagccaa cctgcaggct 50
<210> 480
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 480
   tattctaagg aagtgccccc taaaacaaag ctcaggagcc tcaacccggc 50
<210> 481
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 481
   tcccaacatc aaaaggcaaa ttcttgcccc acttttacag atgagagcgc 50
<210> 482
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 482
   taccatggga aacagacagt ggcccctgtt ctcaagtggc ttagactcta 50
<210> 483
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 483
   cttattggcc ctaagtaaat cttaggttag gtagagctca gttcccaggg 50
<210> 484
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 484
   gtatttttag gaacattcag gaaaacaggt aaagggtatt caggaattca 50
<210> 485
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 485
   ggccttcctc actctgacgg tgagttccag aggacaggga tttgtggttg 50
<210> 486
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 486
   tggttgctaa tttctcttca cttctgggaa accagcccct tataaatcaa 50
<210> 487
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 487
   aacacagagc agtatgtaca ggacagcgtt agaatatacc agagaacaag 50
<210> 488
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 488
   aaacacacct gtcacccacg accctggcat agggcatcgt gaacccatca 50
<210> 489
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 489
   atagtattct gttcttcagg gagttgtggg ttcggatctg tgcaaagata 50
<210> 490
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 490
   taggaatcag ggaactctag atgcgtctag cagctagcct gtggcctcga 50
<210> 491
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 491
   ttcaaattgc ttgattaaaa tggcaaacag tttgaaaatt gtatacctct 50
<210> 492
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 492
   ggataatgga aaagggggtt tctcccaagt agagaactta aacagtgtga 50
<210> 493
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 493
   cacctagtca tgtgtatata aaatcaccat gttattacag aatttagtaa 50
<210> 494
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 494
   caatctattt tccacctggg ttctcgaacc gacttttcct ccctctcttc 50
<210> 495
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 495
   gggtcttcct acgggactgc cttagacgtg ctgggctttg gcctcagtga 50
<210> 496
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 496
   agttttggtt ggggaggaca atgccaggtt aacagacact taatatacat 50
<210> 497
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 497
   aaagagagtg gaagtaccag gtgggcaaag tttacaattt taagtaggat 50
<210> 498
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 498
   atgattcttt ccatgacacc tagtgccctt ctccatctag agctacctct 50
<210> 499
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 499
   aaatgaactc agcaatgaaa tggaacaagc tatccataca tgcagcaatt 50
<210> 500
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 500
   ccatcattgc ctggctgttg aagcagttct tgacatttta aagtaatatg 50
<210> 501
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 501
   ttgctacaag gaggattatg ggtgaaagtc atggatggat tatgagttaa 50
<210> 502
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 502
   gatggacatc actgaaatgt agttttgcct gaagtgtggt ttggatgctc 50
<210> 503
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 503
   cttgtttgtg tatgatacat gaagtagaat tcatacagca caagtacttt 50
<210> 504
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 504
   gaaattctcc ataatttctg atccactctt acattcctct cctttccagc 50
<210> 505
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 505
   gggggctggg gggaagtccc gggacaggtg catgtcatca acacgactgt 50
<210> 506
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 506
   agatcttttc aggcataaaa gttgtcaata ggttttcata aatttctagg 50
<210> 507
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 507
   cccttgcaca ggcacagcta taatttttgt ctctcttctg ttggaaaggt 50
<210> 508
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 508
   gtggtttcta atgatttaat accatccccc agggtgctct tcttgtgata 50
<210> 509
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 509
   gaatattgaa ggtagccaga aaagaaaaaa aggcacattg catgcagagg 50
<210> 510
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 510
   atggcagttc attgctttac tatttggaca tttcaaactg tcccaaggtg 50
<210> 511
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 511
   ttttttcaaa cctttaaaca acagtcccac ttggataaag tctgagagcg 50
<210> 512
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 512
   atagcctaac tttccccccg aagcttccca agccctcatg atatctatta 50
<210> 513
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 513
   acctgagaat tctcacccat ccaattctac ttgatatggg attcctctaa 50
<210> 514
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 514
   aatgggcatg atctcactca catggaacag gatctctttc cttgttagca 50
<210> 515
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 515
   agtcacagaa acatagcaag cccttgaaat caggctttct gactttgtct 50
<210> 516
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 516
   cacctacaca catgcatgca cacacacatg gcctctctct ccaggcttct 50
<210> 517
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 517
   cgtacagacc tggtccaaaa attccaattt cataggtgtg gagttttcat 50
<210> 518
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 518
   caaacaacca ccacatcaaa ataatagcaa agacaacaac taatactaat 50
<210> 519
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 519
   atagtaagtt ttaaagtaag aggtcagaaa catatgttac tttacaaaca 50
<210> 520
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 520
   ttatgtagca ggtcctgatg taacagaatt aagattgcag gtgggattgg 50
<210> 521
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 521
   tccctagaac agcaggacct gcgaaactct gaggccgctt tgtgaggtcc 50
<210> 522
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 522
   ttgaaaagag aaacccacag ggctttctgc ttaaatccct cggacacagt 50
<210> 523
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 523
   taaggatggg acccctactg tccatctcag gctcagcact gccttggggc 50
<210> 524
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 524
   cttctacatc ttagctcacc tgtcctcaca aataaacatc actcttgaat 50
<210> 525
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 525
   ttgttgaaat gtgaccacga actaggtctt aacctagcaa attcacaaat 50
<210> 526
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 526
   ctttctaaac actagcagcc cagaattctc aggccacttt tgggcattgt 50
<210> 527
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 527
   gtctatgaat tggtgaatca gccaagtgaa tgcttcaaaa actgggactc 50
<210> 528
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 528
   cctcctgaga tgaacatcgt gaggagtaaa tagagatgct attctcagct 50
<210> 529
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 529
   aactccgatt aatcactagt tgttcacacc aaaaacccaa gtgccattac 50
<210> 530
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 530
   tcaccaagtc tggttgtccc agtctcctat ctctgtctgt tcctctcctc 50
<210> 531
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 531
   atgagttgga attgcataat gggtagatgc tgatgctgga gaactttgag 50
<210> 532
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 532
   gtcattgact cgactataat tttccaaact acctaaacgt gttatatcat 50
<210> 533
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 533
   tgatgattag gagtctgatg gaggaaagta attttaaaac aacttaatgg 50
<210> 534
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 534
   tggggtttta tttgcttttt tcccagtttc ttagatgtaa agttaggtta 50
<210> 535
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 535
   ggaactctga cgcaatccag ggccgaggaa aaatgattaa aacccaacaa 50
<210> 536
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 536
   tactgcagtg agttcaagtg ttgtacctgc ttaaaatgca gtgacactaa 50
<210> 537
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 537
   ggcagaggga acagcttgtg caaaggccct ggggcaggcc aagggcagag 50
<210> 538
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 538
   aaaagaggat ggctggttta tctcaagtaa tcagacattt aataataata 50
<210> 539
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 539
   gtgctatttt gttgctgtta ggtctatttt cttcatctgt tatttcgcat 50
<210> 540
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 540
   gcctggggga gcggggaatc gcttttcgcc ggcctccgcg taaccttgtt 50
<210> 541
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 541
   ggctcaacgg aagtgaccgt cccacagtta tgcagcacta agtcaatggc 50
<210> 542
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 542
   ttgtgacagg tcccagcgtg aacacgcacg ccctagccgg gccccaaacc 50
<210> 543
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 543
   aaggggaccg caatggagga gcaaagaaga agaacttttt taaactgaac 50
<210> 544
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 544
   gctgacttct tgactgcagc cacaggaagg actcaaccca ggaccatcca 50
<210> 545
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 545
   aatttttcaa tggtaaacag accagagtta ttctaagaaa ttatgaaaag 50
<210> 546
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 546
   aggatttcaa gacttgcctg agcaacataa tgagatgccc tctctcaaaa 50
<210> 547
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 547
   agcaagcaga aaacaaacaa cttcattaaa aatgagcaga ggacctgaac 50
<210> 548
   <211> 50
   <212> DNA
   <213> Artificial Sequence
<220>
   <221> source
   <222> 1..50
   <223> /mol_type="unassigned DNA" /note="Synthetic" /organism="Artificial Sequence"
<400> 548
   ttctgagacc ttcttgcccc tttgtttcta agcccagggc cacaattccc 50

## Description

### BACKGROUND OF THE INVENTION

Corticotropin-releasing hormone (CRH or corticotropin-releasing factor / CRF) is pivotal in modulating the activity of the hypothalamic-pituitary-adrenal (HPA) axis during stress, stress-response and stress-adaptation, as well as in inflammation. CRH is a 41 aa peptide hormone derived from a 196-amino acid pre-prohormone, produced in the hypothalamus and transported in small vessels to the pituitary from which the peripheral stress hormone corticotropin (also known as adrenocorticotropic hormone / ACTH) is released which, in turn, induces secretion of cortisol from the adrenal gland. CRH containing nerve fibers also project to areas in the CNS implicated in behavioral adaptation to stress, including the amygdala, being implied in fear and anxiety, the prefrontal cortex and the hippocampus. Persistent stress is hypothesized to result in anxiety, depressive symptoms and other stress-related disorders in patients with inherited or acquired vulnerability. Among those patients, antagonists of CRH would appear to be the ideally tailored therapy. The effects of CRH in the brain, where CRH acts like a neurotransmitter, are conveyed via the type 1 CRH receptor (CRHR1, or CRF-R1), which mediates a variety of endocrine, behavioural, and autonomic stress-responses (Heinrichs and Koob, J Pharmacol Exp Ther. 2004 Nov;311(2):427-40), including, but not being limited to, psychiatric conditions such as anxiety disorders and major depression (Holsboer and Ising, Eur J Pharmacol 2008, 583(2-3):350-7; Koob and Zorilla, Neuropsychopharmacology 2012, 37(1):308-9). In murine models, CRHR1 deletions displayed less depression-related behaviors, while CRH overexpression in the CNS lead to an increase of several behaviors that can, within certain limitations, be extrapolated to human depression.

The World Health Organization (WHO) considers depression as one of the top ten causes of morbidity and mortality, with a lifetime prevalence for depression ranging, e.g., from 12-16% in Germany. Depressive disorders account for a worldwide number of over one million suicides annually, and create a significant burden on costs in health care, work leave, disability pension, early retirement, loss of productivity of workers, by far surmounting direct costs such as inpatient and outpatient treatments. Finally, depression also multiplies the risk for other conditions such as cardiovascular disease, diabetes and neurodegenerative disorders.

Significant effort has been focused on the development of inhibitors of neuropeptide receptor ligands as drugs for psychiatric diseases and related conditions, including CRHR1 antagonists for the treatment of anxiety and depression (Griebel and Holsboer, Nature Reviews Drug Discovery 2012, 11:462-478). However, essentially all randomized controlled trials using CRHR1 antagonists in humans produced negative results, which has lead several originators to stall CRHR1 antagonist development, see Williams, Expert Opin Ther Pat 2013, 23(8):1057-68.

The present invention rests in part on the recognition that several of these earlier trials testing CRHR1 antagonist only failed to show statistically relevant effects due to the lack of appropriate patient stratification and selection according to their individual, underlying pathophysiology. In other words, a CRHR1 antagonist can only be effective in pathologies where the underlying causality is dominated by CRH over-activity or excessive CRH secretion. In the absence of CRH over-activity, a CRHR1-antagonist is not likely to have any significant effect.

Methods and algorithms for predicting an ACTH response to CRHR1 antagonists using the dex/CRH test in patients with depressive symptoms and/or anxiety symptoms, as well as a set of genotypes of single nucleotide polymorphisms (SNPs) for use in such methods and algorithms, have been described in WO 2013/160315 (A2). Correspondingly, CRHR1 antagonists for use in the treatment of depressive symptoms and/or anxiety symptoms in patients having CRH over-activity have been described in WO 2013/160317 (A2), wherein CRH over-activity is detected by determining the status of the same set of genotypes of SNPs as in WO 2013/160315 (A2). However, there remains a need for improved methods of predicting the treatment response to CRHR1 antagonists. In particular, there is a strong need to provide a direct prediction of clinical response in subjects treated with a treatment with a CRHR1 antagonist, e.g., in subjects having depressive symptoms or anxiety symptoms, or another stress-related condition mediated by CRHR1. A particularly useful CRHR1 antagonist is SSR-125543 or a pharmaceutically acceptable salt thereof.

The present invention rests on additional evidence unknown in the prior art, according to which many polymorphisms are present in essentially all relevant nodes of the CRH/CRHR1 signaling chain. It is, thus, an object of the present invention to provide a method of treatment, wherein a particularly useful set of genomic DNA polymorphisms is used for predicting a central CRH over-activity and/or a clinical response to treatment with SSR-125543, in particular in, but not being limited to, patients with anxiety symptoms or depressive symptoms. Thus, the present invention provides improved methods of treatment comprising SSR-125543.

### SUMMARY OF THE INVENTION

The invention is defined by the appended claims. The present invention is based, at least in part, on the recognition of polymorphism genotypes, including, but not being limited to, single nucleotide polymorphism (SNP) genotypes that are predictive of a subject's clinical responsiveness or non-responsiveness to treatment with a corticotropin releasing hormone receptor type 1 (CRHR1) antagonist. Specifically, the presence or absence of one or more of the polymorphism genotypes disclosed in Table 2 herein can be used to predict the likelihood that a given subject will or will not respond to treatment with SSR-125543 or a pharmaceutically acceptable salt thereof. The set and subsets of polymorphism genotypes, compositions, and methods described herein are thus useful in selecting appropriate treatment modalities (e.g., a treatment with SSR-125543 or a non-CRHR1 antagonist) for a subject having a condition treatable by SSR-125543 or a pharmaceutically acceptable salt thereof.

Thus, in a first aspect, the invention provides a method of treating a condition which is treatable by SSR-125543 or a pharmaceutically acceptable salt thereof in a subject in need thereof, comprising administering an effective amount of SSR-125543 or a pharmaceutically acceptable salt thereof to the subject, wherein the subject has been predicted to respond, or has an increased likelihood of responding, to a treatment with SSR-125543 or a pharmaceutically acceptable salt thereof. In one embodiment, the method of treating comprises predicting a treatment response of a subject to treatment with SSR-125543 or a pharmaceutically acceptable salt thereof, wherein predicting comprises: providing a biological sample obtained from the subject, and detecting the presence or absence of one or more polymorphism genotypes in the biological sample, wherein the one or more polymorphism genotypes comprise: (a) at least one polymorphism genotype selected from the group consisting of rs34169260 (A/G), rs796287 (A/C), rs56149945 (A/G), rs6190 (T/C), rs7179092 (T/C), rs7614867 (A/G), rs920640 (T/C), rs7167722 (T/C), rs920638 (T/C), rs7165629 (T/C), rs80049044 (T/A), rs16941058 (A/G), rs112015971 (A/G), rs10894873 (T/C), rs117455294 (T/G), rs1170303 (T/C), rs16940681 (C/G), rs968519 (T/C), rs28381866 (T/C), rs79320848 (T/G), rs114653646 (T/G), rs2589496 (T/C), rs10482650 (A/G), rs17614642 (A/G), rs73200317 (T/C), rs1380146 (T/A), rs735164 (T/C), rs730976 (T/G), rs55934524 (T/G), rs4570614 (A/G), rs4458044 (C/G), rs77850169 (A/G), rs35339359 (A/G), rs34800935 (T/C), rs72945439 (T/C), rs113959523 (A/G), rs116798177 (A/G), rs11247577 (T/G), rs75869266 (T/C), rs74372553 (T/C), rs11691508 (A/G), rs6493965 (A/G), rs4869476 (T/C), rs3730170 (T/C), rs2145288 (A/C), rs2935752 (A/C), rs146512400 (A/G), rs62057097 (T/C), rs115061314 (T/C), rs34113594 (T/G), rs61751173 (A/G), rs74338736 (A/C), rs10851726 (T/C), rs4610906 (T/C), rs59485211 (T/C), rs7060015 (T/G), rs75710780 (T/G), rs6520908 (T/C), rs487011 (T/G), rs1383699 (A/C), rs67516871 (A/G), rs114106519 (T/C), rs7220091 (A/G), rs12489026 (A/G), rs876270 (T/C), rs4968161 (T/C), rs62056907 (A/G), rs2235013 (T/C), rs16878812 (A/G), rs6549407 (A/G), rs28381848 (A/G), rs79723704 (A/C), rs72814052 (A/G), rs10152908 (T/C), rs172769 (A/C), rs78596668 (T/C), rs73307922 (T/C), rs3842 (A/G), rs7210584 (A/C), rs62402121 (T/C), rs55709291 (A/G), rs72747088 (A/G), rs929610 (G/C), rs6766242 (T/C), rs1468552 (G/C), rs78838114 (T/C), rs62489862 (T/C), rs894342 (A/G), rs58882373 (T/C), rs3811939 (A/G), rs6984688 (T/G), rs1018160 (T/C), rs76602912 (A/G), rs80067508 (A/G), rs74888440 (T/C), rs12481583 (T/C), rs66794218 (A/G), rs16946701 (A/G), rs75726724 (A/G), rs67959715 (T/A), rs11871392 (T/G), rs2044070 (A/G), rs77612799 (T/C), rs6743702 (T/C), rs616870 (T/C), rs79590198 (A/G), rs75715199 (A/G), rs13087555 (T/C), rs4869618 (T/C), rs117397046 (A/G), rs8042817 (A/G), rs2258097 (T/C), rs2260882 (C/G), rs532996 (A/G), rs11747040 (T/C), rs10034039 (T/G), rs116909369 (A/G), rs79134986 (A/G), rs117615688 (T/C), rs8032253 (T/C), rs12818653 (T/A), rs4587884 (A/C), rs77122853 (T/C), rs117615061 (T/C), rs74682905 (A/G), rs2257468 (T/C), rs2032582 (T/G), rs2235015 (T/G), rs2729794 (T/C), rs77549514 (A/G), rs74790420 (A/C), rs73129579 (T/C), rs12913346 (A/C), rs117560908 (T/C), rs72747091 (A/G), rs2935751 (A/G), rs4331446 (A/G), rs7523266 (T/C), rs7648662 (T/C), rs117034065 (A/G), rs4836256 (T/C), rs80238698 (T/C), rs3730173 (T/C), rs11687884 (T/C), rs72693005 (T/C), rs2589476 (T/C), rs9813396 (T/C), rs10482667 (A/G), rs72784444 (A/G), rs75074511 (T/C), rs7951003 (A/G), rs79584784 (A/G), rs2214102 (T/C), rs28811003 (A/G), rs6100261 (A/T), rs77152456 (A/G), rs66624622 (T/G), rs140302965 (A/G), rs11653269 (T/C), rs74405057 (A/G), rs7121 (A/G), rs16977818 (A/C), rs12490095 (T/C), rs118003903 (A/G), rs62377761 (T/C),P1_M_061510_6_34_M (-/CACTTACCTTCTTTGTGCCACAGTTTCCCTATCTAAAACACAAGGTTATCAGTTATCAACATCTCTTGGGATTGTGAGGACTAAAGTAATGCACATAAAG), rs375115639 (-/AAATTACCCTGTTAGGTTTCAATGAAACACCTTTTCTCTTGTAACA AACATCTCCTCC AAGCTAGAATTTCAAAACAG), rs1002204 (A/C), rs10062367 (A/G), rs10482642 (A/G), rs10482658 (A/G), rs1053989 (A/C), rs10851628 (T/C), rs10947562 (T/C), rs11069612 (A/G), rs11071351 (T/C), rs11091175 (A/G), rs11638450 (T/C), rs11715827 (T/G), rs11745958 (T/C), rs11834041 (A/G), rs1202180 (T/C), rs12054781 (A/G), rs12539395 (A/G), rs12720066 (T/G), rs1279754 (A/C), rs12872047 (T/C), rs12876742 (A/C), rs12917505 (A/G), rs13066950 (T/G), rs13229143 (C/G), rs1383707 (T/C), rs1441824 (T/C), rs1652311 (A/G), rs17064 (T/A), rs17100236 (A/G), rs17149699 (A/G), rs1724386 (A/G), rs17250255 (A/G), rs17327624 (T/G), rs17616338 (A/G), rs17687796 (A/G), rs17740874 (T/C), rs17763104 (T/C), rs1880748 (T/C), rs1882478 (A/G), rs1944887 (T/C), rs2028629 (A/G), rs2143404 (A/G), rs2173530 (T/C), rs220806 (T/C), rs2235047 (A/C), rs2242071 (A/G), rs2257474 (T/C), rs2295583 (A/T), rs234629 (T/C), rs234630 (A/G), rs2436401 (A/G), rs258750 (T/C), rs2589487 (T/C), rs28364018 (T/G), rs28381774 (T/C), rs28381784 (A/G), rs2963155 (A/G), rs3133622 (T/G), rs32897 (T/C), rs33388 (A/T), rs3730168 (T/C), rs3735833 (T/G), rs3777747 (A/G), rs3786066 (T/C), rs3798346 (T/C), rs3822736 (A/G), rs389035 (T/C), rs3924144 (A/G), rs4148737 (T/C), rs4148749 (G/C), rs417968 (T/C), rs4458144 (T/C), rs4515335 (T/C), rs4728699 (A/G), rs4758040 (A/G), rs4812040 (A/G), rs4912650 (T/G), rs4957891 (T/C), rs5906392 (A/G), rs6026561 (T/C), rs6026565 (T/A), rs6026567 (A/G), rs6026593 (A/G), rs6092704 (T/G), rs6100260 (A/G), rs6128461 (T/C), rs6415328 (T/C), rs6610868 (T/C), rs6686061 (A/C), rs6730350 (T/G), rs6746197 (T/C), rs6963426 (T/C), rs7121326 (T/C), rs7721799 (A/G), rs7787082 (T/C), rs7799592 (A/C), rs796245 (T/C), rs809482 (A/C), rs8125112 (T/C), rs919196 (A/G), rs920750 (T/C), rs9332385 (A/G), rs930473 (T/G), rs9324921 (A/C), rs9348979 (A/G), rs9571939 (A/C), and rs9892359 (T/C); (b) at least one polymorphism genotype being in linkage disequilibrium with any one of the polymorphism genotypes of (a); or (c) a combination of (a) and (b); and predicting the treatment response from the presence or absence of the one or more polymorphism genotypes of (a), (b), or (c).

In another embodiment, the method of treating comprises detecting a polymorphism genotype associated with a treatment response of a subject to treatment with SSR-125543 or a pharmaceutically acceptable salt thereof, the detecting comprising providing a biological sample obtained from the subject, and detecting the presence or absence of one or more polymorphism genotypes in the biological sample, wherein the one or more polymorphism genotypes comprise: (a) at least one polymorphism genotype selected from the group consisting of rs34169260 (A/G), rs796287 (A/C), rs56149945 (A/G), rs6190 (T/C), rs7179092 (T/C), rs7614867 (A/G), rs920640 (T/C), rs7167722 (T/C), rs920638 (T/C), rs7165629 (T/C), rs80049044 (T/A), rs16941058 (A/G), rs112015971 (A/G), rs10894873 (T/C), rs117455294 (T/G), rs1170303 (T/C), rs16940681 (C/G), rs968519 (T/C), rs28381866 (T/C), rs79320848 (T/G), rs114653646 (T/G), rs2589496 (T/C), rs10482650 (A/G), rs17614642 (A/G), rs73200317 (T/C), rs1380146 (T/A), rs735164 (T/C), rs730976 (T/G), rs55934524 (T/G), rs4570614 (A/G), rs4458044 (C/G), rs77850169 (A/G), rs35339359 (A/G), rs34800935 (T/C), rs72945439 (T/C), rs113959523 (A/G), rs116798177 (A/G), rs11247577 (T/G), rs75869266 (T/C), rs74372553 (T/C), rs11691508 (A/G), rs6493965 (A/G), rs4869476 (T/C), rs3730170 (T/C), rs2145288 (A/C), rs2935752 (A/C), rs146512400 (A/G), rs62057097 (T/C), rs115061314 (T/C), rs34113594 (T/G), rs61751173 (A/G), rs74338736 (A/C), rs10851726 (T/C), rs4610906 (T/C), rs59485211 (T/C), rs7060015 (T/G), rs75710780 (T/G), rs6520908 (T/C), rs487011 (T/G), rs1383699 (A/C), rs67516871 (A/G), rs114106519 (T/C), rs7220091 (A/G), rs12489026 (A/G), rs876270 (T/C), rs4968161 (T/C), rs62056907 (A/G), rs2235013 (T/C), rs16878812 (A/G), rs6549407 (A/G), rs28381848 (A/G), rs79723704 (A/C), rs72814052 (A/G), rs10152908 (T/C), rs172769 (A/C), rs78596668 (T/C), rs73307922 (T/C), rs3842 (A/G), rs7210584 (A/C), rs62402121 (T/C), rs55709291 (A/G), rs72747088 (A/G), rs929610 (G/C), rs6766242 (T/C), rs1468552 (G/C), rs78838114 (T/C), rs62489862 (T/C), rs894342 (A/G), rs58882373 (T/C), rs3811939 (A/G), rs6984688 (T/G), rs1018160 (T/C), rs76602912 (A/G), rs80067508 (A/G), rs74888440 (T/C), rs12481583 (T/C), rs66794218 (A/G), rs16946701 (A/G), rs75726724 (A/G), rs67959715 (T/A), rs11871392 (T/G), rs2044070 (A/G), rs77612799 (T/C), rs6743702 (T/C), rs616870 (T/C), rs79590198 (A/G), rs75715199 (A/G), rs13087555 (T/C), rs4869618 (T/C), rs117397046 (A/G), rs8042817 (A/G), rs2258097 (T/C), rs2260882 (C/G), rs532996 (A/G), rs11747040 (T/C), rs10034039 (T/G), rs116909369 (A/G), rs79134986 (A/G), rs117615688 (T/C), rs8032253 (T/C), rs12818653 (T/A), rs4587884 (A/C), rs77122853 (T/C), rs117615061 (T/C), rs74682905 (A/G), rs2257468 (T/C), rs2032582 (T/G), rs2235015 (T/G), rs2729794 (T/C), rs77549514 (A/G), rs74790420 (A/C), rs73129579 (T/C), rs12913346 (A/C), rs117560908 (T/C), rs72747091 (A/G), rs2935751 (A/G), rs4331446 (A/G), rs7523266 (T/C), rs7648662 (T/C), rs117034065 (A/G), rs4836256 (T/C), rs80238698 (T/C), rs3730173 (T/C), rs11687884 (T/C), rs72693005 (T/C), rs2589476 (T/C), rs9813396 (T/C), rs10482667 (A/G), rs72784444 (A/G), rs75074511 (T/C), rs7951003 (A/G), rs79584784 (A/G), rs2214102 (T/C), rs28811003 (A/G), rs6100261 (A/T), rs77152456 (A/G), rs66624622 (T/G), rs140302965 (A/G), rs11653269 (T/C), rs74405057 (A/G), rs7121 (A/G), rs16977818 (A/C), rs12490095 (T/C), rs118003903 (A/G), rs62377761 (T/C), P1_M_061510_6_34_M (-/CACTTACCTTCTTTGTGCCACAGTTTCCCTATCTAAAACACAAGGTTATCAGTTATC AACATCTCTTGGGATTGTGAGGACTAAAGTAATGCACATAAAG), rs375115639 (-/AAATTACCCTGTTAGGTTTCAATGAAACACCTTTTCTCTTGTAACAAACATCTCCTCCA AGCTAGAATTTCAAAACAG), rs1002204 (A/C), rs10062367 (A/G), rs10482642 (A/G), rs10482658 (A/G), rs1053989 (A/C), rs10851628 (T/C), rs10947562 (T/C), rs11069612 (A/G), rs11071351 (T/C), rs11091175 (A/G), rs11638450 (T/C), rs11715827 (T/G), rs11745958 (T/C), rs11834041 (A/G), rs1202180 (T/C), rs12054781 (A/G), rs12539395 (A/G), rs12720066 (T/G), rs1279754 (A/C), rs12872047 (T/C), rs12876742 (A/C), rs12917505 (A/G), rs13066950 (T/G), rs13229143 (C/G), rs1383707 (T/C), rs1441824 (T/C), rs1652311 (A/G), rs17064 (T/A), rs17100236 (A/G), rs17149699 (A/G), rs1724386 (A/G), rs17250255 (A/G), rs17327624 (T/G), rs17616338 (A/G), rs17687796 (A/G), rs17740874 (T/C), rs17763104 (T/C), rs1880748 (T/C), rs1882478 (A/G), rs1944887 (T/C), rs2028629 (A/G), rs2143404 (A/G), rs2173530 (T/C), rs220806 (T/C), rs2235047 (A/C), rs2242071 (A/G), rs2257474 (T/C), rs2295583 (A/T), rs234629 (T/C), rs234630 (A/G), rs2436401 (A/G), rs258750 (T/C), rs2589487 (T/C), rs28364018 (T/G), rs28381774 (T/C), rs28381784 (A/G), rs2963155 (A/G), rs3133622 (T/G), rs32897 (T/C), rs33388 (A/T), rs3730168 (T/C), rs3735833 (T/G), rs3777747 (A/G), rs3786066 (T/C), rs3798346 (T/C), rs3822736 (A/G), rs389035 (T/C), rs3924144 (A/G), rs4148737 (T/C), rs4148749 (G/C), rs417968 (T/C), rs4458144 (T/C), rs4515335 (T/C), rs4728699 (A/G), rs4758040 (A/G), rs4812040 (A/G), rs4912650 (T/G), rs4957891 (T/C), rs5906392 (A/G), rs6026561 (T/C), rs6026565 (T/A), rs6026567 (A/G), rs6026593 (A/G), rs6092704 (T/G), rs6100260 (A/G), rs6128461 (T/C), rs6415328 (T/C), rs6610868 (T/C), rs6686061 (A/C), rs6730350 (T/G), rs6746197 (T/C), rs6963426 (T/C), rs7121326 (T/C), rs7721799 (A/G), rs7787082 (T/C), rs7799592 (A/C), rs796245 (T/C), rs809482 (A/C), rs8125112 (T/C), rs919196 (A/G), rs920750 (T/C), rs9332385 (A/G), rs930473 (T/G), rs9324921 (A/C), rs9348979 (A/G), rs9571939 (A/C), and rs9892359 (T/C); (b) at least one polymorphism genotype being in linkage disequilibrium with any one of the polymorphism genotypes of (a); or (c) a combination of (a) and (b). In one embodiment, the method further comprises predicting the treatment response from the presence or absence of the polymorphism genotypes of (a), (b), or (c).

In another aspect, SSR-125543 or a pharmaceutically acceptable salt thereof for use in treating a condition which is treatable by a CRHR1 antagonist in a subject in need thereof is provided, wherein the subject has been predicted to respond, or has an increased likelihood of responding, to treatment with SSR-125543 or a pharmaceutically acceptable salt thereof, as determined by the step of predicting a treatment response described above. In another aspect, SSR-125543 or a pharmaceutically acceptable salt thereof for use in treating a condition which is treatable by a CRHR1 antagonist in a subject in need thereof is provided, wherein the subject has been predicted to respond, or has an increased likelihood of responding, to treatment with SSR-125543 or a pharmaceutically acceptable salt thereof, as determined by the step of detecting a polymorphism genotype associated with a treatment response of a subject to treatment with SSR-125543 or a pharmaceutically acceptable salt thereof.

The above aspects of the invention can be put into practice in any one of the following embodiments.

In one embodiment, providing a biological sample comprises extraction and/or purification of nucleic acids such as DNA or RNA, in particular genomic DNA from the subject's sample. In one embodiment, the detecting step can comprise amplification of nucleic acids extracted and/or purified from the sample obtained from the subject, and optionally clean-up of amplified products. The detecting step can further comprise fragmentation of amplified nucleic acids, or labelling of amplified nucleic acids.

In one embodiment, the detecting step can further comprise specific hybridization of at least one polynucleotide to a nucleic acid comprising at least one polymorphism genotype selected from the group disclosed in Table 2 herein. Hybridization can be achieved by mixing and heating the at least one polynucleotide and the sample nucleic acid to a temperature at which denaturation occurs, e.g., at about 90-95°C and subsequent incubation at a temperature at which hybridization occurs, e.g., at about 45-55°C in buffer conditions suitable for specific hybridization. In one embodiment the polynucleotide is labelled. The polynucleotide can be a primer or probe. Specifically, in some embodiments, the detecting step comprises a method selected from the group consisting of allele-specific oligonucleotide (ASO)-dot blot analysis, primer extension assays, iPLEX polymorphism / SNP genotyping, dynamic allele-specific hybridization (DASH) genotyping, the use of molecular beacons, tetra primer ARMS PCR, a flap endonuclease invader assay, an oligonucleotide ligase assay, PCR-single strand conformation polymorphism (SSCP) analysis, quantitative real-time PCR assay, polymorphism / SNP microarray based analysis, restriction enzyme fragment length polymorphism (RFLP) analysis, targeted resequencing analysis and/or whole genome sequencing analysis.

In one embodiment, the predicting step comprises: (a) determining whether the subject will respond, or has an increased likelihood of responding to the treatment with SSR-125543 or a pharmaceutically acceptable salt thereof; and/or (b) determining whether the subject will not respond, or has a decreased likelihood of responding to the treatment with SSR-125543 or a pharmaceutically acceptable salt thereof. The determining step may further comprise, but is not limited to, one or more statistical analysis methods selected from the group consisting of artificial neural network learning, decision tree learning, decision tree forest learning, linear discriminant analysis, non-linear discriminant analysis, genetic expression programming, relevance vector machines, linear models, generalized linear models, generalized estimating equations, generalized linear mixed models, the elastic net, the lasso support vector machine learning, Bayesian network learning, probabilistic neural network learning, clustering, and regression analysis. The predicting step may also comprise providing a value indicative of the subject being responsive, or having an increased likelihood of responding to the treatment with SSR-125543 or a pharmaceutically acceptable salt thereof; and/or providing a value indicative of the subject being non-responsive, or having a decreased likelihood of responding to the treatment with SSR-125543 or a pharmaceutically acceptable salt thereof.

In one embodiment, the one or more polymorphism genotypes comprise at least two, at least three, at least four, at least five, at least six, at least seven, at least eight, at least nine, at least ten, at least 15, at least 20, at least 30, at least 40, at least 50, at least 60, at least 70, at least 80, at least 90, at least 100, at least 200, or all (a) polymorphism genotypes selected from the group consisting of the polymorphism genotypes disclosed in Table 2, (b) polymorphism genotypes being in linkage disequilibrium with the polymorphism genotypes disclosed in Table 2; or (c) a combination of (a) and (b).

In a specific embodiment, the one or more polymorphism genotypes comprise (a) at least two polymorphism genotypes selected from the group consisting of the polymorphism genotypes disclosed in Table 2, (b) at least two polymorphism genotypes being in linkage disequilibrium with the polymorphism genotypes disclosed in Table 2; or (c) a combination of (a) and (b). Exemplary sets of at least two polymorphism genotypes useful in the methods of the invention are disclosed in Table 5. Therefore, the specific combinations of at least two polymorphism genotypes disclosed in Table 5 are used in specific embodiments of the invention, while further combinations of at least two polymorphism genotypes are expressly contemplated.

In another specific embodiment, the one or more polymorphism genotypes comprise (a) at least four polymorphism genotypes selected from the group consisting of the polymorphism genotypes disclosed in Table 2, (b) at least four polymorphism genotypes being in linkage disequilibrium with the polymorphism genotypes disclosed in Table 2, or (c) a combination of (a) and (b). Exemplary sets of at least four polymorphism genotypes useful in the methods of the invention are disclosed in Table 6. Therefore, the specific combinations of at least four polymorphism genotypes disclosed in Table 6 are used in specific embodiments of the invention, while further combinations of at least four polymorphism genotypes are expressly contemplated.

In another specific embodiment, the one or more polymorphism genotypes comprise (a) at least eight polymorphism genotypes selected from the group consisting of the polymorphism genotypes disclosed in Table 2, (b) at least eight polymorphism genotypes being in linkage disequilibrium with the polymorphism genotypes disclosed in Table 2, or (c) a combination of (a) and (b). Exemplary sets of at least eight polymorphism genotypes useful in the methods of the invention are shown in Table 7. Therefore, the specific combinations of at least eight polymorphism genotypes disclosed in Table 7 are used in specific embodiments of the invention, while further combinations are expressly contemplated.

In another embodiment, the one or more polymorphism genotypes comprise (a) at least 16 polymorphism genotypes selected from the group consisting of the polymorphism genotypes disclosed in Table 2, (b) at least 16 polymorphism genotypes being in linkage disequilibrium with the polymorphism genotypes disclosed in Table 2, or (c) a combination of (a) and (b). In another embodiment, the one or more polymorphism genotypes comprise (a) at least 32 polymorphism genotypes selected from the group consisting of the polymorphism genotypes disclosed in Table 2, (b) at least 16 polymorphism genotypes being in linkage disequilibrium with the polymorphism genotypes disclosed in Table 2, or (c) a combination of (a) and (b). In another embodiment, the one or more polymorphism genotypes comprise at least 150 polymorphism genotypes selected from the group consisting of the polymorphism genotypes disclosed in Table 2, (b) at least 16 polymorphism genotypes being in linkage disequilibrium with the polymorphism genotypes disclosed in Table 2, or (c) a combination of (a) and (b). In another embodiment, the one or more polymorphism genotypes comprise all polymorphism genotypes disclosed in Table 2.

In some embodiments, the method can include detecting the presence or absence of (a) one or more of the polymorphism genotypes disclosed in Tables 2, 5, 6, or 7, (b) one or more polymorphism genotypes being in linkage disequilibrium with the polymorphism genotypes disclosed in Tables 2, 5, 6, or 7, or (c) a combination of (a) and (b), predicting that the subject will respond, or is likely to respond to treatment with SSR-125543 or a pharmaceutically acceptable salt thereof and selecting a treatment with a CRHR1 agent for the subject. The method can further include administering SSR-125543 or a pharmaceutically acceptable salt thereof to the subject.

In some embodiments, the predicting step can include creating a record indicating that the subject will respond, or is likely to respond to treatment with SSR-125543 or a pharmaceutically acceptable salt thereof. The record can be created on a computer readable medium.

In some embodiments, the method can include detecting the presence or absence of (a) one or more of any of the polymorphism genotypes disclosed in Tables 2, 5, 6 or 7, (b) one or more polymorphism genotypes being in linkage disequilibrium with the polymorphism genotypes disclosed in Tables 2, 5, 6, or 7, or (c) a combination of (a) and (b), predicting that the subject will not respond, or is not likely to respond to a treatment with SSR-125543 or a pharmaceutically acceptable salt thereof and selecting a treatment with treatment with a non-CRHR1 antagonist for the subject. The method can further include administering the treatment with the non-CRHR1 antagonist to the subject.

In some embodiments, the method can include creating a record indicating that the subject will not respond, or is not likely to respond to a treatment with SSR-125543 or a pharmaceutically acceptable salt thereof. The record can be created on a computer readable medium.

In one embodiment, the subject is a mammal. Preferably, in all aspects of the invention, the subject is human.

In one embodiment, the subject has a condition which is treatable by a treatment with SSR-125543 or a pharmaceutically acceptable salt thereof, as described herein. The condition can be characterized, caused or accompanied by CRH overproduction or over-activity. The condition can be characterized, caused or accompanied by ACTH overproduction or over-activity. The condition can be characterized, caused or accompanied by over-activity of the Hypothalamic-pituitary-adrenal (HPA) axis.

In another embodiment, the subject has and/or the treatment is a treatment of a condition selected from the group consisting of anxiety symptoms, generalized anxiety disorder, panic, phobias, obsessive-compulsive disorder, post-traumatic stress disorder, sleep disorders such as insomnia, hypersomnia, narcolepsy, idiopathic hypersomnia, excessive amounts of sleepiness, lack of alertness, lack of attentiveness, absentmindedness and/or lack of or aversion to movement or exercise, sleep disorders induced by stress, pain perception such as fibromyalgia, mood disorders such as depressive symptoms, including major depression, single episode depression, recurrent depression, child abuse induced depression, mood disorders associated with premenstrual syndrome, and postpartum depression, dysthymia, bipolar disorders, cyclothymia, chronic fatigue syndrome, stress-induced headache, eating disorders such as anorexia and bulimia nervosa, hemorrhagic stress, stress-induced psychotic episodes, endocrine disorders involving ACTH overproduction, ACTH over-activity, e.g., adrenal disorders, including, but not limited to congenital adrenal hyperplasia (CAH), euthyroid sick syndrome, syndrome of inappropriate antidiarrhetic hormone (ADH), obesity, infertility, head traumas, spinal cord trauma, ischemic neuronal damage (e.g., cerebral ischemia such as cerebral hippocampal ischemia), excitotoxic neuronal damage, epilepsy, senile dementia of the Alzheimers type, multi-infarct dementia, amyotrophic lateral sclerosis, chemical dependencies and addictions (e.g., dependencies on alcohol, nicotine, cocaine, heroin, benzodiazepines, or other drugs), drug and alcohol withdrawal symptoms, hypertension, tachycardia, congestive heart failure, osteoporosis, premature birth, and hypoglycaemia, inflammatory disorders such as rheumatoid arthritis and osteoarthritis, pain, asthma, psoriasis and allergies, irritable bowel syndrome, Crohn's disease, spastic colon, post-operative ileus, ulcer, diarrhea, stress-induced fever, human immunodeficiency virus (HIV) infections, neurodegenerative diseases such as Alzheimer's disease, Parkinson's disease and Huntington's disease, gastrointestinal diseases, stroke, stress induced immune dysfunctions, muscular spasms, urinary incontinence.

In a specific embodiment, the subject has and/or the treatment is a treatment of depressive symptoms, anxiety symptoms or both depressive symptoms and anxiety symptoms. In another specific embodiment, the subject has and/or the treatment is a treatment of depressive disorder, anxiety disorder or both depressive disorder and anxiety disorder. In another specific embodiment, the subject has and/or the treatment is a treatment of a sleep disorder.

In contrast to the prior art, the present invention identifies sets of polymorphisms indicative of a clinical response in subjects which are in need of a treatment with SSR-125543 or a pharmaceutically acceptable salt thereof. Therefore, in all aspects of the invention, the treatment response to treatment with SSR-125543 or a pharmaceutically acceptable salt thereof is preferably a clinical response. Generally, the clinical response can be a prevention, alteration, alleviation or complete remission of a clinical parameter in any of the above conditions. In particular, the clinical response can be a prevention, alteration, alleviation or complete remission of depressive symptoms and/or anxiety symptoms, or a decrease in adverse effects resulting from the treatment.

In some embodiments, the clinical response is a prevention, alteration, alleviation or complete remission of depressive symptoms, as determined using a scale selected from the group consisting of the Hamilton Depression Rating Scale (HAM-D), the Beck Depression Inventory (BDI), the Montgomery-Asberg Depression Scale (MADRS), the Geriatric Depression Scale (GDS), and/or the Zung Self-Rating Depression Scale (ZSRDS).

In some embodiments, the clinical response is a prevention, alteration, alleviation or complete remission of anxiety symptoms, as determined using a scale selected from the group consisting of Hamilton Anxiety Rating Scale (HAM-A) and/or the State-Trait Anxiety Rating Scale (STAI).

Any of the methods described herein can further include a step of prescribing a treatment with SSR-125543 or a pharmaceutically acceptable salt thereof or non-CRHR1 antagonist (the choice of which depends upon the outcome of the predictive methods described herein) for the subject.

In all aspects, the sample obtained from the subject can comprise any type of cells containing genomic DNA. Specifically, the sample can be, e.g., a buccal sample, a blood sample, a tissue sample, a formalin-fixed, paraffin-embedded tissue sample, or a hair follicle.

In all embodiments of the invention, the CRHR1 antagonist is SSR-12554.

### BRIEF DESCRIPTION OF THE DRAWINGS

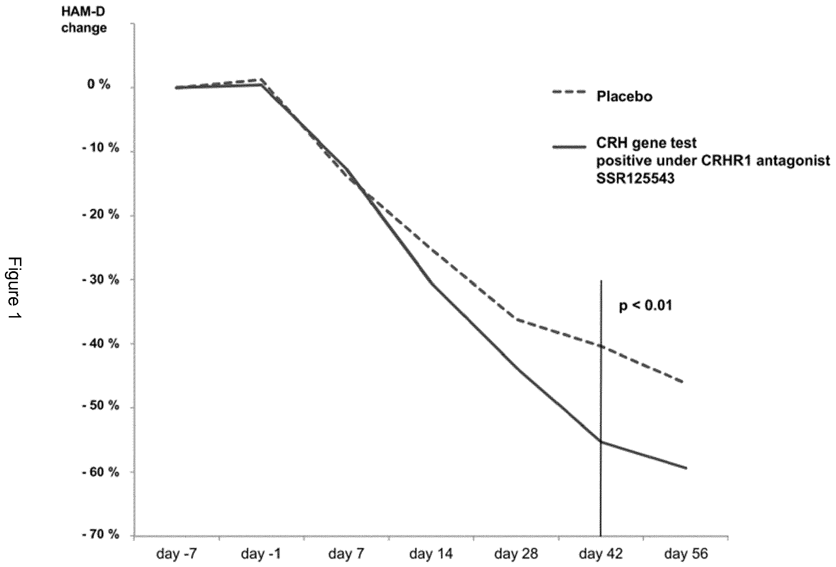

Figure 1 shows a time course curve of the clinical response of depressive patients as measured by the HAM-D scale upon treatment using placebo, or using SSR-125543, wherein the surveyed subjects were predicted to positively respond to CRHR1 antagonist treatment using the method of prediction. The dashed line indicates a significant effect in treatment response at day 42 (p-value < 0.01).

### DETAILED DESCRIPTION OF THE INVENTION

### General definitions

The term "comprise" or "comprising" as used herein is to be construed as "containing" or "including" and does generally not exclude other elements or steps, but encompasses the term "consisting of" as an optional, specific embodiment. Thus, a group defined as comprising a certain number of embodiments, is also to be construed as a disclosure of a group which optionally consists only of these embodiments. Where an indefinite or a definite article is used when referring to a singular noun such as "a" or "an" or "the", it includes a plural form of that noun unless specifically stated. *Vice versa,* when the plural form of a noun is used it refers also to the singular form. For example, when polymorphism genotypes are mentioned, this is also to be understood as a single polymorphism genotype.

Furthermore, the terms "first", "second", "third" or "(a)", "(b)", "(c)" and the like in the description and in the claims are used for distinguishing between elements and not necessarily for describing a sequential or chronological order. It is to be understood that the terms so used can be interchangeable under appropriate circumstances and that the embodiments of the invention described herein are capable of operation in other sequences than described or illustrated herein.

"Corticotropin releasing hormone" or "CRH" is used synonymously to the term "corticotropin releasing factor" or "CRF" herein, and refers to the known human 41 aa peptide or its mammalian homologues. The term "corticotropin releasing hormone receptor 1" or "CRHR1" refers to the receptor which binds to CRH and is used synonymously to the term "corticotropin-releasing factor receptor 1", or CRF-R1, or CRFR-1 herein.

A "CRHR1 antagonist", as used herein, refers to SSR-125543 or a pharmaceutically acceptable salt thereof, a compound capable of binding directly or indirectly to CRHR1 so as to modulate the receptor mediated activity. SSR-12554, as used herein, is used synonymous to SSR-125543A, 4-(2-chloro-4-methoxy-5-methylphenyl)-N(2-cyclopropyl-1-(3-fluoro-4-methyl phenyl)ethyl)-5-methyl-N-(2-propynyl)-1,3-thiazol-2-amine, or specifically 4-(2-chloro-4-methoxy-5-methylphenyl)-N-[(1S)-2-cyclopropyl-1-(3-fluoro-4-methylphenyl)ethyl]-5-methyl -N-prop-2-ynyl-1,3-thiazol-2-amine and encompasses any pharmaceutically acceptable salt thereof. The CRHR1 mediated activity may be exerted on a downstream target within the signalling pathway of CRHR1. A "downstream target" may refer to a molecule such as an endogenous molecule (e.g. peptide, protein, lipid, nucleic acid or oligonucleotide), that is regulated by CRHR1 directly or indirectly, comprising direct or indirect modulation of the activity and/or expression level and/or localization, degradation or stability of the downstream target. SSR-125543 is shown in the following table.

**Table 1 -SSR-125543**

| Structure | Name (synonym) |
|---|---|
| 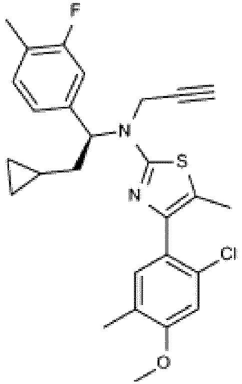 | SSR-125543 |

### Methods of Treatment

In one aspect, the present invention provides a method of treating a condition which is treatable by SSR-125543 or a pharmaceutically acceptable salt thereof in a subject in need thereof, comprising administering an effective amount of SSR-125543 or a pharmaceutically acceptable salt thereof to the subject, wherein the subject has been predicted to respond, or has an increased likelihood of responding, to a treatment with SSR-125543 or a pharmaceutically acceptable salt thereof. The method of treating can comprise the step of predicting a treatment response of a subject (such as a human patient) to a treatment with SSR-125543 or a pharmaceutically acceptable salt thereof.

Specifically, predicting a treatment response of a subject to treatment with SSR-125543 or a pharmaceutically acceptable salt thereof, comprises providing a biological sample obtained from the subject, detecting the presence or absence of one or more polymorphism genotypes in the biological sample, wherein the one or more polymorphism genotypes comprise: (a) at least one polymorphism genotype selected from the group consisting of the polymorphism genotypes disclosed in Table 2, (b) at least one polymorphism genotype being in linkage disequilibrium with any one of the polymorphism genotypes of (a); or (c) a combination of (a) and (b), and predicting the treatment response from the presence or absence of the one or more polymorphism genotypes of (a), (b), or (c).

A "subject", as used herein, can generally be any mammal, in which one or more polymorphism genotypes as disclosed in Table 2 or polymorphism genotypes being in linkage disequilibrium with any one of the polymorphism genotypes of Table 2 are conserved or homologous. In particular, the term "subject" includes a human subject, and any model organism such as mice, rats, cats, dogs, simians, cattle. Preferably, the subject is a human subject.

A "treatment with SSR-125543 or a pharmaceutically acceptable salt thereof", as used herein, refers to the treatment of a condition in the subject which can be treated by administration of SSR-125543 or a pharmaceutically acceptable salt thereof, as is made plausible herein or in the prior art. "Conditions treatable with SSR-125543 or a pharmaceutically acceptable salt thereof", as used herein, are conditions which can generally be treated by administration of SSR-125543 or a pharmaceutically acceptable salt thereof and/or are commonly characterized, caused or accompanied by CRH over-activity, by ACTH over-activity and/or by over-activity of the Hypothalamic-pituitary-adrenal (HPA) axis.

The term "CRH over-activity" is used herein synonymously to the terms "CRH system over-activity", "CRH hyperactivity", "CRH hyperdrive" or "central CRH hyperdrive". An indication for CRH over-activity may be an increase in activity or concentration of CRH or of one or several molecules downstream of the CRHR1 receptor, that are activated or whose concentration is increased based on the activation of CRHR1 receptor upon CRH binding, for instance, but not being limited to, ACTH. A further indication for CRH over-activity may be a decrease in activity or concentration of one or several molecules downstream of the CRHR1 receptor, that are inactivated or whose concentration is decreased resulting from the activation of CRHR1 receptor upon CRH binding. For instance, the concentrations or activities of adrenocorticotrophin (ACTH) and/or cortisol can be used for determining a value indicative for CRH over-activity. The CRH over-activity in each patient may be determined by a CRH test as described in Holsboer et al., N Engl J Med. 1984;311(17):1127, or by a combined dexamethasone suppression/CRH stimulation test (dex/CRH test) as described in Heuser et al., J Psychiatr Res 1994, 28(4):341-56.

In particular, conditions which can be treated using SSR-125543 or a pharmaceutically acceptable salt thereof in a subject comprise, but are not limited to, behavioural disorders, neuropsychiatric disorders, mood disorders, neurological disorders, neurodegenerative disorders, endocrine disorders, inflammatory or stress-induced immune disorders, CRH-related cardiovascular diseases or metabolic diseases. Specifically, such conditions comprise anxiety symptoms, generalized anxiety disorder, panic, phobias, obsessive-compulsive disorder, post-traumatic stress disorder, sleep disorders such as insomnia, hypersomnia, narcolepsy, idiopathic hypersomnia, excessive amounts of sleepiness, lack of alertness, lack of attentiveness, absentmindedness and/or lack of or aversion to movement or exercise, sleep disorders induced by stress, pain perception such as fibromyalgia, mood disorders such as depressive symptoms, including major depression, single episode depression, recurrent depression, child abuse induced depression, mood disorders associated with premenstrual syndrome, and postpartum depression, dysthymia, bipolar disorders, cyclothymia, chronic fatigue syndrome, stress-induced headache, eating disorders such as anorexia and bulimia nervosa, hemorrhagic stress, stress-induced psychotic episodes, endocrine disorders involving ACTH overproduction, ACTH over-activity, e.g., adrenal disorders, including, but not limited to congenital adrenal hyperplasia (CAH), euthyroid sick syndrome, syndrome of inappropriate antidiarrhetic hormone (ADH), obesity, infertility, head traumas, spinal cord trauma, ischemic neuronal damage (e.g., cerebral ischemia such as cerebral hippocampal ischemia), excitotoxic neuronal damage, epilepsy, senile dementia of the Alzheimers type, multi-infarct dementia, amyotrophic lateral sclerosis, chemical dependencies and addictions (e.g., dependencies on alcohol, nicotine, cocaine, heroin, benzodiazepines, or other drugs), drug and alcohol withdrawal symptoms, hypertension, tachycardia, congestive heart failure, osteoporosis, premature birth, and hypoglycaemia, inflammatory disorders such as rheumatoid arthritis and osteoarthritis, pain, asthma, psoriasis and allergies, irritable bowel syndrome, Crohn's disease, spastic colon, post-operative ileus, ulcer, diarrhea, stress-induced fever, human immunodeficiency virus (HIV) infections, neurodegenerative diseases such as Alzheimer's disease, Parkinson's disease and Huntington's disease, gastrointestinal diseases, stroke, stress induced immune dysfunctions, muscular spasms, urinary incontinence. In a specific embodiment, the subject has and/or the treatment is a treatment of depressive symptoms, anxiety symptoms or both depressive symptoms and anxiety symptoms. The depressive and/or anxiety symptoms can be symptoms of a depressive disorder, an anxiety disorder or both a depressive disorder and anxiety disorder. In another specific embodiment, the subject has and/or the treatment is a treatment of a sleep disorder.

A "treatment response", as used herein, generally refers to any measurable response specific for the treatment with SSR-125543 or a pharmaceutically acceptable salt thereof and/or the condition being treated, during and/or shortly after treatment as compared to before said treatment. Generally, the treatment response can be a biological response or a clinical response. A biological response would include, for example, any alteration in CRH over-activity, as defined above.

Preferably, according to the invention, the treatment response is a clinical treatment response. A "clinical treatment response", as used herein, refers to a prevention, alteration, alleviation or complete remission, as measured by the alteration in severity and/or frequency of relapse of individual symptoms and/or the mean change on a diagnostic marker or scale of any type commonly used in assessing clinical responses in the conditions described herein, see, for instance, Harrison's Principles of Internal Medicine, 18th ed. / editors Longo et al., Mcgraw-Hill Publ. Comp, NY, US (2011). A clinical treatment response can also include an alteration, increase or decrease in adverse effects resulting from the treatment with SSR-125543 or a pharmaceutically acceptable salt thereof. Predicting a clinical response, or lack thereof, is expressly distinguished from predicting merely biological responses, since a clinical response is to be seen as target variable directly linked to treatment success, or failure, respectively. Therefore, while biological responses can also be predicted by the methods described herein, the methods of the invention are particularly suited for predicting a clinical response, as defined above.

In preferred embodiments, the clinical response can be a prevention, alteration, alleviation or complete remission of depressive symptoms and/or anxiety symptoms. In preferred embodiments, the clinical response can be a prevention, alteration, alleviation or complete remission of a sleep disorder. Depressive symptoms comprise, but are not limited to, low mood, low self-esteem, loss of interest or pleasure, psychosis, poor concentration and memory, social isolation, psychomotor agitation/retardation, thoughts of death or suicide, significant weight change (loss/gain), fatigue, and feeling of worthlessness. The depressive symptoms can last for weeks to lifelong with periodic reoccurring depressive episodes. For the diagnosis of the depression mode (e.g. moderate or severe depression) the Hamilton Depression Rating Scale (HAM-D) (Hamilton, J Neurol Neurosurg Psychiatry, 1960) may be used. In addition or alternatively, the depression mode may be also rated by alternative scales as the Beck Depression Inventory (BDI), the Montgomery-Asberg Depression Scale (MADRS), the Geriatric Depression Scale (GDS), and/or the Zung Self-Rating Depression Scale (ZSRDS). Therefore, in some embodiments, the clinical response is a prevention, alteration, alleviation or complete remission of depressive symptoms as determined using a scale selected from the group consisting of HAM-D, BDI, MADRS, GDS, ZSRDS.

Anxiety symptoms comprise, but are not limited to, panic disorders, generalized anxiety disorder, phobias and posttraumatic stress disorder, avoidance behavior which may lead to social isolation, physical ailments like tachycardia, dizziness and sweating, mental apprehension, stress and/or tensions. The severity of anxiety symptoms ranges from nervousness and discomfort to panic and terror in subjects. Anxiety symptoms may persist for several days, weeks, or even months and years, if not suitably treated. The severity of anxiety symptoms may be measured by the Hamilton Anxiety Rating Scale (HAM-A) and/or the State-Trait Anxiety Rating Scale (STAI). Therefore, in some embodiments, the clinical response is a prevention, alteration, alleviation or complete remission of anxiety symptoms as determined using a scale selected from the group consisting of HAM-A and STAI. Sleep disorders comprise, but are not limited to, insomnia, hypersomnia, narcolepsy, idiopathic hypersomnia, excessive amounts of sleepiness, lack of alertness, lack of attentiveness, absentmindedness and/or lack of or aversion to movement or exercise, sleep disorders induced by stress

"Alteration", as used herein, refers to any change in a clinical response as defined above. "Alleviation", as used herein, refers to any amelioration in a clinical response, including partial amelioration of one or more symptoms, temporary disappearance of one or more symptoms, wherein relapse is not excluded, as well as complete remission of one or more symptoms. "Complete remission" refers to disappearance of all manifestations and symptoms of a disease to be treated, as described herein.

The present method of treatment identifies sets of polymorphism genotypes that are predictive for the treatment response of a subject to treatment with SSR-125543 or a pharmaceutically acceptable salt thereof. Thus, the presence of one or more of these polymorphism genotypes can be used to predict the likelihood of responding or not responding to treatment with SSR-125543 or a pharmaceutically acceptable salt thereof in a subject.

The term "polymorphism", as used herein, refers to a sequential variation of a genomic allele at the same locus within a population of subjects and having a certain frequency in the population, including deletions / insertions (designated "[-/I]" herein), point mutations and translocations. The term "polymorphism", as used herein, in particular includes, but is not limited to, single nucleotide polymorphisms (SNPs). For instance, as used herein, the term "polymorphism" can also include polymorphic deletions, or insertions, respectively, of more than one nucleotide. The term "single nucleotide polymorphism" or "SNP" is well understood by the skilled person and refers to a point mutation of a genomic allele at the same locus within a population of subjects and having a certain frequency in a population. The term "genotype", as used herein, encompasses one or both genomic alleles at the same locus of a subject. The term "polymorphism genotype" or "SNP genotype", as used herein, refers to the presence of a polymorphism or SNP within the genotype of a subject, either in one or both genomic alleles at the same locus. The allele being present in the majority of the population, is also referred to herein as wild-type allele or major allele. As used herein, this state is defined as the "absence of one or more polymorphism genotypes". The nucleotide being present in the minority of the population is also referred to herein as the variation, point mutation, mutated nucleotide or minor allele. As used herein this state is defined as "presence of one or more polymorphism genotype". For instance, P_ID 1 as identified in Table 2 below, (rs34169260, TOP, [A/G]) exhibits a variation to nucleotide G instead of the wild-type nucleotide A. Typically, a polymorphism or SNP genotype occurs in a certain percentage of a population, for example in at least 5 % or at least 10% of a population. In other words, the minor allele frequency (MAF) is equal or higher than about 0.05 or about 0.10 (MAF > 0.05 or MAF > 0.10).

Theoretically, a wild-type allele could be mutated to three alternative nucleotides. However, a mutation to a first nucleotide within germline cells of an individual which persists within a population occurs very rarely. The chance of the same nucleotide being mutated to yet another nucleotide and again persisting within a population is virtually non-existent and can be therefore neglected. Therefore, as used herein, a certain nucleotide position in the genome of an individual can only have the above two states, namely the wild-type state (absence of a polymorphism genotype from both alleles of a single subject) and the mutated state (presence of a polymorphism genotype in one or both alleles of a single subject). The presence of a polymorphism genotype in both alleles may have a higher predictive value than the presence of a polymorphism genotype in one allele only, the other allele comprising a wild-type genotype. The presence or absence of a polymorphism genotype on one or two alleles may be associated with an algorithm for predicting the treatment response to the CRHR1 antagonist as described herein.

Sets of polymorphism genotypes useful in predicting a treatment response are disclosed in Table 2. Table 2 provides a consecutively numbered identifier (P_ID) for internal reference, an rs-identifier (rs_ID), as commonly known in polymorphism databases such as NCBI's dbSNP, the polymorphism (P, indicated in bold and defined as [wild-type / variation]), the strand designation (Str, see, e.g., Illumina Inc. *"TOP*/*BOT" Strand and "A*/*B" Allele* - *A guide to Illumina's method for determining Strand and Allele for the GoldenGate*® *and Infinium*™ *Assays",* Technical Note, © 2006; http://www.illumina.com/documents/products/technotes/technote_topbot.pdf;), specific probe sequences for the respective allele in humans (AlleleA Probe, see also SEQ ID NOs: 275-548), a human chromosomal identifier (Chr), and a reference to the sequence of the genomic flanking sequence in humans (TopGenomicSequence), as disclosed in SEQ ID NOs: 1-274. A person skilled in the art is able to derive the exact position and polymorphism genotype sequence from the rs-nomenclature identified in Table 2 from suitable database entries and associated information systems, e.g. the NCBI's Single Nucleotide Polymorphism database (dbSNP; http://www.ncbi.nlm.nih.gov/SNP/), even where the nomenclature, or the surrounding sequence elements were subject to alterations over time.

**Table 2 - Polymorphism genotypes as used herein**

| P_ID | rs_ID | Str | P | AlleleA Probe | Chr | TopGenomic Sequence |
|---|---|---|---|---|---|---|
| 1 | rs34169260 | TOP | **[A/G]** | | 17 | SEQ ID NO: 1 |
| 2 | rs796287 | TOP | **[A/C]** | | 2 | SEQ ID NO: 2 |
| 3 | rs56149945 | TOP | **[A/G]** | | 5 | SEQ ID NO: 3 |
| 4 | rs6190 | BOT | **[T/C]** | | 5 | SEQ ID NO: 4 |
| 5 | rs7179092 | BOT | **[T/C]** | | 15 | SEQ ID NO: 5 |
| 6 | rs7614867 | TOP | **[A/G]** | | 3 | SEQ ID NO: 6 |
| | | | | | | |
| 7 | rs920640 | BOT | **[T/C]** | | 15 | SEQ ID NO: 7 |
| 8 | rs7167722 | BOT | **[T/C]** | | 15 | SEQ ID NO: 8 |
| 9 | rs920638 | BOT | **[T/C]** | | 15 | SEQ ID NO: 9 |
| 10 | rs7165629 | BOT | **[T/C]** | | 15 | SEQ ID NO: 10 |
| 11 | rs80049044 | BOT | **[T/A]** | | 4 | SEQ ID NO: 11 |
| 12 | rs16941058 | TOP | **[A/G]** | | 17 | SEQ ID NO: 12 |
| 13 | rs112015971 | TOP | **[A/G]** | | 20 | SEQ ID NO: 13 |
| 14 | rs10894873 | BOT | **[T/C]** | | 11 | SEQ ID NO: 14 |
| 15 | rs117455294 | BOT | **[T/G]** | | 20 | SEQ ID NO: 15 |
| 16 | rs1170303 | BOT | **[T/C]** | | 4 | SEQ ID NO: 16 |
| 17 | rs16940681 | TOP | **[C/G]** | | 17 | SEQ ID NO: 17 |
| 18 | rs968519 | BOT | **[T/C]** | | 20 | SEQ ID NO: 18 |
| 19 | rs28381866 | BOT | **[T/C]** | | 7 | SEQ ID NO: 19 |
| 20 | rs79320848 | BOT | **[T/G]** | | 20 | SEQ ID NO: 20 |
| 21 | rs11465364 6 | BOT | **[T/G]** | | 7 | SEQ ID NO: 21 |
| 22 | rs2589496 | BOT | **[T/C]** | | 17 | SEQ ID NO: 22 |
| 23 | rs10482650 | TOP | **[A/G]** | | 5 | SEQ ID NO: 23 |
| 24 | rs17614642 | TOP | **[A/G]** | | 6 | SEQ ID NO: 24 |
| 25 | rs73200317 | BOT | **[T/C]** | | 7 | SEQ ID NO: 25 |
| 26 | rs1380146 | BOT | **[T/A]** | | 12 | SEQ ID NO: 26 |
| 27 | rs735164 | BOT | **[T/C]** | | 16 | SEQ ID NO: 27 |
| 28 | rs730976 | BOT | **[T/G]** | | 5 | SEQ ID NO: 28 |
| 29 | rs55934524 | BOT | **[T/G]** | | 17 | SEQ ID NO: 29 |
| 30 | rs4570614 | TOP | **[A/G]** | | 11 | SEQ ID NO: 30 |
| 31 | rs4458044 | TOP | **[C/G]** | | 17 | SEQ ID NO: 31 |
| 32 | rs77850169 | TOP | **[A/G]** | | 17 | SEQ ID NO: 32 |
| 33 | rs35339359 | TOP | **[A/G]** | | 17 | SEQ ID NO: 33 |
| 34 | rs34800935 | BOT | **[T/C]** | | 7 | SEQ ID NO: 34 |
| 35 | rs72945439 | BOT | **[T/C]** | | 2 | SEQ ID NO: 35 |
| 36 | rs113959523 | TOP | **[A/G]** | | 20 | SEQ ID NO: 36 |
| 37 | rs116798177 | TOP | **[A/G]** | | 5 | SEQ ID NO: 37 |
| 38 | rs11247577 | BOT | **[T/G]** | | 17 | SEQ ID NO: 38 |
| 39 | rs75869266 | BOT | **[T/C]** | | 15 | SEQ ID NO: 39 |
| 40 | rs74372553 | BOT | **[T/C]** | | 17 | SEQ ID NO: 40 |
| 41 | rs11691508 | TOP | **[A/G]** | | 2 | SEQ ID NO: 41 |
| 42 | rs6493965 | TOP | **[A/G]** | | 15 | SEQ ID NO: 42 |
| 43 | rs4869476 | BOT | **[T/C]** | | 5 | SEQ ID NO: 43 |
| 44 | rs3730170 | BOT | **[T/C]** | | 20 | SEQ ID NO: 44 |
| 45 | rs2145288 | TOP | **[A/C]** | | 20 | SEQ ID NO: 45 |
| 46 | rs2935752 | TOP | **[A/C]** | | 8 | SEQ ID NO: 46 |
| 47 | rs146512400 | TOP | **[A/G]** | | 7 | SEQ ID NO: 47 |
| 48 | rs62057097 | BOT | **[T/C]** | | 17 | SEQ ID NO: 48 |
| 49 | rs115061314 | BOT | **[T/C]** | | 6 | SEQ ID NO: 49 |
| 50 | rs34113594 | BOT | **[T/G]** | | 17 | SEQ ID NO: 50 |
| 51 | rs61751173 | TOP | **[A/G]** | | 5 | SEQ ID NO: 51 |
| 52 | rs74338736 | TOP | **[A/C]** | | 20 | SEQ ID NO: 52 |
| 53 | rs10851726 | BOT | **[T/C]** | | 15 | SEQ ID NO: 53 |
| 54 | rs4610906 | BOT | **[T/C]** | | X | SEQ ID NO: 54 |
| 55 | rs59485211 | BOT | **[T/C]** | | X | SEQ ID NO: 55 |
| 56 | rs7060015 | BOT | **[T/G]** | | X | SEQ ID NO: 56 |
| 57 | rs75710780 | BOT | **[T/G]** | | 6 | SEQ ID NO: 57 |
| 58 | rs6520908 | BOT | **[T/C]** | | X | SEQ ID NO: 58 |
| 59 | rs487011 | BOT | **[T/G]** | | X | SEQ ID NO: 59 |
| 60 | rs1383699 | TOP | **[A/C]** | | 4 | SEQ ID NO: 60 |
| 61 | rs67516871 | TOP | **[A/G]** | | X | SEQ ID NO: 61 |
| 62 | rs114106519 | BOT | **[T/C]** | | 7 | SEQ ID NO: 62 |
| 63 | rs7220091 | TOP | **[A/G]** | | 17 | SEQ ID NO: 63 |
| 64 | rs12489026 | TOP | **[A/G]** | | 3 | SEQ ID NO: 64 |
| 65 | rs876270 | BOT | **[T/C]** | | 12 | SEQ ID NO: 65 |
| 66 | rs4968161 | BOT | **[T/C]** | | 17 | SEQ ID NO: 66 |
| 67 | rs62056907 | TOP | **[A/G]** | | 17 | SEQ ID NO: 67 |
| 68 | rs2235013 | BOT | **[T/C]** | | 7 | SEQ ID NO: 68 |
| 69 | rs16878812 | TOP | **[A/G]** | | 6 | SEQ ID NO: 69 |
| 70 | rs6549407 | TOP | **[A/G]** | | 3 | SEQ ID NO: 70 |
| 71 | rs28381848 | TOP | **[A/G]** | | 7 | SEQ ID NO: 71 |
| 72 | rs79723704 | TOP | **[A/C]** | | 20 | SEQ ID NO: 72 |
| 73 | rs72814052 | TOP | **[A/G]** | | 17 | SEQ ID NO: 73 |
| 74 | rs10152908 | BOT | **[T/C]** | | 15 | SEQ ID NO: 74 |
| 75 | rs172769 | TOP | **[A/C]** | | 2 | SEQ ID NO: 75 |
| 76 | rs78596668 | BOT | **[T/C]** | | 6 | SEQ ID NO: 76 |
| 77 | rs73307922 | BOT | **[T/C]** | | 20 | SEQ ID NO: 77 |
| 78 | rs3842 | TOP | **[A/G]** | | 7 | SEQ ID NO: 78 |
| 79 | rs7210584 | TOP | **[A/C]** | | 17 | SEQ ID NO: 79 |
| 80 | rs62402121 | BOT | **[T/C]** | | 6 | SEQ ID NO: 80 |
| 81 | rs55709291 | TOP | **[A/G]** | | 17 | SEQ ID NO: 81 |
| 82 | rs72747088 | TOP | **[A/G]** | | 15 | SEQ ID NO: 82 |
| 83 | rs929610 | BOT | **[G/C]** | | 14 | SEQ ID NO: 83 |
| 84 | rs6766242 | BOT | **[T/C]** | | 3 | SEQ ID NO: 84 |
| 85 | rs1468552 | BOT | **[G/C]** | | 16 | SEQ ID NO: 85 |
| 86 | rs78838114 | BOT | **[T/C]** | | 15 | SEQ ID NO: 86 |
| 87 | rs62489862 | BOT | **[T/C]** | | 7 | SEQ ID NO: 87 |
| 88 | rs894342 | TOP | **[A/G]** | | 15 | SEQ ID NO: 88 |
| 89 | rs58882373 | BOT | **[T/C]** | | 3 | SEQ ID NO: 89 |
| 90 | rs3811939 | TOP | **[A/G]** | | 5 | SEQ ID NO: 90 |
| 91 | rs6984688 | BOT | **[T/G]** | | 8 | SEQ ID NO: 91 |
| 92 | rs1018160 | BOT | **[T/C]** | | 5 | SEQ ID NO: 92 |
| 93 | rs76602912 | TOP | **[A/G]** | | 20 | SEQ ID NO: 93 |
| 94 | rs80067508 | TOP | **[A/G]** | | 17 | SEQ ID NO: 94 |
| 95 | rs74888440 | BOT | **[T/C]** | | 5 | SEQ ID NO: 95 |
| 96 | rs12481583 | BOT | **[T/C]** | | 20 | SEQ ID NO: 96 |
| 97 | rs66794218 | TOP | **[A/G]** | | 17 | SEQ ID NO: 97 |
| 98 | rs16946701 | TOP | **[A/G]** | | 15 | SEQ ID NO: 98 |
| 99 | rs75726724 | TOP | **[A/G]** | | 15 | SEQ ID NO: 99 |
| 100 | rs67959715 | BOT | **[T/A]** | | 13 | SEQ ID NO: 100 |
| 101 | rs11871392 | BOT | **[T/G]** | | 17 | SEQ ID NO: 101 |
| 102 | rs2044070 | TOP | **[A/G]** | | 15 | SEQ ID NO: 102 |
| 103 | rs77612799 | BOT | **[T/C]** | | 6 | SEQ ID NO: 103 |
| 104 | rs6743702 | BOT | **[T/C]** | | 2 | SEQ ID NO: 104 |
| 105 | rs616870 | BOT | **[T/C]** | | 3 | SEQ ID NO: 105 |
| 106 | rs79590198 | TOP | **[A/G]** | | 5 | SEQ ID NO: 106 |
| 107 | rs75715199 | TOP | **[A/G]** | | 17 | SEQ ID NO: 107 |
| 108 | rs13087555 | BOT | **[T/C]** | | 3 | SEQ ID NO: 108 |
| 109 | rs4869618 | BOT | **[T/C]** | | 5 | SEQ ID NO: 109 |
| 110 | rs117397046 | TOP | **[A/G]** | | 17 | SEQ ID NO: 110 |
| 111 | rs8042817 | TOP | **[A/G]** | | 15 | SEQ ID NO: 111 |
| 112 | rs2258097 | BOT | **[T/C]** | | 17 | SEQ ID NO: 112 |
| 113 | rs2260882 | TOP | **[C/G]** | | 17 | SEQ ID NO: 113 |
| 114 | rs532996 | TOP | **[A/G]** | | 13 | SEQ ID NO: 114 |
| 115 | rs11747040 | BOT | **[T/C]** | | 5 | SEQ ID NO: 115 |
| 116 | rs10034039 | BOT | **[T/G]** | | 4 | SEQ ID NO: 116 |
| 117 | rs11690936 9 | TOP | **[A/G]** | | 17 | SEQ ID NO: 117 |
| 118 | rs79134986 | TOP | **[A/G]** | | 6 | SEQ ID NO: 118 |
| 119 | rs117615688 | BOT | **[T/C]** | | 17 | SEQ ID NO: 119 |
| 120 | rs8032253 | BOT | **[T/C]** | | 15 | SEQ ID NO: 120 |
| 121 | rs12818653 | BOT | **[T/A]** | | 12 | SEQ ID NO: 121 |
| 122 | rs4587884 | TOP | **[A/C]** | | 14 | SEQ ID NO: 122 |
| 123 | rs77122853 | BOT | **[T/C]** | | 20 | SEQ ID NO: 123 |
| 124 | rs117615061 | BOT | **[T/C]** | | 20 | SEQ ID NO: 124 |
| 125 | rs74682905 | TOP | **[A/G]** | | 7 | SEQ ID NO: 125 |
| 126 | rs2257468 | BOT | **[T/C]** | | 17 | SEQ ID NO: 126 |
| 127 | rs2032582 | BOT | **[T/G]** | | 7 | SEQ ID NO: 127 |
| 128 | rs2235015 | BOT | **[T/G]** | | 7 | SEQ ID NO: 128 |
| 129 | rs2729794 | BOT | **[T/C]** | | 15 | SEQ ID NO: 129 |
| 130 | rs77549514 | TOP | **[A/G]** | | 2 | SEQ ID NO: 130 |
| 131 | rs74790420 | TOP | **[A/C]** | | 17 | SEQ ID NO: 131 |
| 132 | rs73129579 | BOT | **[T/C]** | | 20 | SEQ ID NO: 132 |
| 133 | rs12913346 | TOP | **[A/C]** | | 15 | SEQ ID NO: 133 |
| 134 | rs117560908 | BOT | **[T/C]** | | 17 | SEQ ID NO: 134 |
| 135 | rs72747091 | TOP | **[A/G]** | | 15 | SEQ ID NO: 135 |
| 136 | rs2935751 | TOP | **[A/G]** | | 8 | SEQ ID NO: 136 |
| 137 | rs4331446 | TOP | **[A/G]** | | 2 | SEQ ID NO: 137 |
| 138 | rs7523266 | BOT | **[T/C]** | | 1 | SEQ ID NO: 138 |
| 139 | rs7648662 | BOT | **[T/C]** | | 3 | SEQ ID NO: 139 |
| 140 | rs117034065 | TOP | **[A/G]** | | 15 | SEQ ID NO: 140 |
| 141 | rs4836256 | BOT | **[T/C]** | | 5 | SEQ ID NO: 141 |
| 142 | rs80238698 | BOT | **[T/C]** | | 7 | SEQ ID NO: 142 |
| 143 | rs3730173 | BOT | **[T/C]** | | 20 | SEQ ID NO: 143 |
| 144 | rs11687884 | BOT | **[T/C]** | | 2 | SEQ ID NO: 144 |
| 145 | rs72693005 | BOT | **[T/C]** | | 4 | SEQ ID NO: 145 |
| 146 | rs2589476 | BOT | **[T/C]** | | 17 | SEQ ID NO: 146 |
| 147 | rs9813396 | BOT | **[T/C]** | | 3 | SEQ ID NO: 147 |
| 148 | rs10482667 | TOP | **[A/G]** | | 5 | SEQ ID NO: 148 |
| 149 | rs72784444 | TOP | **[A/G]** | | 5 | SEQ ID NO: 149 |
| 150 | rs75074511 | BOT | **[T/C]** | | 17 | SEQ ID NO: 150 |
| 151 | rs7951003 | TOP | **[A/G]** | | 11 | SEQ ID NO: 151 |
| 152 | rs79584784 | TOP | **[A/G]** | | 7 | SEQ ID NO: 152 |
| 153 | rs2214102 | BOT | **[T/C]** | | 7 | SEQ ID NO: 153 |
| 154 | rs28811003 | TOP | **[A/G]** | | 15 | SEQ ID NO: 154 |
| 155 | rs6100261 | TOP | **[A/T]** | | 20 | SEQ ID NO: 155 |
| 156 | rs77152456 | TOP | **[A/G]** | | 15 | SEQ ID NO: 156 |
| 157 | rs66624622 | BOT | **[T/G]** | | 5 | SEQ ID NO: 157 |
| 158 | rs140302965 | TOP | **[A/G]** | | 7 | SEQ ID NO: 158 |
| 159 | rs11653269 | BOT | **[T/C]** | | 17 | SEQ ID NO: 159 |
| 160 | rs74405057 | TOP | **[A/G]** | | 20 | SEQ ID NO: 160 |
| 161 | rs7121 | TOP | **[A/G]** | | 20 | SEQ ID NO: 161 |
| 162 | rs16977818 | TOP | **[A/C]** | | 15 | SEQ ID NO: 162 |
| 163 | rs12490095 | BOT | **[T/C]** | | 3 | SEQ ID NO: 163 |
| 164 | rs118003903 | TOP | **[A/G]** | | 17 | SEQ ID NO: 164 |
| 165 | rs62377761 | BOT | **[T/C]** | | 5 | SEQ ID NO: 165 |
| 166 | P1_M_061510_6_34_M | MIN US | **[-/I*]** | | 6 | SEQ ID NO: 166 |
| 167 | rs375115639 | MIN US | **[-/I*]** | | 6 | SEQ ID NO: 167 |
| 168 | rs1002204 | TOP | **[A/C]** | | 7 | SEQ ID NO: 168 |
| 169 | rs10062367 | TOP | **[A/G]** | | 5 | SEQ ID NO: 169 |
| 170 | rs10482642 | TOP | **[A/G]** | | 5 | SEQ ID NO: 170 |
| 171 | rs10482658 | TOP | **[A/G]** | | 5 | SEQ ID NO: 171 |
| 172 | rs1053989 | TOP | **[A/C]** | | 5 | SEQ ID NO: 172 |
| 173 | rs10851628 | BOT | **[T/C]** | | 15 | SEQ ID NO: 173 |
| | | | | | | |
| 174 | rs10947562 | BOT | **[T/C]** | | 6 | SEQ ID NO: 174 |
| 175 | rs11069612 | TOP | **[A/G]** | | 13 | SEQ ID NO: 175 |
| 176 | rs11071351 | BOT | **[T/C]** | | 15 | SEQ ID NO: 176 |
| 177 | rs11091175 | TOP | **[A/G]** | | X | SEQ ID NO: 177 |
| 178 | rs11638450 | BOT | **[T/C]** | | 15 | SEQ ID NO: 178 |
| 179 | rs11715827 | BOT | **[T/G]** | | 3 | SEQ ID NO: 179 |
| 180 | rs11745958 | BOT | **[T/C]** | | 5 | SEQ ID NO: 180 |
| 181 | rs11834041 | TOP | **[A/G]** | | 12 | SEQ ID NO: 181 |
| 182 | rs1202180 | BOT | **[T/C]** | | 7 | SEQ ID NO: 182 |
| 183 | rs12054781 | TOP | **[A/G]** | | 5 | SEQ ID NO: 183 |
| 184 | rs12539395 | TOP | **[A/G]** | | 7 | SEQ ID NO: 184 |
| 185 | rs12720066 | BOT | **[T/G]** | | 7 | SEQ ID NO: 185 |
| 186 | rs1279754 | TOP | **[A/C]** | | 5 | SEQ ID NO: 186 |
| 187 | rs12872047 | BOT | **[T/C]** | | 13 | SEQ ID NO: 187 |
| 188 | rs12876742 | TOP | **[A/C]** | | 13 | SEQ ID NO: 188 |
| 189 | rs12917505 | TOP | **[A/G]** | | 15 | SEQ ID NO: 189 |
| 190 | rs13066950 | BOT | **[T/G]** | | 3 | SEQ ID NO: 190 |
| 191 | rs13229143 | TOP | **[C/G]** | | 7 | SEQ ID NO: 191 |
| 192 | rs1383707 | BOT | **[T/C]** | | 4 | SEQ ID NO: 192 |
| 193 | rs1441824 | BOT | **[T/C]** | | 15 | SEQ ID NO: 193 |
| 194 | rs1652311 | TOP | **[A/G]** | | 2 | SEQ ID NO: 194 |
| 195 | rs17064 | BOT | **[T/A]** | | 7 | SEQ ID NO: 195 |
| 196 | rs17100236 | TOP | **[A/G]** | | 5 | SEQ ID NO: 196 |
| 197 | rs17149699 | TOP | **[A/G]** | | 7 | SEQ ID NO: 197 |
| 198 | rs1724386 | TOP | **[A/G]** | | 17 | SEQ ID NO: 198 |
| 199 | rs17250255 | TOP | **[A/G]** | | 7 | SEQ ID NO: 199 |
| 200 | rs17327624 | BOT | **[T/G]** | | 7 | SEQ ID NO: 200 |
| 201 | rs17616338 | TOP | **[A/G]** | | 4 | SEQ ID NO: 201 |
| 202 | rs17687796 | TOP | **[A/G]** | | 17 | SEQ ID NO: 202 |
| 203 | rs17740874 | BOT | **[T/C]** | | 2 | SEQ ID NO: 203 |
| 204 | rs17763104 | BOT | **[T/C]** | | 17 | SEQ ID NO: 204 |
| 205 | rs1880748 | BOT | **[T/C]** | | 17 | SEQ ID NO: 205 |
| 206 | rs1882478 | TOP | **[A/G]** | | 7 | SEQ ID NO: 206 |
| 207 | rs1944887 | BOT | **[T/C]** | | 11 | SEQ ID NO: 207 |
| 208 | rs2028629 | TOP | **[A/G]** | | 17 | SEQ ID NO: 208 |
| 209 | rs2143404 | TOP | **[A/G]** | | 6 | SEQ ID NO: 209 |
| 210 | rs2173530 | BOT | **[T/C]** | | 13 | SEQ ID NO: 210 |
| 211 | rs220806 | BOT | **[T/C]** | | 6 | SEQ ID NO: 211 |
| 212 | rs2235047 | TOP | **[A/C]** | | 7 | SEQ ID NO: 212 |
| 213 | rs2242071 | TOP | **[A/G]** | | 2 | SEQ ID NO: 213 |
| 214 | rs2257474 | BOT | **[T/C]** | | 17 | SEQ ID NO: 214 |
| 215 | rs2295583 | TOP | **[A/T]** | | 20 | SEQ ID NO: 215 |
| 216 | rs234629 | BOT | **[T/C]** | | 20 | SEQ ID NO: 216 |
| 217 | rs234630 | TOP | **[A/G]** | | 20 | SEQ ID NO: 217 |
| 218 | rs2436401 | TOP | **[A/G]** | | 5 | SEQ ID NO: 218 |
| 219 | rs258750 | BOT | **[T/C]** | | 5 | SEQ ID NO: 219 |
| 220 | rs2589487 | BOT | **[T/C]** | | 17 | SEQ ID NO: 220 |
| 221 | rs28364018 | BOT | **[T/G]** | | 8 | SEQ ID NO: 221 |
| 222 | rs28381774 | BOT | **[T/C]** | | 7 | SEQ ID NO: 222 |
| 223 | rs28381784 | TOP | **[A/G]** | | 7 | SEQ ID NO: 223 |
| 224 | rs2963155 | TOP | **[A/G]** | | 5 | SEQ ID NO: 224 |
| 225 | rs3133622 | BOT | **[T/G]** | | 8 | SEQ ID NO: 225 |
| 226 | rs32897 | BOT | **[T/C]** | | 5 | SEQ ID NO: 226 |
| 227 | rs33388 | TOP | **[A/T]** | | 5 | SEQ ID NO: 227 |
| 228 | rs3730168 | BOT | **[T/C]** | | 20 | SEQ ID NO: 228 |
| 229 | rs3735833 | BOT | **[T/G]** | | 8 | SEQ ID NO: 229 |
| 230 | rs3777747 | TOP | **[A/G]** | | 6 | SEQ ID NO: 230 |
| 231 | rs3786066 | BOT | **[T/C]** | | 17 | SEQ ID NO: 231 |
| 232 | rs3798346 | BOT | **[T/C]** | | 6 | SEQ ID NO: 232 |
| 233 | rs3822736 | TOP | **[A/G]** | | 5 | SEQ ID NO: 233 |
| 234 | rs389035 | BOT | **[T/C]** | | 2 | SEQ ID NO: 234 |
| 235 | rs3924144 | TOP | **[A/G]** | | 2 | SEQ ID NO: 235 |
| 236 | rs4148737 | BOT | **[T/C]** | | 7 | SEQ ID NO: 236 |
| 237 | rs4148749 | BOT | **[G/C]** | | 7 | SEQ ID NO: 237 |
| 238 | rs417968 | BOT | **[T/C]** | | 17 | SEQ ID NO: 238 |
| 239 | rs4458144 | BOT | **[T/C]** | | 2 | SEQ ID NO: 239 |
| 240 | rs4515335 | BOT | **[T/C]** | | 5 | SEQ ID NO: 240 |
| 241 | rs4728699 | TOP | **[A/G]** | | 7 | SEQ ID NO: 241 |
| 242 | rs4758040 | TOP | **[A/G]** | | 11 | SEQ ID NO: 242 |
| 243 | rs4812040 | TOP | **[A/G]** | | 20 | SEQ ID NO: 243 |
| 244 | rs4912650 | BOT | **[T/G]** | | 5 | SEQ ID NO: 244 |
| 245 | rs4957891 | BOT | **[T/C]** | | 5 | SEQ ID NO: 245 |
| 246 | rs5906392 | TOP | **[A/G]** | | X | SEQ ID NO: 246 |
| 247 | rs6026561 | BOT | **[T/C]** | | 20 | SEQ ID NO: 247 |
| 248 | rs6026565 | BOT | **[T/A]** | | 20 | SEQ ID NO: 248 |
| 249 | rs6026567 | TOP | **[A/G]** | | 20 | SEQ ID NO: 249 |
| 250 | rs6026593 | TOP | **[A/G]** | | 20 | SEQ ID NO: 250 |
| 251 | rs6092704 | BOT | **[T/G]** | | 20 | SEQ ID NO: 251 |
| 252 | rs6100260 | TOP | **[A/G]** | | 20 | SEQ ID NO: 252 |
| 253 | rs6128461 | BOT | **[T/C]** | | 20 | SEQ ID NO: 253 |
| 254 | rs6415328 | BOT | **[T/C]** | | 7 | SEQ ID NO: 254 |
| 255 | rs6610868 | BOT | **[T/C]** | | X | SEQ ID NO: 255 |
| 256 | rs6686061 | TOP | **[A/C]** | | 1 | SEQ ID NO: 256 |
| 257 | rs6730350 | BOT | **[T/G]** | | 2 | SEQ ID NO: 257 |
| 258 | rs6746197 | BOT | **[T/C]** | | 2 | SEQ ID NO: 258 |
| 259 | rs6963426 | BOT | **[T/C]** | | 7 | SEQ ID NO: 259 |
| 260 | rs7121326 | BOT | **[T/C]** | | 11 | SEQ ID NO: 260 |
| 261 | rs7721799 | TOP | **[A/G]** | | 5 | SEQ ID NO: 261 |
| 262 | rs7787082 | BOT | **[T/C]** | | 7 | SEQ ID NO: 262 |
| 263 | rs7799592 | TOP | **[A/C]** | | 7 | SEQ ID NO: 263 |
| 264 | rs796245 | BOT | **[T/C]** | | 2 | SEQ ID NO: 264 |
| 265 | rs809482 | TOP | **[A/C]** | | 2 | SEQ ID NO: 265 |
| 266 | rs8125112 | BOT | **[T/C]** | | 20 | SEQ ID NO: 266 |
| 267 | rs919196 | TOP | **[A/G]** | | 20 | SEQ ID NO: 267 |
| 268 | rs920750 | BOT | **[T/C]** | | 17 | SEQ ID NO: 268 |
| 269 | rs9332385 | TOP | **[A/G]** | | 7 | SEQ ID NO: 269 |
| 270 | rs930473 | BOT | **[T/G]** | | 15 | SEQ ID NO: 270 |
| 271 | rs9324921 | TOP | **[A/C]** | | 5 | SEQ ID NO: 271 |
| 272 | rs9348979 | TOP | **[A/G]** | | 6 | SEQ ID NO: 272 |
| 273 | rs9571939 | TOP | **[A/C]** | | 13 | SEQ ID NO: 273 |
| 274 | rs9892359 | BOT | **[T/C]** | | 17 | SEQ ID NO: 274 |
| ***[-/I]** designates an allelic deletion/insertion polymorphism as defined in the respective SEQ ID NOs: 166 and 167 | | | | | | |

Further useful combinations of more than one polymorphism genotype are disclosed in Tables 5, 6, and 7 below, which all refer to the consecutively numbered, internal polymorphism-identifier (P_ID) of Table 2 to specify the genotype identity.

For the purposes of the present invention, the one or more polymorphism genotypes described above may be represented, for instance, within a nucleic acid of a length of, e.g., 1 nt, 2 nt, 3 nt, 4 nt, 5 nt, 10 nt, 15 nt, 20 nt, 25 nt, 30 nt, 35 nt, 40 nt, 45 nt, 50 nt, 60 nt, 70 nt, 80 nt, 90 nt, 100 nt, 200 nt, 300 nt, 400 nt, 500 nt, 1000 nt, 2000 nt, or more or any length in between these lengths. The nucleic acid may be any nucleic acid molecule, e.g. a DNA molecule, e.g., a genomic DNA molecule or a cDNA molecule, an RNA molecule, or a derivative thereof. The one or more polymorphism genotypes may further be represented by translated forms of the nucleic acid, e.g. a peptide or protein, as long as the polymorphic modification leads to a corresponding modification of the peptide or protein. Corresponding information is readily available in the art, e.g., from databases such as the NCBI dbSNP repository or the NCBI Genbank.

The polymorphism genotypes as described herein may be present on both strands of genomic DNA or its derivatives, i.e. on the chromosomal / genomic 5'→3' strand and/or the chromosomal / genomic 3'→5' strand. For example, a polymorphism can be present on the 5'→3' strand as A, it is present on the 3'→5' strand as T and *vice versa.* Also the insertion or deletion of bases may be detected on both DNA strands, with correspondence as defined above. For analytic purposes, the strand identity may be defined, or fixed, or may be chosen at will, e.g. in dependence on factors such the availability of binding elements, GC-content etc. Furthermore, for more universally applicable designation, a polymorphism genotype may be defined on both strands at the same time, or using the commonly known designations, such as the "probe/target"-designation, the "plus(+)/minus(-)"-designation, the "TOP/BOT"-designation or the "forward/reverse"-designation, as described in Nelson et al., Trends Genet. 2012, 28(8):361-3, or Illumina Inc. *"TOP*/*BOT" Strand and "A*/*B" Allele* - *A guide to Illumina's method for determining Strand and Allele for the GoldenGate*® *and Infinium*™ *Assays",* Technical Note, © 2006; http://www.illumina.com/documents/products/technotes/ technote_topbot.pdf. For the sake of unambiguity in polymorphism genotype designation, e.g., the "TOP/BOT"-designation can be used to identify the polymorphism genotypes in Table 2 above. In the alternative, the probe sequence or the genomic flanking sequences can be used to identify the polymorphism genotypes in Table 2 above.

A "polymorphic site" or "polymorphic variant" as used herein relates to the position of a polymorphism or SNP as described herein within the genome or portion of a genome of a subject, or within a genetic element derived from the genome or portion of a genome of a subject.

"Linkage disequilibrium" as used herein refers to co-inheritance of two or more alleles at frequencies greater than would be expected from the separate frequencies of occurrence of each allele in the corresponding control population. The expected frequency of occurrence of two or more alleles that are inherited independently is the population frequency of the first allele multiplied by the population frequency of the second allele. Alleles or polymorphisms that co-occur at expected frequencies are said to be in linkage equilibrium. Polymorphisms in linkage disequilibrium with a polymorphism of Table 2 can be identified by methods known to one skilled in the art. For example, Devlin and Risch (Genomics 1995, 29(2):311-22;) provide guidance for determining the parameter delta (also referred to as "r") as a standard measure of the linkage disequilibrium. Gabriel et al. (Science 2002, 296(5576):2225-9;) provides instructions for finding the maximal r² value in populations for disease gene mapping. Further, Carlson et al. (Am J Hum Genet 2004; 74(1): 106-120) disclose methods for selecting and analyzing polymorphisms based on linkage disequilibrium for disease gene association mapping. Stoyanovich and Pe'er (Bioinformatics, 2008, 24(3):440-2;) show that polymorphisms in linkage disequilibrium with identified polymorphisms have virtually identical response profiles. Currently, several databases provide datasets that can be searched for polymorphisms in strong linkage disequilibrium, which can be accessed by the following addresses: http://1000.genomes.org, http://www.hapmap.org, http://www.broadinsitute.org/mpg/snap. An example workflow for determining polymorphisms in linkage disequilibrium to a specific polymorphism is outlined in Uhr et al. (Neuron 2008, 57(2):203-9;). Preferably, the linkage disequilibrium referred to herein is strong linkage disequilibrium. "Strong linkage disequilibrium", as used herein, means that the polymorphism is in linkage disequilibrium with an r² higher than 0.7 or higher than 0.8 in the tested population or an ethnically close reference population with the identified polymorphism.

A "sample obtained from a subject" as used herein may be any sample any biological sample comprising a bodily fluid, cell, tissue, or fraction thereof, which includes analyte biomolecules of interest such as nucleic acids (e.g., DNA or RNA). For instance, the sample obtained from the subject can be a buccal sample, a blood sample, plasma, serum, semen, sputum, cerebral spinal fluid, tears, a tissue sample, a formalin-fixed, paraffin-embedded tissue sample, or a hair follicle. Such samples are routinely collected, processed, preserved and/or stored by methods well known in the art. A biological sample can be further fractionated, if desired, to a fraction containing particular cell types. If desired, a sample can be a combination of samples from a subject such as a combination of a tissue and fluid sample.

In some embodiments, the subject's nucleic acid or DNA is extracted, isolated, and/or purified from the sample by any method commonly known in the art prior to polymorphism and/or SNP genotyping analysis. The term "isolated nucleic acid molecule", as used herein, refers to a nucleic acid entity, e.g. DNA, RNA etc, being substantially free of other biological molecules, such as, proteins, lipids, carbohydrates, other nucleic acids or other material, such as cellular debris and growth media. Generally, the term "isolated" is not intended to refer to the complete absence of such material, or to the absence of water, buffers, or salts, unless they are present in amounts which substantially interfere with the steps of detecting and/or predicting. In alternative embodiments, detection of one or more polymorphism genotypes may also be performed by using a non-extracted, non-isolated or non-purified sample. In some embodiments, DNA amplification by any suitable method known in the art is used prior to the detection of one or more polymorphism genotypes.

The term "detecting the presence or absence of one or more polymorphism / SNP genotypes" is used herein synonymously to a "polymorphism / SNP genotyping analysis" and refers to a step of determining in one or several patients the presence or absence of at least one polymorphism / SNP genotype, typically several polymorphism / SNP genotypes, or all polymorphism / SNP genotypes disclosed in Table 2, or, in some embodiments, all (known) polymorphism / SNP genotypes of the human genome, including endogenous and exogenous regions. In particular, detecting the presence or absence of one or more polymorphism genotypes as used herein may not be limited to the CRHR1 gene or to genes of the CRH pathway, but can encompass a genome-wide screening for polymorphism genotypes.

A detection step or polymorphism / SNP genotyping analysis can be performed by any suitable method known in the art. Such methods include, but are not limited to, PCR-related methods using polymorphism / SNP-specific primers and/or probes, a primer extension reaction, polymorphism / SNP microarrays analysis, sequencing analysis, mass spectrometry, 5'-nuclease assays, allele specific hybridization, high-throughput / multiplex variants of these techniques or combinations thereof, as described in the prior art, for example in Rampal, DNA Arrays: Methods and Protocols (Methods in Molecular Biology) 2010; Graham & Hill, DNA Sequencing Protocols (Methods in Molecular Biology) 2001; Schuster, Nat. Methods, 2008 and Brenner, Nat. Biotech., 2000; Mardis, Annu Rev Genomics Hum Genet., 2008. Genome-wide arrays can be purchased from different suppliers such as Illumina or Affymetrix. For primer selection, multiplexing and assay design, and the mass-extension for producing primer extension products the MassARRAY Assay Designer software may be used using the sequences presented in Table 2 as input. The MassARRAY Typer 3.4 software may be used for genotype calling.

For example, the presence or absence of a polymorphism genotype can be detected by determining the nucleotide sequence at the respective locus and may be carried out by allele-specific oligonucleotide (ASO)-dot blot analysis, primer extension assays, iPLEX polymorphism / SNP genotyping, dynamic allele-specific hybridization (DASH) genotyping, the use of molecular beacons, tetra primer ARMS PCR, a flap endonuclease invader assay, an oligonucleotide ligase assay, PCR-single strand conformation polymorphism (SSCP) analysis, quantitative real-time PCR assay, polymorphism / SNP microarray based analysis, restriction enzyme fragment length polymorphism (RFLP) analysis, targeted resequencing analysis and/or whole genome sequencing analysis. In some embodiments, any of the methods described herein can comprise the determination of the haplotype for two copies of the chromosome comprising the polymorphism genotypes identified herein.

In another example, genomic DNA isolated from a biological sample can be amplified using PCR as described above. The amplicons can be detectably-labeled during the amplification (e.g., using one or more detectably labeled dNTPs) or subsequent to the amplification. Following amplification and labeling, the detectably-labeled-amplicons are then contacted with a plurality of polynucleotides, containing one or more of a polynucleotide (e.g., an oligonucleotide) being capable of specifically hybridizing to a corresponding amplicon containing a specific polymorphism, and where the plurality contains many probe sets each corresponding to a different, specific polymorphism. Generally, the probe sets are bound to a solid support and the position of each probe set is predetermined on the solid support. The binding of a detectably-labeled amplicon to a corresponding probe of a probe set indicates the presence of a nucleic acid containing the polymorphism so amplified in the biological sample. Suitable conditions and methods for detecting a polymorphism or SNP using nucleic acid arrays are further described in, e.g., Lamy et al. (2006) Nucleic Acids Research 34(14): e100; European Patent Publication No. 1234058; U.S. Publication Nos. 2006/0008823 and 2003/0059813; and U.S. Patent No. 6,410,231.

In yet another example, MALDI-TOF (matrix-assisted laser desorption ionization time of flight) mass spectrometry on the Sequenom platform (San Diego, USA) may be used to detect one or more polymorphism genotypes.

Polynucleotides for use in detection of one or more of the polymorphism genotypes disclosed in Tables 2, 5, 6 or 7 are capable of specifically hybridizing to nucleic acids comprising said one or more polymorphism genotypes and can comprise the nucleic acid sequences of the polymorphism genotypes themselves, including up and/or downstream, flanking sequences, e.g., as hybridization polynucleotide probes or primers (e.g., for amplification or reverse transcription). "Capable of specifically hybridizing", as used herein, refers to capability of hybridization under stringent conditions in any one of the methods of detection involving hybridization disclosed herein, as known to one skilled in the art. In that sense, primers and probes useful in such detection methods are particular polynucleotides capable of specifically hybridizing.

Primers or probes should contain a sequence of sufficient length and complementarity to a corresponding polymorphism locus to specifically hybridize with that locus under suitable hybridization conditions. For example, the polymorphism probes can include at least one (e.g., at least two, at least three, at least four, at least five, at least six, at least seven, at least eight, at least nine, at least 10, at least 11, at least 12, at least 15, at least 20, at least 25, at least 30, at least 35, at least 40, at least 45, at least 50, or 55 or more) nucleotides 5' or 3' to the polymorphism of interest. The polymorphic site of each probe (i.e., the polymorphism region) is generally flanked on one or both sides by sequence that is common among the different alleles. In specific embodiments, the polynucleotides capable of specifically hybridizing to the polymorphism genotypes are selected from the group consisting of the polynucleotides disclosed as "AlleleA Probe" in Table 2. The term "primer" may denote an oligo- or polynucleotide that acts as an initiation point of nucleotide synthesis under conditions in which synthesis of a primer extension product complementary to a nucleic acid strand is induced. The term "probe" may denote an oligonucleotide that is capable of specifically hybridizing to a target nucleic acid under suitable conditions, e.g., stringent conditions suitable for allele-specific hybridization. Primers and probes can be designed such are suitable for discriminating between wildtype allele or mutated allele of the position of a polymorphism to be analyzed, as described, e.g., by Coleman, and Tsongalis, Molecular Diagnostics: For the Clinical Laboratorian, 2007; Weiner et al. Genetic Variation: A Laboratory Manual, 2010.

Any of the methods of detecting a polymorphism can, optionally, be performed in multiplex formats that allow for rapid preparation, processing, and analysis of multiple samples, see above.

The detected polymorphism genotypes may be represented by values 0, 1 or 2. The value "0" may indicate that the polymorphism is present on none of the two homologous chromosomes, or in no allele, or is absent. The value "1" may indicate that the polymorphism is present on one of the two homologous chromosomes, or in one allele, or that the polymorphism genotype is heterozygous. The value "2" may indicate that the polymorphism is present on both homologous chromosomes, or in both alleles, or that the polymorphism genotype is homozygous.

The term "predicting a treatment response from the presence or absence of the one or more polymorphism genotypes", as used herein, generally refers to a prediction step that provides a reasonably high prediction performance by associating the presence or absence of a polymorphism genotype with a treatment response. Similarly, the term "polymorphism genotype associated with a treatment response of a subject to treatment with SSR-125543 or a pharmaceutically acceptable salt thereof", as used herein, generally refers to a polymorphism genotype being predicted to be associated with a treatment response with a reasonably high prediction performance. Specifically, the predicting step may comprise determining whether the subject will respond, or has an increased likelihood of responding to the treatment with SSR-125543 or a pharmaceutically acceptable salt thereof; and/or (b) determining whether the subject will not respond, or has a decreased likelihood of responding to the treatment with SSR-125543 or a pharmaceutically acceptable salt thereof. This is generally achieved herein by associating the presence or absence of the one or more polymorphism genotypes as a variable with a value indicative for treatment response within an algorithm for predicting a treatment response to a treatment with SSR-125543 or a pharmaceutically acceptable salt thereof, which is commonly a computer-implemented algorithm. The evaluation of the performance of the algorithm may depend on the problem the algorithm is applied for. If the algorithm is used to identify patients that are likely to respond to treatment with SSR-125543 or a pharmaceutically acceptable salt thereof, the performance is preferably expressed by a high accuracy and/or sensitivity and/or precision. If patients should be identified which are likely not to respond to the treatment with SSR-125543 or a pharmaceutically acceptable salt thereof, specificity and/or negative predictive value can be statistical measures to describe the performance of the prediction algorithm. For optimizing the prediction performance of the predicting step, a step of determining and/or optimizing the algorithm by a machine-learning method in a first subset of the test set and testing the prediction performance in an second independent subset of the test set may be carried out and repeated based on different numbers and groups of polymorphism genotypes, until the desired prediction performance is reached. Specifically, the algorithm for predicting may comprise a classification function (also known as binary classification test), which can comprise one or more statistical analysis methods and/or machine learning methods which are available to one of skill in the art. Specifically, statistical analysis methods and/or machine learning methods to be used in the invention may be selected from the group consisting of artificial neural network learning, decision tree learning, decision tree forest learning, linear discriminant analysis, non-linear discriminant analysis, genetic expression programming, relevance vector machines, linear models, generalized linear models, generalized estimating equations, generalized linear mixed models, the elastic net, the lasso support vector machine learning, Bayesian network learning, probabilistic neural network learning, clustering, and regression analysis, e.g., as described and exemplified herein. Statistical methods and/or machine learning methods from the group mentioned above may exist in different variants, especially applying or not applying prior and posterior weights in the analysis leading to solutions which may be applicable in different settings and may lead to models with more or less explanatory variables. The results of single methods may be used in a method called "ENSEMBLE learning" in which the results of several single analysis with one of the methods mentioned above are combined to arrive at a better prediction using either simply majority vote or using one of the machine learning algorithms with the results of the single analyses again as input to that specific algorithm..

In an exemplary embodiment of the method of the invention, the number of minor alleles for both polymorphism rs74888440 (P1) and rs9813396 (P2) is coded as a numeric variable, which can take one of the following values: 0, 1 or 2, denoting the two variables thus created as V1 for rs74888440 and V2 for rs9813396. Each subject is designated a value of the predictive quantitative variable PQV such that PQV = 0.3205619 + (0.2923413*V1) + (0.2362708 *V2) + (-0.0104643 *V1*V2). Depending on whether a subject's PQV is above or below a value of 0.5, that person is then predicted to not to respond, or to have a decreased likelihood of responding to a treatment with a CRHR1 antagonist (if PQV <= 0.5), or to respond, or to have an increased likelihood of responding to a treatment with a CRHR1 antagonist (if PQV > 0.5). For example, a subject who has no minor alleles at either of the two polymorphisms (homozygous for the common allele at both loci, such that V1 = V2 = 0) is designated a PQV of 0.3205619 and is consequently predicted to be a non-responder. In another example, a subject who is heterozygous at P1 (V1=1) and homozygous for P2 (V2=2) is then designated a PQV of (0.3205619) + ( 0.2923413 *1) + (0.2362708 *2) + (-0.0104643 *1*2) = 1.064516 and is, in consequence, predicted to be a responder. In this example, a sensitivity of 0.6285714 and a specificity of 0.6626506 is reached.

In another exemplary embodiment of the method of the invention, the number of minor alleles for both SNPs rs74888440 (P1) and rs220806 (P2) is coded as a numeric variable, which can take one of the following values: 0, 1 or 2, denoting the two variables thus created as V1 for rs74888440 and V2 for rs220806. Each subject is designated a value of the predictive quantitative variable PQV such that PQV = 0.539548 + (0.460452 *V1) + (-0.1765537 *V2) + (-0.1567797 *V1*V2). Depending on whether a subject's PQV is above or below a value of 0.5, that subject is then predicted to not to respond, or to have a decreased likelihood of responding to a treatment with a CRHR1 antagonist (if PQV <= 0.5), or to respond, or to have an increased likelihood of responding to a treatment with a CRHR1 antagonist (if PQV > 0.5). For example, a subject who has no minor alleles at either of the two SNPs (homozygous for the common allele at both loci, such that V1 = V2 = 0) is designated a PQV of 0.539548 and is consequently predicted to be a responder. In another example, a subject who is heterozygous at SNP1 (V1=1) and homozygous for SNP2 (V2=2) is then designated a PQV of (0.539548) + (0.460452 *1) + (-0.1765537 *2) + (-0.1567797 *1*2) = 0.3333333 and is, in consequence, predicted to be a non-responder. In this example, a sensitivity of 0.6857143 and a specificity of 0.626506 is reached.

In a similar manner, one of skill in the art, having the polymorphisms of Table 2 and the additional information above at hand, will readily derive suitable methods, combinations of methods, parameters and/or coefficients such as those exemplified herein, for use in the methods of the invention, achieving a high performance of prediction.

Preferably, the prediction of the treatment response is made with a high accuracy, sensitivity, precision, specificity and/or negative predictive value.

"Accuracy", "sensitivity", "precision", "specificity" and "negative predictive value" are exemplary statistical measure of the performance of the prediction algorithm. In the following, examples are given for determining the performance of the prediction algorithm.

As used herein, accuracy may be calculated as (number of true positives + number of true negatives) / (number of true positives + number of false positives + number of true negatives + number of false negatives), e.g., (number of patients correctly diagnosed as responding to CRHR1 antagonist + number of patients correctly diagnosed as not responding to CRHR1 antagonist) / (number of patients correctly diagnosed as responding to CRHR1 antagonist + number of patients wrongly diagnosed as responding to CRHR1 antagonist + number of patients correctly diagnosed as not responding to CRHR1 antagonist + number of patients wrongly diagnosed as not responding to CRHR1 antagonist). In some embodiments, the accuracy of prediction is higher than 50%, at least 60%, at least 70%, at least 80% or at least 90%.

As used herein, sensitivity may be calculated as (true positives) / (true positives + false negatives), e.g., (number of patients correctly diagnosed as responding to CRHR1 antagonist) / (number of patients correctly diagnosed as responding to CRHR1 antagonist + number of patients wrongly diagnosed as not responding to CRHR1 antagonist). In some embodiments, the sensitivity of prediction is higher than 50%, at least 60%, at least 70%, at least 80% or at least 90%.

As used herein, precision (also referred to as positive predictive value) may be calculated as (true positives) / (true positives + false positives), e.g.: (number of patients correctly diagnosed as responding to CRHR1 antagonist) / (number of patients correctly diagnosed as responding to CRHR1 antagonist + number of patients wrongly diagnosed as responding to CRHR1 antagonist). In some embodiments, the precision of prediction is higher than 50%, at least 60%, at least 70%, at least 80% or at least 90%.

As used herein, specificity is calculated as (true negatives) / (true negatives + false positives), e.g.: (number of patients correctly diagnosed as not responding to CRHR1 antagonist) / (number of patients correctly diagnosed as not responding to CRHR1 antagonist + number of patients wrongly diagnosed as responding to CRHR1 antagonist). In some embodiments, the specificity of prediction is higher than 50%, at least 60%, at least 70%, at least 80% or at least 90%.

As used herein, negative predictive value is calculated as (true negatives) / (true negatives + false negatives), e.g.: (number of patients correctly diagnosed as not responding to CRHR1 antagonist) / (number of patients correctly diagnosed as not responding to CRHR1 antagonist + number of patients wrongly diagnosed as not responding to CRHR1 antagonist). In some embodiments, the negative predictive value is higher than 50%, at least 60%, at least 70%, at least 80% or at least 90%.

Other statistical measures useful for describing the performance of the prediction algorithm are geometric mean of sensitivity and specificity, geometric mean of positive predictive value and negative predictive value, F-measure and area under ROC curve, and the positive and negative likelihood ratios, the false discovery rate and Matthews correlation coefficient. These measures and method for their determination are well known in the art.

In general, a prediction algorithm with high sensitivity may have low specificity and *vice versa.* For the purposes of the present invention, it is generally preferable that the prediction algorithm is based on a number of polymorphism genotypes selected from Table 2 sufficient to achieve a sensitivity and specificity of higher than 50% each, optionally at least 60% each, at least 70% each, at least 80% each, or at least 90% each.

For a prediction whether a patient will respond, or has an increased likelihood of responding to a treatment with SSR-125543 or a pharmaceutically acceptable salt thereof, the prediction algorithm may be based on a number of polymorphisms sufficient to achieve a prediction sensitivity and/or precision of higher than 50%, optionally at least 60%, at least 70%, at least 80%, or at least 90%.

For the prediction whether the subject will not respond, or has a decreased likelihood of responding to a treatment with SSR-125543 or a pharmaceutically acceptable salt thereof, the prediction algorithm may be based on a number of polymorphisms sufficient to achieve a prediction specificity and/or negative predictive value of higher than 50%, optionally at least 60%, at least 70%, at least 80%, or at least 90%.

For a prediction whether a patient responds to a treatment with SSR-125543 or a pharmaceutically acceptable salt thereof or not, the prediction algorithm may be based on a number of polymorphisms sufficient to achieve sensitivity and/or precision and/or specificity and/or negative predictive value of higher than 50%, optionally at least 60%, at least 70%, at least 80%, or at least 90%.

Based on the disclosure of the present invention, in particular of the highly useful set of polymorphism genotypes disclosed in Table 2, the skilled person in the art is enabled to employ the statistical analysis methods and/or machine-learning methods disclosed herein and to identify suitable parameters for further improving the prediction performance, as defined above. The whole statistical work-flow can be automated by the use of an algorithm as described above, implemented and/or stored on a machine-readable medium, e.g., implemented and/or stored on a computer.

Typically, at least 1, at least 2, at least 3, at least 4, at least 5, at least 6, at least 7, at least 8, at least 9, at least 10, at least 20, at least 30, at least 50, at least 100, at least 100, at least 200 or all polymorphism genotypes disclosed in Table 2 are used for predicting the treatment response to SSR-125543 or a pharmaceutically acceptable salt thereof.

Using various such polymorphism genotype sets and statistical analysis methods as described above, the present invention consistently achieves a high predictive performance in directly predicting a clinical response. For instance, Example 1 describes a study with clinical data from 300 enrolled patients, wherein 150 polymorphism genotypes were used for predicting the clinical treatment response of subjects to a treatment with SSR-125543. Therein, a sensitivity of about 78% and a specificity of about 73% was observed, which is considered to reflect a superior reliability in predicting both true positive responses and true negative responses. Further, Example 2 provides examples of minimal subsets of only one, two, four or eight polymorphism genotypes selected from the group of polymorphism genotypes disclosed in Table 2, achieving a performance of predicting a clinical treatment response with values for specificity and sensitivity which are still higher than 60%, or even higher than 70%. Predictive performance in terms of sensitivity and specificity can be further increased to at least 75% each, e.g., by including specific combinations of 32 polymorphism genotypes, as is also shown in Example 2.

Furthermore, in patients with depressive symptoms and/or anxiety symptoms, another embodiment of the method of treatment, the step of predicting a treatment response as described above may be also accompanied by analyzing the rapid-eye-movement (REM) sleep, e.g. during night sleep of a patient in a sleep EEC. In some embodiments, an alteration in REM sleep may serve as an additional biomarker to identify subjects who would benefit from treatment with SSR-125543 or a pharmaceutically acceptable salt thereof. REM sleep typically comprises a characteristic coincidence of nearly complete muscle atonia, a waking-like pattern of brain oscillations and rapid eye movements (REMs). The amount of REMs during consecutive REM sleep episodes is usually increasing throughout the night. Single and short REMs with low amplitude can be characteristic for initial parts of REM sleep. The amount of REMs, in particular within the first REM sleep episode, can be of clinical relevance. Recent clinical and animal data supports the correlation of REM density with an increased CRH activity. For example, Kimura et al. (Mol. Psychiatry, 2010) showed that mice overexpressing CRH in the forebrain exhibit constantly increased rapid eye movement (REM) sleep compared to wildtype mice. In addition, it could be shown that treatment with another CRHR1 antagonist, DMP696 could reverse the REM enhancement. Further, the polymorphism analysis and REM density analysis as described herein may be combined for predicting the response of patients with depressive symptoms and/or anxiety symptoms to treatment with SSR-125543 or a pharmaceutically acceptable salt thereof. The REM analysis may be carried out before, concomitant or after the polymorphism analysis. For example, the REM density analysis may be carried out on subjects that where identified by the polymorphism analysis as responding, or having an increased likelihood of responding to the treatment with SSR-125543 or a pharmaceutically acceptable salt thereof; or as not responding, or having a decreased likelihood of responding to the treatment with SSR-125543 or a pharmaceutically acceptable salt thereof. The recording of a "sleep-EEG" (also referred to "polysomatic recordings") may comprise electroencephalography (EEG), vertical and horizontal elecrooculography (EOG), electromyography (EMG) and/or electrocardiography (ECG). In EOG, muscle activities of right and left eye may be recorded by electrooculograms (one or typically two channels) in order to visualize the phasic components of REM sleep. "REM analysis" or "analyzing the rapid-eye-movement (REM)" may refer to a method comprising recoding of muscle activities of right and left eye by EOG and then analyzing the electrooculogram. The recognition of REM in the electrooculogram may be done manually, for example by standard guidelines Rechtschaffen and Kales, 1968, Bethesda, MD: National Institute of Neurological Diseases and Blindness.

According to the invention, SSR-125543 or a pharmaceutically acceptable salt thereof is used in the method of treatment of the conditions which are treatable by SSR-125543 or a pharmaceutically acceptable salt thereof.

SSR-125543 or a pharmaceutically acceptable salt thereof may be administered as the raw chemical but the active ingredient is preferably formulated in a pharmaceutical composition suitable for administration by any convenient route, preferably in a form suitable for use in human medicine. The treatment can comprise any suitable route of administration, such as oral, buccal, parenteral, topical (including ophthalmic and nasal), depot or rectal administration or in a form suitable for administration by inhalation or insufflation (either through the mouth or nose) administration of SSR-125543 or a pharmaceutically acceptable salt thereof.

CRHR1 antagonists can be administered at any suitable efficacious dose, which one skilled in the art will readily adapt, e.g., to the specific condition to be treated. For many therapeutic indications as encompassed herein, a dose of about 1 mg to about 2000 mg per day, about 2 mg to about 1000 mg per day, about 5 mg to about 500 mg per day, about 10 mg to about 250 mg, or about 20 to about 100 mg daily will be efficacious. It will be appreciated that it may be necessary to make routine variations to the dosage, depending on the age and condition of the patient and the precise dosage will be ultimately at the discretion of the attendant physician or veterinarian. The dosage will also depend on the route of administration and the particular compound selected. Thus, for parenteral administration a daily dose will typically be in the range of 1 to about 100 mg, preferably 1 to 80 mg per day. For oral administration a daily dose will typically be within the range of 1 to 300 mg e.g. 1 to 100 mg of a CRHR1 antagonist. For instance, in treating depressive symptoms and/or anxiety symptoms, daily oral doses of about 10 mg, about 20 mg, or about 100 mg of SSR-125543 or a pharmaceutically acceptable salt thereof can be efficacious.

### Compositions, kits and arrays for use in the method of treatment

The disclosure further provides compositions comprising polynucleotides (e.g., probes), as well as kits and arrays for use in the detection step of the method of treatment. Polynucleotide compositions, kits, and arrays are useful in, e.g., detecting the presence of (a) one or more polymorphism genotypes as disclosed in Table 2, (b) one or more polymorphism genotypes being in linkage disequilibrium with any one of the polymorphism genotypes of (a), or a combination of (a) and (b). The compositions, kits and arrays are further useful for predicting the treatment response of a subject to treatment with a CRHR1 antagonist.

The compositions, kits or arrays can include at least one polynucleotide capable of specifically hybridizing to a nucleic acid comprising: (a) at least one polymorphism genotype as disclosed in Table 2; (b) at least one polymorphism genotype being in linkage disequilibrium with any one of the polymorphism genotypes of (a), or (c) a combination of (a) and (b). The at least one polynucleotide can comprise less than 100,000, less than 90,000, less than 80,000, less than 70,000, less than 60,000, less than 50,000, less than 40,000, less than 30,000, less than 20,000, less than 15,000, less than 10,000, less than 5,000, less than 4,000, less than 3,000, less than 2,000, less than 1,500, less than 1,000, less than 750, less than 500, less than 200, less than 100, or less than 50 different polynucleotides in total. Specifically, the compositions, kits or arrays can include at least two, at least three, at least four, at least five, at least six, at least seven, at least eight, at least nine, at least 10, at least 11, at least 12, at least 15, at least 20, or at least 30, or at least 50, or at least 100, or at least 200, or 274 polynucleotides capable of specifically hybridizing to each of at least two, at least three, at least four, at least five, at least six, at least seven, at least eight, at least nine, at least 10, at least 11, at least 12, at least 15, at least 20, or at least 30, or at least 50, or at least 100, or at least 200, or 274 of (a) at least one polymorphism genotype as disclosed in Table 2; (b) at least one polymorphism genotype being in linkage disequilibrium with any one of the polymorphism genotypes of (a), or (c) a combination of (a) and (b).

A polynucleotide can include a coding sequence or non-coding sequence (e.g., exons, introns, or 5' or 3' regulatory sequences). The polynucleotide can also be single or double-stranded and of variable length. The length of one strand of a polynucleotide capable of specifically hybridizing to a nucleic acid comprising: (a) at least one a polymorphism genotype as disclosed in Table 2; (b) at least one polymorphism genotype being in linkage disequilibrium with any one of the polymorphism genotypes of (a), or (c) a combination of (a) and (b) can be about six nucleotides (e.g., about seven nucleotides, about eight nucleotides, about nine nucleotides, about 10 nucleotides, about 12 nucleotides, about 13 nucleotides, about 14 nucleotides, about 15 nucleotides, about 20 nucleotides, about 25 nucleotides, about 30 nucleotides, about 35 nucleotides, about 40 nucleotides, about 50 nucleotides, about 75 nucleotides, about 100 nucleotides, or about 150 or more nucleotides) in length. As is commonly known in the art, a longer polynucleotide often allows for higher stringency hybridization and wash conditions. The polynucleotide can be DNA, RNA, modified DNA or RNA, or a hybrid, where the nucleic acid contains any combination of deoxyribo- and ribo-nucleotides, and any combination of uracil, adenine, thymine, cytosine and guanine, as well as other bases such as inosine, xanthine, and hypoxanthine.

The polynucleotides can be attached to a solid support, e.g., a porous or non-porous material that is insoluble. The polynucleotides can be arranged in an array on the solid support, e.g., in a microarray. A solid support can be composed of a natural or synthetic material, an organic or inorganic material. The composition of the solid support on which the polynucleotide sequences are attached by either 5' or 3' terminal attachment generally depend on the method of attachment (e.g., covalent attachment). Suitable solid supports include, but are not limited to, plastics, resins, polysaccharides, silica or silica-based materials, functionalized glass, modified silicon, carbon, metals, inorganic glasses, membranes, nylon, natural fibers such as silk, wool and cotton, or polymers. The material comprising the solid support can have reactive groups such as carboxy, amino, or hydroxyl groups, which are used for attachment of the polynucleotides. Polymeric solid supports can include, e.g., polystyrene, polyethylene glycol tetraphthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly)vinylidenefluoride, polycarbonate, or polymethylpentene (see, e.g., U.S. Patent No. 5,427,779). Alternatively, polynucleotides can be attached to the solid support without the use of such functional groups.

Arrays of polynucleotides can also be conjugated to solid support particles. Many suitable solid support particles are known in the art and illustratively include, e.g., particles, such as Luminex®-type encoded particles, magnetic particles, and glass particles. Exemplary particles that can be used can have a variety of sizes and physical properties. Particles can be selected to have a variety of properties useful for particular experimental formats. For example, particles can be selected that remain suspended in a solution of desired viscosity or to readily precipitate in a solution of desired viscosity. Particles can be selected for ease of separation from sample constituents, for example, by including purification tags for separation with a suitable tag-binding material, paramagnetic properties for magnetic separation, and the like. Encoded particles can be used. Each particle includes a unique code (such as a bar code, luminescence code, fluorescence code, a nucleic acid code, and the like). Encoding can be used to provide particles for evaluating different nucleic acids in a single biological sample. The code is embedded (for example, within the interior of the particle) or otherwise attached to the particle in a manner that is stable through hybridization and analysis. The code can be provided by any detectable means, such as by holographic encoding, by a fluorescence property, color, shape, size, weight, light emission, quantum dot emission and the like to identify particle and thus the capture probes immobilized thereto. Encoding can also be the ratio of two or more dyes in one particle that is different than the ratio present in another particle. For example, the particles may be encoded using optical, chemical, physical, or electronic tags. Examples of such coding technologies are optical bar codes fluorescent dyes, or other means. The particle code can be a nucleic acid, e.g., a single stranded nucleic acid.

Different encoded particles can be used to detect or measure multiple nucleic acids (e.g., polymorphism genotypes or mRNAs) in parallel, so long as the encoding can be used to identify the polynucleotide (corresponding to an analyte nucleic acid) on a particular particle, and hence the presence or amount of the analyte nucleic acid (e.g., a polymorphism genotypes or mRNA from a biological sample) being evaluated. A sample can be contacted with a plurality of such coded particles. When the particles are evaluated, e.g., using a fluorescent scanner, the particle code is read as is the fluorescence associated with the particle from any probe used to evaluate modification of the intact substrate associated with the particles.

One exemplary platform utilizes mixtures of fluorescent dyes impregnated into polymer particles as the means to identify each member of a particle set to which a specific capture probe has been immobilized. Another exemplary platform uses holographic barcodes to identify cylindrical glass particles. For example, Chandler et al. (U.S. Patent No. 5,981,180) describes a particle-based system in which different particle types are encoded by mixtures of various proportions of two or more fluorescent dyes impregnated into polymer particles. Soini (U.S. Patent No. 5,028,545) describes a particle-based multiplexed assay system that employs time-resolved fluorescence for particle identification. Fulwyler (U.S. Patent No. 4,499,052) describes an exemplary method for using particle distinguished by color and/or size. U.S. Publication Nos. 2004-0179267, 2004-0132205, 2004-0130786, 2004-0130761, 2004-0126875, 2004-0125424, and 2004-0075907 describe exemplary particles encoded by holographic barcodes.

U.S. Patent No. 6,916,661 describes polymeric microparticles that are associated with nanoparticles that have dyes that provide a code for the particles. The polymeric microparticles can have a diameter of less than one millimeter, e.g., a size ranging from about 0.1 to about 1,000 micrometers in diameter, e.g., 3-25 µm or about 6-12 µm. The nanoparticles can have, e.g., a diameter from about 1 nanometer (nm) to about 100,000 nm in diameter, e.g., about 10 - 1,000 nm or 200 - 500 nm.

An "array", as used herein, refers to a plurality of polynucleotides comprised in the composition or kit being immobilized at predetermined positions on a solid support such that each polynucleotide can be identified by its position.

The compositions, kits and arrays can be, but are not necessarily used in genome-wide genotyping analysis, but for efficient, low cost, and application-specific genotyping analysis, can be tailored to be used in the methods of treatment of the invention for detecting and/or predicting a treatment response to a treatment with a CRHR1 antagonist, as disclosed herein. Thus, the array of polynucleotides can have less than 100,000 (e.g., less than 90,000; less than 80,000; less than 70,000; less than 60,000; less than 50,000; less than 40,000; less than 30,000; less than 20,000; less than 15,000; less than 10,000; less than 5,000; less than 4,000; less than 3,000; less than 2,000; less than 1,500; less than 1,000; less than 750; less than 500, less than 200, less than 100, or less than 50) different polynucleotides.

The kits described above can, optionally, contain instructions for detecting the presence or absence of the at least one polymorphism genotype in a sample obtained from a subject. The kits can include one or more reagents for processing a biological sample. For example, a kit can include reagents for isolating mRNA or genomic DNA from a biological sample and/or reagents for amplifying isolated mRNA (e.g., reverse transcriptase, primers for reverse transcription or PCR amplification, or dNTPs) and/or genomic DNA. The kits can also, optionally, contain one or more reagents for detectably-labeling an mRNA, mRNA amplicon, genomic DNA or DNA amplicon, which reagents can include, e.g., an enzyme such as a Klenow fragment of DNA polymerase, T4 polynucleotide kinase, one or more detectably-labeled dNTPs, or detectably-labeled gamma phosphate ATP (e.g., 33P-ATP). The kits can include a software package for analyzing the results of, e.g., a microarray analysis. The kits described herein can also, optionally, include instructions for administering a CRHR1 antagonist where presence or absence of one or more polymorphism genotypes detectable by the plurality of polynucleotides or the array predicts that a subject will response to a CRHR1 antagonist.

The following are examples of the practice of the invention. They are not to be construed as limiting the scope of the invention in any way.

### EXAMPLES

### Example 1

Based on basic science studies, the role of CRH was recognized as causal for signs and symptoms prevalent in depression, rendering blocking of CRH/CRHR1 signalling as a viable treatment option. Further clinical findings have found that CRH is elevated in a subgroup of patients with depression, where CRH causes core symptoms. Compound SSR-125543 has been developed elsewhere as a specific CRHR1 antagonist blocking the effect of CRH. A clinical trial evaluating the efficacy and tolerability of SSR-125543 in comparison to placebo and a standard antidepressant has been carried out previously without having predicted the treatment response according to the invention. However, based on additional studies (not published), it was recognized that among patients diagnosed with major depression, only a fraction of 20-30% has central CRH over-activity. Thus, a substantial fraction of non-stratified patients might not show a treatment response, in view of about 70-80% of patients treated with the CRHR1 antagonist not having a central CRH increase. Given the pharmacological specificity, only patients with central CRH-over-activity are likely to benefit from treatment with SSR-125543.

Here, a method of predicting a clinical treatment response (e.g., as measured by the HAM-D score) has been devised, which detects one or more polymorphism genotypes selected from the polymorphism genotypes disclosed in Table 2, using a chip containing probes specific for these polymorphism genotypes, allowing for identification of depressive patients being likely to respond to a treatment with SSR-125543. DNA samples obtained from 300 subjects enrolled in the earlier clinical trial, as mentioned above, were extensively analyzed by polymorphism genotyping. Using a machine-learning algorithm as described herein, polymorphism genotypes predictive of a response to SSR-125543 were identified, as disclosed in Table 2. Further, 150 or more polymorphism genotypes of this set were used to further "train" the algorithm, assisted by common machine-learning algorithms as described herein, and to test the prediction. Thus, having the set of useful polymorphism genotypes as disclosed in Table 2, at hand, a prediction algorithm can be readily devised, which provides superior prediction of a clinical response with high sensitivity and specificity. As is shown in Table 3, test predictions of a clinical response with a sensitivity of about 78% and a specificity of about 73% have been achieved.

**Table 3**

| | | Observed phenotype | |
|---|---|---|---|
| | | Good response | Poor response |
| Test prediction | Good response | **21** | 13 |
| | Poor response | 6 | **36** |
| | | Sensitivity 78% | Specificity 73% |

To exclude the possibility that the polymorphism genotypes disclosed herein are merely identifying patients that are good responders to any kind of drug intervention, the performance of the method among patients treated with the standard antidepressant escitalopram used as comparator in the earlier clinical trial has also been tested. The sensitivity was 50%, and specificity was 43%, and thus insensitive and unspecific regarding prediction of response to a standard antidepressant, see Table 4. Therefore, the present method is to be considered highly specific for predicting the response to SSR-125543.

**Table 4**

| | | Observed phenotype | |
|---|---|---|---|
| | | Good response | Poor response |
| Test prediction | Good response | **23** | 17 |
| | Poor response | 23 | **13** |
| | | Sensitivity 50% | Specificity 43% |

The above results were further challenged by considering a "lucky split" between the training and the testing cohort. Another 10.000 random splits were calculated which corroborated the initial result, achieving an odds ratio of 5, which indicates that chances of non-response are 5 times higher if the CRH genotyping analysis described herein predicts poor response. Transforming these findings into a time course curve where those depressed patients that where tested positive in the CRH genotyping analysis and treated with SSR-125543 were compared with patients treated by placebo resulted in a clear superiority of the investigational drug, see Figure 1. The time course curves revealed a marked difference between placebo and SSR-125543 beginning after 2 weeks of treatment, as measured using, e.g., the HAM-D scale. The difference in response between patients treated with SSR-125543 and those under placebo is significant (p < 0.01). In essence, subjects which are tested positive using the method of prediction described herein, based on a CRH genotyping analysis using 150 of the polymorphism genotypes disclosed in Table 2, constitute 28% of the overall patient sample and 78% patients from this sample were responders when treated with SSR-125543.

### Example 2

To further evaluate the usefulness of the set of polymorphism genotypes provided in Table 2, further predictions have been tested using minimal subsets selected as prediction variables. As few as singular polymorphism genotypes selected from Table 2, as well as subsets of two, four or eight polymorphism genotypes selected from Table 2 proved useful in the method of predicting a clinical response, e.g., as measured by the HAM-D scale.

Treatment response to an anti-depressant therapy comprising SSR-125543 was predicted based on the same patient data of the earlier clinical trial and polymorphism genotyping set as described above, using statistical tools selected from the group consisting of random forests, support vector machines, neural networks, linear discriminant analyses, clustering methods such as k-nearest neighbours and their respective derivatives, linear models and their derivatives, as well as their combinations.

Surprisingly, even this univariate, bivariate, quadrivariate or octovariate analyses using combinations of polymorphism genotypes as disclosed in Tables 2, 5-7 herein, yielded clinical response predictions of a quality significantly better (i.e. both sensitivity and specificity > 50%) than randomness, based on assessing the P-value of concordance between observed and predicted outcome in a 10-fold cross-validation procedure.

In particular, a total number of 78 singular polymorphism genotypes was identified with nominally significant P-values. Of those, 46 yielded a specificity and sensitivity of > 50% each in predicting a clinical response. One singular polymorphism yielded both a sensitivity and specificity of higher than 60% each in predicting a clinical response.

Of all tested combinations of two of the univariate significantly predicting polymorphisms, 237 exhibited both a sensitivity and specificity of at least 60% each in predicting a clinical response. Finally, a number of 46 tested combinations of two of the univariate significantly predicting polymorphism genotypes yielded a sensitivity and specificity beyond 65% each in predicting a clinical response, see Table 5.

**Table 5 - Bivariate sets of polymorphism genotypes**

| P_ID1 | P_ID2 | rs_p1 | p2 | p-value | sensitivity | specificity |
|---|---|---|---|---|---|---|
| 11 | 181 | rs74888440 | rs9813396 | 0.00027897 | 0.62857143 | 0.6626506 |
| 11 | 192 | rs74888440 | rs72693005 | 0.00060709 | 0.67142857 | 0.60240964 |
| 11 | 207 | rs74888440 | rs220806 | 0.00010088 | 0.68571429 | 0.62650602 |
| 11 | 218 | rs74888440 | rs1944887 | 0.00015583 | 0.62857143 | 0.6746988 |
| 11 | 226 | rs74888440 | rs532996 | 0.00082753 | 0.62857143 | 0.63855422 |
| 11 | 227 | rs74888440 | rs9571939 | 0.00082753 | 0.62857143 | 0.63855422 |
| 11 | 228 | rs74888440 | rs2173530 | 0.00082753 | 0.62857143 | 0.63855422 |
| 11 | 244 | rs74888440 | rs2044070 | 0.00352822 | 0.62857143 | 0.60240964 |
| 11 | 245 | rs74888440 | rs920640 | 0.00352822 | 0.62857143 | 0.60240964 |
| 112 | 175 | rs2260882 | rs7648662 | 2.12E-05 | 0.64285714 | 0.69879518 |
| 112 | 237 | rs2260882 | rs12917505 | 0.00090174 | 0.61428571 | 0.65060241 |
| 112 | 238 | rs2260882 | rs16977818 | 2.19E-05 | 0.71428571 | 0.62650602 |
| 112 | 240 | rs2260882 | rs10851628 | 0.00039921 | 0.65714286 | 0.62650602 |
| 112 | 243 | rs2260882 | rs6493965 | 0.00137357 | 0.62857143 | 0.62650602 |
| 112 | 245 | rs2260882 | rs920640 | 0.0006793 | 0.65714286 | 0.61445783 |
| 112 | 246 | rs2260882 | rs920638 | 0.00202984 | 0.64285714 | 0.60240964 |
| 112 | 250 | rs2260882 | rs735164 | 0.00383837 | 0.61428571 | 0.61445783 |
| 112 | 277 | rs2260882 | rs2044230 | 0.00048656 | 0.62857143 | 0.65060241 |
| 116 | 179 | rs2257474 | rs6549407 | 0.00352822 | 0.62857143 | 0.60240964 |
| 116 | 182 | rs2257474 | rs12489026 | 0.00030332 | 0.61428571 | 0.6746988 |
| 116 | 191 | rs2257474 | rs1383699 | 7.55E-05 | 0.71428571 | 0.60240964 |
| 116 | 234 | rs2257474 | rs8042817 | 0.00011443 | 0.67142857 | 0.63855422 |
| 116 | 235 | rs2257474 | rs28811003 | 0.00039921 | 0.65714286 | 0.62650602 |
| 121 | 127 | rs2028629 | rs79320848 | 0.00383837 | 0.61428571 | 0.61445783 |
| 121 | 184 | rs2028629 | rs11715827 | 0.00015583 | 0.62857143 | 0.6746988 |
| 121 | 185 | rs2028629 | rs58882373 | 0.00015583 | 0.62857143 | 0.6746988 |
| 121 | 191 | rs2028629 | rs1383699 | 0.00082753 | 0.62857143 | 0.63855422 |
| 121 | 202 | rs2028629 | rs4836256 | 2.30E-05 | 0.62857143 | 0.71084337 |
| 121 | 233 | rs2028629 | rs929610 | 4.11E-05 | 0.64285714 | 0.68674699 |
| 121 | 237 | rs2028629 | rs12917505 | 7.00E-05 | 0.65714286 | 0.6626506 |
| 121 | 238 | rs2028629 | rs16977818 | 7.75E-05 | 0.64285714 | 0.6746988 |
| 121 | 239 | rs2028629 | rs11071351 | 0.00112948 | 0.65714286 | 0.60240964 |
| 121 | 240 | rs2028629 | rs10851628 | 3.32E-06 | 0.7 | 0.6746988 |
| 121 | 241 | rs2028629 | rs930473 | 7.72E-06 | 0.68571429 | 0.6746988 |
| 121 | 242 | rs2028629 | rs1441824 | 0.00011443 | 0.67142857 | 0.63855422 |
| 121 | 243 | rs2028629 | rs6493965 | 1.53E-05 | 0.68571429 | 0.6626506 |
| 121 | 244 | rs2028629 | rs2044070 | 3.32E-06 | 0.7 | 0.6746988 |
| 121 | 245 | rs2028629 | rs920640 | 3.72E-05 | 0.65714286 | 0.6746988 |
| 121 | 246 | rs2028629 | rs920638 | 3.32E-06 | 0.7 | 0.6746988 |
| 123 | 218 | rs4812040 | rs1944887 | 0.00125068 | 0.64285714 | 0.61445783 |
| 123 | 235 | rs4812040 | rs28811003 | 0.00052981 | 0.61428571 | 0.6626506 |
| 127 | 192 | rs79320848 | rs72693005 | 0.00035634 | 0.67142857 | 0.61445783 |
| 127 | 207 | rs79320848 | rs220806 | 0.00025408 | 0.64285714 | 0.65060241 |
| 127 | 218 | rs79320848 | rs1944887 | 0.00030332 | 0.61428571 | 0.6746988 |
| 132 | 184 | rs6026567 | rs11715827 | 2.69E-06 | 0.61428571 | 0.75903614 |
| 132 | 185 | rs6026567 | rs58882373 | 1.22E-05 | 0.61428571 | 0.73493976 |
| 132 | 213 | rs6026567 | rs2935752 | 0.00030332 | 0.61428571 | 0.6746988 |
| 132 | 214 | rs6026567 | rs2935751 | 0.00030332 | 0.61428571 | 0.6746988 |
| 132 | 237 | rs6026567 | rs12917505 | 4.82E-05 | 0.61428571 | 0.71084337 |
| 132 | 238 | rs6026567 | rs16977818 | 9.16E-05 | 0.61428571 | 0.69879518 |
| 132 | 239 | rs6026567 | rs11071351 | 0.00242522 | 0.61428571 | 0.62650602 |
| 132 | 240 | rs6026567 | rs10851628 | 7.00E-05 | 0.65714286 | 0.6626506 |
| 132 | 241 | rs6026567 | rs930473 | 0.00030332 | 0.61428571 | 0.6746988 |
| 132 | 244 | rs6026567 | rs2044070 | 0.00027897 | 0.62857143 | 0.6626506 |
| 133 | 190 | rs968519 | rs1383707 | 0.00016904 | 0.61428571 | 0.68674699 |
| 133 | 238 | rs968519 | rs16977818 | 0.00052981 | 0.61428571 | 0.6626506 |
| 133 | 240 | rs968519 | rs10851628 | 9.16E-05 | 0.61428571 | 0.69879518 |
| 133 | 241 | rs968519 | rs930473 | 9.16E-05 | 0.61428571 | 0.69879518 |
| 133 | 243 | rs968519 | rs6493965 | 9.16E-05 | 0.61428571 | 0.69879518 |
| 133 | 245 | rs968519 | rs920640 | 0.00052981 | 0.61428571 | 0.6626506 |
| 141 | 157 | rs6092704 | rs2242071 | 0.0006793 | 0.65714286 | 0.61445783 |
| 141 | 181 | rs6092704 | rs9813396 | 4.11E-05 | 0.71428571 | 0.61445783 |
| 141 | 187 | rs6092704 | rs10034039 | 0.00012826 | 0.65714286 | 0.65060241 |
| 141 | 190 | rs6092704 | rs1383707 | 0.00012826 | 0.65714286 | 0.65060241 |
| 141 | 191 | rs6092704 | rs1383699 | 0.00202984 | 0.64285714 | 0.60240964 |
| 141 | 212 | rs6092704 | rs3133622 | 0.00383837 | 0.61428571 | 0.61445783 |
| 141 | 259 | rs6092704 | rs487011 | 0.00149683 | 0.61428571 | 0.63855422 |
| 155 | 207 | rs7523266 | rs220806 | 0.00090174 | 0.61428571 | 0.65060241 |
| 156 | 207 | rs6686061 | rs220806 | 0.00090174 | 0.61428571 | 0.65060241 |
| 157 | 215 | rs2242071 | rs4570614 | 0.00352822 | 0.62857143 | 0.60240964 |
| 168 | 192 | rs809482 | rs72693005 | 0.00352822 | 0.62857143 | 0.60240964 |
| 176 | 234 | rs616870 | rs8042817 | 0.00593832 | 0.61428571 | 0.60240964 |
| 179 | 223 | rs6549407 | rs876270 | 0.00039921 | 0.65714286 | 0.62650602 |
| 179 | 224 | rs6549407 | rs11834041 | 0.00020436 | 0.67142857 | 0.62650602 |
| 179 | 248 | rs6549407 | rs7165629 | 0.00015717 | 0.7 | 0.60240964 |
| 180 | 187 | rs6766242 | rs10034039 | 4.11E-05 | 0.71428571 | 0.61445783 |
| 180 | 220 | rs6766242 | rs7121326 | 0.00082753 | 0.62857143 | 0.63855422 |
| 180 | 223 | rs6766242 | rs876270 | 7.75E-05 | 0.64285714 | 0.6746988 |
| 180 | 224 | rs6766242 | rs11834041 | 3.72E-05 | 0.65714286 | 0.6746988 |
| 180 | 227 | rs6766242 | rs9571939 | 0.00593832 | 0.61428571 | 0.60240964 |
| 180 | 234 | rs6766242 | rs8042817 | 0.00030332 | 0.61428571 | 0.6746988 |
| 180 | 235 | rs6766242 | rs28811003 | 0.00090174 | 0.61428571 | 0.65060241 |
| 182 | 187 | rs12489026 | rs10034039 | 2.94E-05 | 0.68571429 | 0.65060241 |
| 182 | 188 | rs12489026 | rs17616338 | 0.00052981 | 0.61428571 | 0.6626506 |
| 182 | 218 | rs12489026 | rs1944887 | 0.00383837 | 0.61428571 | 0.61445783 |
| 182 | 224 | rs12489026 | rs11834041 | 4.82E-05 | 0.61428571 | 0.71084337 |
| 184 | 218 | rs11715827 | rs1944887 | 0.00082753 | 0.62857143 | 0.63855422 |
| 184 | 219 | rs11715827 | rs10894873 | 0.00383837 | 0.61428571 | 0.61445783 |
| 184 | 236 | rs11715827 | rs894342 | 4.48E-05 | 0.62857143 | 0.69879518 |
| 184 | 237 | rs11715827 | rs12917505 | 2.30E-05 | 0.62857143 | 0.71084337 |
| 184 | 238 | rs11715827 | rs16977818 | 2.30E-05 | 0.62857143 | 0.71084337 |
| 184 | 239 | rs11715827 | rs11071351 | 8.72E-06 | 0.67142857 | 0.68674699 |
| 184 | 240 | rs11715827 | rs10851628 | 1.14E-05 | 0.62857143 | 0.72289157 |
| 184 | 241 | rs11715827 | rs930473 | 4.82E-05 | 0.61428571 | 0.71084337 |
| 184 | 242 | rs11715827 | rs1441824 | 2.30E-05 | 0.62857143 | 0.71084337 |
| 184 | 243 | rs11715827 | rs6493965 | 9.16E-05 | 0.61428571 | 0.69879518 |
| 184 | 244 | rs11715827 | rs2044070 | 4.48E-05 | 0.62857143 | 0.69879518 |
| 184 | 245 | rs11715827 | rs920640 | 1.06E-05 | 0.64285714 | 0.71084337 |
| 184 | 246 | rs11715827 | rs920638 | 2.12E-05 | 0.64285714 | 0.69879518 |
| 185 | 219 | rs58882373 | rs10894873 | 0.00137357 | 0.62857143 | 0.62650602 |
| 185 | 234 | rs58882373 | rs8042817 | 0.00149683 | 0.61428571 | 0.63855422 |
| 185 | 236 | rs58882373 | rs894342 | 0.00015583 | 0.62857143 | 0.6746988 |
| 185 | 237 | rs58882373 | rs12917505 | 1.14E-05 | 0.62857143 | 0.72289157 |
| 185 | 238 | rs58882373 | rs16977818 | 2.30E-05 | 0.62857143 | 0.71084337 |
| 185 | 239 | rs58882373 | rs11071351 | 8.72E-06 | 0.67142857 | 0.68674699 |
| 185 | 240 | rs58882373 | rs10851628 | 1.14E-05 | 0.62857143 | 0.72289157 |
| 185 | 241 | rs58882373 | rs930473 | 4.82E-05 | 0.61428571 | 0.71084337 |
| 185 | 242 | rs58882373 | rs1441824 | 2.30E-05 | 0.62857143 | 0.71084337 |
| 185 | 243 | rs58882373 | rs6493965 | 4.48E-05 | 0.62857143 | 0.69879518 |
| 185 | 244 | rs58882373 | rs2044070 | 4.48E-05 | 0.62857143 | 0.69879518 |
| 185 | 245 | rs58882373 | rs920640 | 4.48E-05 | 0.62857143 | 0.69879518 |
| 185 | 246 | rs58882373 | rs920638 | 4.48E-05 | 0.62857143 | 0.69879518 |
| 186 | 236 | rs12490095 | rs894342 | 2.57E-06 | 0.62857143 | 0.74698795 |
| 187 | 188 | rs10034039 | rs17616338 | 8.78E-05 | 0.7 | 0.61445783 |
| 187 | 193 | rs10034039 | rs1170303 | 0.00090174 | 0.61428571 | 0.65060241 |
| 187 | 198 | rs10034039 | rs66624622 | 0.00015583 | 0.62857143 | 0.6746988 |
| 187 | 215 | rs10034039 | rs4570614 | 0.00052981 | 0.61428571 | 0.6626506 |
| 187 | 216 | rs10034039 | rs4758040 | 0.00014215 | 0.64285714 | 0.6626506 |
| 187 | 239 | rs10034039 | rs11071351 | 0.00030332 | 0.61428571 | 0.6746988 |
| 188 | 191 | rs17616338 | rs1383699 | 0.00018028 | 0.68571429 | 0.61445783 |
| 189 | 218 | rs80049044 | rs1944887 | 0.00018028 | 0.68571429 | 0.61445783 |
| 190 | 193 | rs1383707 | rs1170303 | 0.00039921 | 0.65714286 | 0.62650602 |
| 190 | 212 | rs1383707 | rs3133622 | 1.61E-06 | 0.75714286 | 0.62650602 |
| 190 | 216 | rs1383707 | rs4758040 | 0.00039921 | 0.65714286 | 0.62650602 |
| 190 | 234 | rs1383707 | rs8042817 | 1.53E-05 | 0.74285714 | 0.60240964 |
| 190 | 237 | rs1383707 | rs12917505 | 0.00027897 | 0.62857143 | 0.6626506 |
| 190 | 242 | rs1383707 | rs1441824 | 0.00149683 | 0.61428571 | 0.63855422 |
| 190 | 252 | rs1383707 | rs4610906 | 0.00090174 | 0.61428571 | 0.65060241 |
| 191 | 216 | rs1383699 | rs4758040 | 0.00137357 | 0.62857143 | 0.62650602 |
| 191 | 234 | rs1383699 | rs8042817 | 0.00015717 | 0.7 | 0.60240964 |
| 191 | 235 | rs1383699 | rs28811003 | 0.00031476 | 0.68571429 | 0.60240964 |
| 191 | 237 | rs1383699 | rs12917505 | 2.19E-05 | 0.71428571 | 0.62650602 |
| 191 | 238 | rs1383699 | rs16977818 | 4.03E-06 | 0.74285714 | 0.62650602 |
| 191 | 240 | rs1383699 | rs10851628 | 2.19E-05 | 0.71428571 | 0.62650602 |
| 191 | 241 | rs1383699 | rs930473 | 0.00010088 | 0.68571429 | 0.62650602 |
| 191 | 242 | rs1383699 | rs1441824 | 0.00112948 | 0.65714286 | 0.60240964 |
| 191 | 243 | rs1383699 | rs6493965 | 1.14E-05 | 0.71428571 | 0.63855422 |
| 191 | 244 | rs1383699 | rs2044070 | 0.0006793 | 0.65714286 | 0.61445783 |
| 191 | 245 | rs1383699 | rs920640 | 1.14E-05 | 0.71428571 | 0.63855422 |
| 191 | 246 | rs1383699 | rs920638 | 3.27E-06 | 0.75714286 | 0.61445783 |
| 191 | 259 | rs1383699 | rs487011 | 4.11E-05 | 0.71428571 | 0.61445783 |
| 192 | 252 | rs72693005 | rs4610906 | 0.00202984 | 0.64285714 | 0.60240964 |
| 192 | 259 | rs72693005 | rs487011 | 1.53E-05 | 0.74285714 | 0.60240964 |
| 193 | 218 | rs1170303 | rs1944887 | 0.00112948 | 0.65714286 | 0.60240964 |
| 193 | 259 | rs1170303 | rs487011 | 0.00137357 | 0.62857143 | 0.62650602 |
| 198 | 226 | rs66624622 | rs532996 | 0.00039921 | 0.65714286 | 0.62650602 |
| 198 | 227 | rs66624622 | rs9571939 | 0.00039921 | 0.65714286 | 0.62650602 |
| 198 | 228 | rs66624622 | rs2173530 | 0.00137357 | 0.62857143 | 0.62650602 |
| 199 | 259 | rs72784444 | rs487011 | 0.00149683 | 0.61428571 | 0.63855422 |
| 201 | 237 | rs62377761 | rs12917505 | 0.00060709 | 0.67142857 | 0.60240964 |
| 201 | 238 | rs62377761 | rs16977818 | 0.00137357 | 0.62857143 | 0.62650602 |
| 201 | 244 | rs62377761 | rs2044070 | 0.00052981 | 0.61428571 | 0.6626506 |
| 202 | 206 | rs4836256 | rs730976 | 0.00593832 | 0.61428571 | 0.60240964 |
| 202 | 218 | rs4836256 | rs1944887 | 0.00016904 | 0.61428571 | 0.68674699 |
| 202 | 225 | rs4836256 | rs67959715 | 0.00044281 | 0.64285714 | 0.63855422 |
| 202 | 236 | rs4836256 | rs894342 | 7.00E-05 | 0.65714286 | 0.6626506 |
| 202 | 237 | rs4836256 | rs12917505 | 1.82E-06 | 0.68571429 | 0.69879518 |
| 202 | 238 | rs4836256 | rs16977818 | 2.12E-05 | 0.64285714 | 0.69879518 |
| 202 | 239 | rs4836256 | rs11071351 | 0.00044281 | 0.64285714 | 0.63855422 |
| 202 | 240 | rs4836256 | rs10851628 | 4.11E-05 | 0.64285714 | 0.68674699 |
| 202 | 241 | rs4836256 | rs930473 | 4.27E-06 | 0.67142857 | 0.69879518 |
| 202 | 242 | rs4836256 | rs1441824 | 0.00012826 | 0.65714286 | 0.65060241 |
| 202 | 243 | rs4836256 | rs6493965 | 4.27E-06 | 0.67142857 | 0.69879518 |
| 202 | 244 | rs4836256 | rs2044070 | 1.73E-05 | 0.67142857 | 0.6746988 |
| 202 | 245 | rs4836256 | rs920640 | 8.72E-06 | 0.67142857 | 0.68674699 |
| 202 | 246 | rs4836256 | rs920638 | 8.72E-06 | 0.67142857 | 0.68674699 |
| 206 | 218 | rs730976 | rs1944887 | 0.00242522 | 0.61428571 | 0.62650602 |
| 211 | 235 | rs3735833 | rs28811003 | 8.47E-05 | 0.62857143 | 0.68674699 |
| 213 | 233 | rs2935752 | rs929610 | 0.00149683 | 0.61428571 | 0.63855422 |
| 213 | 236 | rs2935752 | rs894342 | 0.00011443 | 0.67142857 | 0.63855422 |
| 213 | 237 | rs2935752 | rs12917505 | 9.69E-06 | 0.65714286 | 0.69879518 |
| 213 | 238 | rs2935752 | rs16977818 | 4.73E-06 | 0.65714286 | 0.71084337 |
| 213 | 239 | rs2935752 | rs11071351 | 0.00014215 | 0.64285714 | 0.6626506 |
| 213 | 240 | rs2935752 | rs10851628 | 4.73E-06 | 0.65714286 | 0.71084337 |
| 213 | 241 | rs2935752 | rs930473 | 1.06E-05 | 0.64285714 | 0.71084337 |
| 213 | 242 | rs2935752 | rs1441824 | 6.25E-05 | 0.67142857 | 0.65060241 |
| 213 | 243 | rs2935752 | rs6493965 | 1.06E-05 | 0.64285714 | 0.71084337 |
| 213 | 244 | rs2935752 | rs2044070 | 9.69E-06 | 0.65714286 | 0.69879518 |
| 213 | 245 | rs2935752 | rs920640 | 9.69E-06 | 0.65714286 | 0.69879518 |
| 213 | 246 | rs2935752 | rs920638 | 4.48E-05 | 0.62857143 | 0.69879518 |
| 214 | 236 | rs2935751 | rs894342 | 0.00044281 | 0.64285714 | 0.63855422 |
| 214 | 237 | rs2935751 | rs12917505 | 9.69E-06 | 0.65714286 | 0.69879518 |
| 214 | 238 | rs2935751 | rs16977818 | 4.73E-06 | 0.65714286 | 0.71084337 |
| 214 | 239 | rs2935751 | rs11071351 | 0.00014215 | 0.64285714 | 0.6626506 |
| 214 | 240 | rs2935751 | rs10851628 | 2.30E-05 | 0.62857143 | 0.71084337 |
| 214 | 241 | rs2935751 | rs930473 | 1.06E-05 | 0.64285714 | 0.71084337 |
| 214 | 242 | rs2935751 | rs1441824 | 6.25E-05 | 0.67142857 | 0.65060241 |
| 214 | 243 | rs2935751 | rs6493965 | 4.82E-05 | 0.61428571 | 0.71084337 |
| 214 | 244 | rs2935751 | rs2044070 | 4.48E-05 | 0.62857143 | 0.69879518 |
| 214 | 245 | rs2935751 | rs920640 | 9.69E-06 | 0.65714286 | 0.69879518 |
| 214 | 246 | rs2935751 | rs920638 | 9.69E-06 | 0.65714286 | 0.69879518 |
| 215 | 218 | rs4570614 | rs1944887 | 0.00011443 | 0.67142857 | 0.63855422 |
| 215 | 237 | rs4570614 | rs12917505 | 0.00137357 | 0.62857143 | 0.62650602 |
| 215 | 240 | rs4570614 | rs10851628 | 0.00593832 | 0.61428571 | 0.60240964 |
| 215 | 246 | rs4570614 | rs920638 | 0.00149683 | 0.61428571 | 0.63855422 |
| 216 | 237 | rs4758040 | rs12917505 | 0.00202984 | 0.64285714 | 0.60240964 |
| 216 | 240 | rs4758040 | rs10851628 | 0.00112948 | 0.65714286 | 0.60240964 |
| 216 | 244 | rs4758040 | rs2044070 | 0.00352822 | 0.62857143 | 0.60240964 |
| 216 | 245 | rs4758040 | rs920640 | 0.00090174 | 0.61428571 | 0.65060241 |
| 216 | 246 | rs4758040 | rs920638 | 0.00052981 | 0.61428571 | 0.6626506 |
| 218 | 234 | rs1944887 | rs8042817 | 3.33E-05 | 0.67142857 | 0.6626506 |
| 218 | 259 | rs1944887 | rs487011 | 0.00593832 | 0.61428571 | 0.60240964 |
| 223 | 234 | rs876270 | rs8042817 | 0.00022908 | 0.65714286 | 0.63855422 |
| 223 | 235 | rs876270 | rs28811003 | 0.00039921 | 0.65714286 | 0.62650602 |
| 223 | 259 | rs876270 | rs487011 | 0.00075306 | 0.64285714 | 0.62650602 |
| 224 | 234 | rs11834041 | rs8042817 | 0.00011443 | 0.67142857 | 0.63855422 |
| 224 | 235 | rs11834041 | rs28811003 | 0.00020436 | 0.67142857 | 0.62650602 |
| 224 | 248 | rs11834041 | rs7165629 | 0.00039921 | 0.65714286 | 0.62650602 |
| 225 | 246 | rs67959715 | rs920638 | 5.82E-06 | 0.61428571 | 0.74698795 |
| 233 | 236 | rs929610 | rs894342 | 0.0006793 | 0.65714286 | 0.61445783 |
| 233 | 237 | rs929610 | rs12917505 | 7.72E-06 | 0.68571429 | 0.6746988 |
| 233 | 239 | rs929610 | rs11071351 | 0.00039921 | 0.65714286 | 0.62650602 |
| 233 | 240 | rs929610 | rs10851628 | 4.11E-05 | 0.64285714 | 0.68674699 |
| 233 | 243 | rs929610 | rs6493965 | 4.11E-05 | 0.64285714 | 0.68674699 |
| 233 | 244 | rs929610 | rs2044070 | 7.75E-05 | 0.64285714 | 0.6746988 |
| 233 | 245 | rs929610 | rs920640 | 1.73E-05 | 0.67142857 | 0.6746988 |
| 233 | 246 | rs929610 | rs920638 | 1.73E-05 | 0.67142857 | 0.6746988 |
| 234 | 237 | rs8042817 | rs12917505 | 0.00075306 | 0.64285714 | 0.62650602 |
| 234 | 240 | rs8042817 | rs10851628 | 0.00149683 | 0.61428571 | 0.63855422 |
| 237 | 239 | rs12917505 | rs11071351 | 6.46E-06 | 0.75714286 | 0.60240964 |
| 237 | 259 | rs12917505 | rs487011 | 6.73E-06 | 0.7 | 0.6626506 |
| 238 | 239 | rs16977818 | rs11071351 | 3.27E-06 | 0.75714286 | 0.61445783 |
| 238 | 259 | rs16977818 | rs487011 | 5.45E-07 | 0.72857143 | 0.6746988 |
| 239 | 240 | rs11071351 | rs10851628 | 3.27E-06 | 0.75714286 | 0.61445783 |
| 239 | 241 | rs11071351 | rs930473 | 7.95E-06 | 0.74285714 | 0.61445783 |
| 239 | 243 | rs11071351 | rs6493965 | 7.95E-06 | 0.74285714 | 0.61445783 |
| 239 | 244 | rs11071351 | rs2044070 | 3.27E-06 | 0.75714286 | 0.61445783 |
| 239 | 245 | rs11071351 | rs920640 | 3.27E-06 | 0.75714286 | 0.61445783 |
| 239 | 246 | rs11071351 | rs920638 | 3.27E-06 | 0.75714286 | 0.61445783 |
| 240 | 259 | rs10851628 | rs487011 | 5.45E-07 | 0.72857143 | 0.6746988 |
| 241 | 259 | rs930473 | rs487011 | 1.37E-06 | 0.71428571 | 0.6746988 |
| 242 | 259 | rs1441824 | rs487011 | 0.00018028 | 0.68571429 | 0.61445783 |
| 243 | 248 | rs6493965 | rs7165629 | 0.00352822 | 0.62857143 | 0.60240964 |
| 243 | 259 | rs6493965 | rs487011 | 1.73E-05 | 0.67142857 | 0.6746988 |
| 244 | 259 | rs2044070 | rs487011 | 6.73E-06 | 0.7 | 0.6626506 |
| 245 | 259 | rs920640 | rs487011 | 1.16E-06 | 0.72857143 | 0.6626506 |
| 246 | 259 | rs920638 | rs487011 | 1.16E-06 | 0.72857143 | 0.6626506 |

In higher order analyses, using sets of four and eight polymorphism genotypes selected from the group disclosed in Table 2, a complete enumeration becomes unpractical (over a million combinations for the sets of four and over 10¹⁰ for the set of eight polymorphism genotypes). Therefore, randomly sampled sets (1000 combinations each) of such cardinalities k are presented herein.

For k = 4, 72.1% of tested polymorphism genotype combinations yield a sensitivity and specificity of higher than 50% each, 20,5% of polymorphism genotype combinations yield a sensitivity and specificity of higher than 60% each, and 5,8% of polymorphism genotype combinations yield a sensitivity and specificity of higher than 65% each in predicting a clinical response. Two quadriavariate combinations even yield at least 70% in both sensitivity and specificity in predicting a clinical response, see Table 6.

**Table 6 - Quadrivariate sets of polymorphism genotypes**

| P_ID1 | P_ID2 | P_ID3 | P_ID4 | p-value | sensitivity | specificity |
|---|---|---|---|---|---|---|
| 233 | 123 | 121 | 127 | 8.72E-06 | 0.67142857 | 0.68674699 |
| 236 | 186 | 223 | 215 | 1.82E-06 | 0.68571429 | 0.69879518 |
| 202 | 215 | 184 | 233 | 9.40E-07 | 0.67142857 | 0.72289157 |
| 207 | 171 | 185 | 121 | 1.94E-07 | 0.65714286 | 0.75903614 |
| 240 | 207 | 141 | 157 | 8.01E-08 | 0.65714286 | 0.77108434 |
| 158 | 214 | 133 | 246 | 1.53E-05 | 0.68571429 | 0.6626506 |
| 241 | 219 | 188 | 127 | 8.72E-06 | 0.67142857 | 0.68674699 |
| 233 | 157 | 185 | 158 | 4.58E-08 | 0.78571429 | 0.65060241 |
| 188 | 225 | 223 | 237 | 7.45E-07 | 0.7 | 0.69879518 |
| 225 | 247 | 202 | 179 | 3.72E-05 | 0.65714286 | 0.6746988 |
| 157 | 213 | 219 | 218 | 7.00E-05 | 0.65714286 | 0.6626506 |
| 188 | 242 | 112 | 192 | 6.25E-05 | 0.67142857 | 0.65060241 |
| 237 | 226 | 158 | 216 | 6.25E-05 | 0.67142857 | 0.65060241 |
| 205 | 226 | 156 | 181 | 2.04E-06 | 0.67142857 | 0.71084337 |
| 191 | 239 | 226 | 234 | 0.00012826 | 0.65714286 | 0.65060241 |
| 116 | 243 | 246 | 158 | 2.24E-06 | 0.65714286 | 0.72289157 |
| 193 | 233 | 240 | 198 | 9.69E-06 | 0.65714286 | 0.69879518 |
| 202 | 141 | 204 | 160 | 7.00E-05 | 0.65714286 | 0.6626506 |
| 184 | 233 | 192 | 215 | 3.72E-05 | 0.65714286 | 0.6746988 |
| 191 | 188 | 159 | 243 | 2.94E-05 | 0.68571429 | 0.65060241 |
| 246 | 227 | 238 | 224 | 1.94E-07 | 0.65714286 | 0.75903614 |
| 202 | 241 | 224 | 183 | 3.90E-09 | 0.7 | 0.77108434 |
| 227 | 191 | 112 | 246 | 7.00E-05 | 0.65714286 | 0.6626506 |
| 252 | 161 | 192 | 240 | 4.55E-07 | 0.65714286 | 0.74698795 |
| 161 | 207 | 202 | 160 | 1.68E-07 | 0.75714286 | 0.6626506 |
| 212 | 243 | 190 | 116 | 4.95E-10 | 0.65714286 | 0.8313253 |
| 246 | 184 | 11 | 243 | 3.33E-05 | 0.67142857 | 0.6626506 |
| 184 | 241 | 259 | 187 | 7.00E-05 | 0.65714286 | 0.6626506 |
| 226 | 243 | 190 | 224 | 4.73E-06 | 0.65714286 | 0.71084337 |
| 237 | 157 | 240 | 160 | 1.53E-05 | 0.68571429 | 0.6626506 |
| 223 | 245 | 132 | 184 | 1.03E-06 | 0.65714286 | 0.73493976 |
| 188 | 207 | 182 | 228 | 1.03E-06 | 0.65714286 | 0.73493976 |
| 224 | 205 | 227 | 186 | 7.00E-05 | 0.65714286 | 0.6626506 |
| 223 | 176 | 245 | 206 | 4.73E-06 | 0.65714286 | 0.71084337 |
| 190 | 204 | 234 | 238 | 6.29E-08 | 0.7 | 0.73493976 |
| 201 | 192 | 240 | 187 | 1.73E-05 | 0.67142857 | 0.6746988 |
| 227 | 185 | 190 | 215 | 7.45E-07 | 0.7 | 0.69879518 |
| 185 | 241 | 202 | 186 | 1.93E-05 | 0.65714286 | 0.68674699 |
| 214 | 11 | 157 | 220 | 9.61E-07 | 0.74285714 | 0.65060241 |
| 242 | 190 | 192 | 245 | 2.86E-06 | 0.71428571 | 0.6626506 |
| 121 | 246 | 238 | 190 | 9.69E-06 | 0.65714286 | 0.69879518 |
| 223 | 157 | 241 | 190 | 1.82E-06 | 0.68571429 | 0.69879518 |
| 233 | 116 | 132 | 243 | 3.72E-05 | 0.65714286 | 0.6746988 |
| 218 | 158 | 250 | 244 | 9.40E-07 | 0.67142857 | 0.72289157 |
| 250 | 158 | 141 | 213 | 3.33E-05 | 0.67142857 | 0.6626506 |
| 240 | 215 | 213 | 158 | 9.69E-06 | 0.65714286 | 0.69879518 |
| 235 | 243 | 214 | 208 | 4.73E-06 | 0.65714286 | 0.71084337 |
| 202 | 244 | 234 | 127 | 1.33E-05 | 0.7 | 0.65060241 |
| 175 | 184 | 127 | 219 | 4.27E-06 | 0.67142857 | 0.69879518 |
| 190 | 240 | 212 | 223 | 1.81E-07 | 0.67142857 | 0.74698795 |
| 248 | 219 | 233 | 185 | 6.44E-07 | 0.71428571 | 0.68674699 |
| 184 | 234 | 205 | 244 | 9.69E-06 | 0.65714286 | 0.69879518 |
| 201 | 246 | 192 | 233 | 4.27E-06 | 0.67142857 | 0.69879518 |
| 251 | 245 | 191 | 176 | 1.82E-06 | 0.68571429 | 0.69879518 |
| 233 | 223 | 235 | 225 | 3.72E-05 | 0.65714286 | 0.6746988 |
| 237 | 220 | 236 | 192 | 9.69E-06 | 0.65714286 | 0.69879518 |
| 241 | 236 | 248 | 218 | 4.73E-06 | 0.65714286 | 0.71084337 |
| 252 | 218 | 219 | 239 | 6.98E-08 | 0.68571429 | 0.74698795 |

For k = 8, 93.3% of tested polymorphism genotype combinations yield a sensitivity and specificity of higher than 50% each, 32.6% of polymorphism genotype yield a sensitivity and specificity of higher than 60% each, 8.7% of polymorphism genotype combinations yield a sensitivity and specificity of 65% each, and, finally, 0.5% (5 combinations) of octovariate polymorphism genotype combinations yield a sensitivity and specificity at least 70% in sensitivity and specificity in predicting a clinical response, see Table 7.

**Table 7 - Octovariate sets of polymorphism genotypes**

| P_ID1 | P_ID2 | P_ID3 | P_ID4 | P_ID5 | P_ID6 | P_ID7 | P_ID8 | p-value | sensitivity | specificity |
|---|---|---|---|---|---|---|---|---|---|---|
| 201 | 198 | 191 | 248 | 176 | 213 | 220 | 239 | 3.85E-06 | 0.65714286 | 0.72289157 |
| 206 | 112 | 186 | 247 | 205 | 171 | 184 | 246 | 2.41E-05 | 0.65714286 | 0.68674699 |
| 188 | 243 | 227 | 191 | 240 | 202 | 242 | 176 | 3.85E-06 | 0.65714286 | 0.72289157 |
| 160 | 193 | 132 | 235 | 121 | 192 | 188 | 236 | 7.10E-07 | 0.67142857 | 0.73493976 |
| 246 | 112 | 237 | 220 | 190 | 185 | 116 | 186 | 6.78E-07 | 0.65714286 | 0.74698795 |
| 116 | 189 | 241 | 246 | 213 | 225 | 191 | 132 | 1.57E-08 | 0.68571429 | 0.77108434 |
| 132 | 202 | 236 | 245 | 184 | 193 | 192 | 198 | 4.11E-08 | 0.67142857 | 0.77108434 |
| 244 | 188 | 225 | 206 | 192 | 214 | 234 | 213 | 2.99E-07 | 0.68571429 | 0.73493976 |
| 235 | 214 | 211 | 156 | 245 | 190 | 188 | 237 | 1.66E-08 | 0.7 | 0.75903614 |
| 185 | 238 | 244 | 206 | 237 | 184 | 183 | 259 | 1.64E-06 | 0.65714286 | 0.73493976 |
| 185 | 168 | 191 | 193 | 184 | 160 | 238 | 141 | 1.93E-05 | 0.65714286 | 0.69879518 |
| 159 | 244 | 202 | 133 | 259 | 243 | 223 | 121 | 4.03E-06 | 0.67142857 | 0.71084337 |
| 211 | 238 | 235 | 158 | 228 | 218 | 214 | 189 | 4.11E-08 | 0.67142857 | 0.77108434 |
| 190 | 238 | 185 | 259 | 213 | 179 | 184 | 188 | 2.71E-07 | 0.65714286 | 0.75903614 |
| 11 | 157 | 223 | 188 | 236 | 185 | 244 | 201 | 2.41E-05 | 0.65714286 | 0.68674699 |
| 188 | 246 | 171 | 242 | 127 | 184 | 234 | 132 | 6.78E-07 | 0.65714286 | 0.74698795 |
| 240 | 158 | 112 | 235 | 259 | 242 | 226 | 205 | 1.06E-05 | 0.68571429 | 0.6746988 |
| 211 | 213 | 205 | 171 | 202 | 185 | 259 | 116 | 1.50E-07 | 0.72857143 | 0.69879518 |
| 187 | 121 | 250 | 116 | 233 | 243 | 198 | 220 | 7.46E-07 | 0.68571429 | 0.72289157 |
| 216 | 168 | 185 | 132 | 183 | 112 | 213 | 238 | 1.49E-08 | 0.67142857 | 0.78313253 |
| 157 | 248 | 236 | 259 | 171 | 238 | 239 | 192 | 4.86E-05 | 0.65714286 | 0.6746988 |
| 234 | 227 | 224 | 251 | 277 | 198 | 187 | 245 | 1.05E-07 | 0.65714286 | 0.77108434 |
| 237 | 223 | 11 | 215 | 116 | 218 | 182 | 233 | 1.49E-08 | 0.67142857 | 0.78313253 |
| 201 | 220 | 127 | 234 | 157 | 219 | 186 | 141 | 6.78E-07 | 0.65714286 | 0.74698795 |
| 218 | 247 | 193 | 241 | 192 | 236 | 224 | 186 | 2.84E-07 | 0.67142857 | 0.74698795 |
| 233 | 201 | 158 | 226 | 235 | 132 | 223 | 190 | 3.85E-06 | 0.65714286 | 0.72289157 |
| 225 | 186 | 156 | 241 | 204 | 214 | 218 | 212 | 2.71E-07 | 0.65714286 | 0.75903614 |
| 116 | 179 | 112 | 184 | 190 | 259 | 239 | 215 | 1.64E-06 | 0.65714286 | 0.73493976 |
| 121 | 252 | 186 | 189 | 241 | 133 | 141 | 223 | 1.41E-08 | 0.65714286 | 0.79518072 |
| 250 | 248 | 241 | 184 | 159 | 206 | 187 | 192 | 7.10E-07 | 0.67142857 | 0.73493976 |
| 168 | 277 | 250 | 238 | 245 | 218 | 227 | 184 | 1.57E-08 | 0.68571429 | 0.77108434 |
| 212 | 181 | 184 | 159 | 237 | 223 | 179 | 213 | 2.84E-07 | 0.67142857 | 0.74698795 |
| 241 | 219 | 175 | 187 | 156 | 233 | 157 | 184 | 2.99E-07 | 0.68571429 | 0.73493976 |
| 224 | 192 | 206 | 121 | 202 | 214 | 241 | 239 | 5.48E-09 | 0.68571429 | 0.78313253 |
| 241 | 192 | 214 | 141 | 179 | 227 | 212 | 121 | 8.76E-06 | 0.65714286 | 0.71084337 |
| 212 | 241 | 239 | 121 | 191 | 187 | 224 | 238 | 5.48E-09 | 0.68571429 | 0.78313253 |
| 245 | 225 | 236 | 132 | 160 | 211 | 244 | 238 | 2.85E-09 | 0.65714286 | 0.81927711 |
| 121 | 237 | 234 | 205 | 132 | 244 | 190 | 238 | 1.22E-07 | 0.7 | 0.73493976 |
| 121 | 220 | 241 | 245 | 219 | 214 | 248 | 132 | 8.76E-06 | 0.65714286 | 0.71084337 |
| 240 | 220 | 252 | 250 | 157 | 214 | 218 | 245 | 5.48E-09 | 0.68571429 | 0.78313253 |
| 193 | 211 | 179 | 132 | 185 | 246 | 238 | 240 | 2.85E-09 | 0.65714286 | 0.81927711 |
| 243 | 241 | 252 | 237 | 192 | 141 | 259 | 190 | 7.10E-07 | 0.67142857 | 0.73493976 |
| 227 | 190 | 213 | 250 | 191 | 218 | 214 | 248 | 4.91E-09 | 0.65714286 | 0.80722892 |
| 242 | 214 | 239 | 179 | 201 | 190 | 181 | 192 | 1.78E-11 | 0.7 | 0.8313253 |
| 224 | 121 | 259 | 246 | 207 | 228 | 204 | 219 | 3.85E-06 | 0.65714286 | 0.72289157 |
| 236 | 186 | 116 | 187 | 184 | 204 | 219 | 121 | 2.84E-07 | 0.67142857 | 0.74698795 |
| 179 | 11 | 239 | 184 | 159 | 202 | 123 | 185 | 4.33E-08 | 0.68571429 | 0.75903614 |
| 248 | 127 | 240 | 141 | 133 | 233 | 156 | 201 | 1.05E-07 | 0.65714286 | 0.77108434 |
| 185 | 237 | 188 | 191 | 247 | 189 | 216 | 158 | 2.84E-07 | 0.67142857 | 0.74698795 |
| 219 | 132 | 176 | 191 | 277 | 214 | 236 | 175 | 1.49E-08 | 0.67142857 | 0.78313253 |
| 133 | 241 | 214 | 220 | 189 | 191 | 233 | 211 | 6.78E-07 | 0.65714286 | 0.74698795 |
| 202 | 182 | 233 | 259 | 218 | 127 | 243 | 159 | 2.71E-07 | 0.65714286 | 0.75903614 |
| 189 | 238 | 216 | 223 | 214 | 158 | 190 | 179 | 2.85E-09 | 0.65714286 | 0.81927711 |
| 123 | 112 | 243 | 141 | 202 | 121 | 190 | 116 | 1.76E-08 | 0.71428571 | 0.74698795 |
| 237 | 193 | 116 | 185 | 228 | 202 | 186 | 132 | 2.71E-07 | 0.65714286 | 0.75903614 |
| 190 | 11 | 237 | 182 | 202 | 132 | 214 | 246 | 1.10E-07 | 0.67142857 | 0.75903614 |
| 214 | 237 | 224 | 218 | 250 | 181 | 155 | 160 | 3.92E-08 | 0.65714286 | 0.78313253 |
| 237 | 252 | 234 | 133 | 185 | 250 | 239 | 188 | 5.48E-09 | 0.68571429 | 0.78313253 |
| 188 | 228 | 245 | 185 | 248 | 234 | 161 | 224 | 4.03E-06 | 0.67142857 | 0.71084337 |
| 204 | 228 | 188 | 202 | 212 | 223 | 168 | 141 | 2.71E-07 | 0.65714286 | 0.75903614 |
| 206 | 238 | 186 | 245 | 191 | 220 | 155 | 192 | 6.78E-07 | 0.65714286 | 0.74698795 |
| 237 | 246 | 168 | 188 | 141 | 198 | 192 | 190 | 3.85E-06 | 0.65714286 | 0.72289157 |
| 223 | 252 | 190 | 160 | 205 | 212 | 184 | 233 | 4.03E-06 | 0.67142857 | 0.71084337 |
| 141 | 187 | 121 | 188 | 246 | 193 | 185 | 133 | 1.16E-07 | 0.68571429 | 0.74698795 |
| 218 | 238 | 228 | 234 | 184 | 213 | 132 | 248 | 1.10E-07 | 0.67142857 | 0.75903614 |
| 11 | 213 | 238 | 219 | 246 | 112 | 187 | 248 | 2.30E-05 | 0.67142857 | 0.6746988 |
| 121 | 190 | 160 | 213 | 184 | 239 | 246 | 189 | 1.10E-07 | 0.67142857 | 0.75903614 |
| 168 | 225 | 176 | 251 | 236 | 189 | 190 | 218 | 4.11E-08 | 0.67142857 | 0.77108434 |
| 235 | 116 | 187 | 250 | 168 | 220 | 238 | 190 | 6.78E-07 | 0.65714286 | 0.74698795 |
| 216 | 214 | 246 | 116 | 244 | 182 | 240 | 186 | 7.10E-07 | 0.67142857 | 0.73493976 |
| 208 | 188 | 187 | 218 | 245 | 238 | 199 | 157 | 1.64E-06 | 0.65714286 | 0.73493976 |
| 239 | 112 | 176 | 185 | 246 | 250 | 219 | 202 | 4.86E-05 | 0.65714286 | 0.6746988 |
| 250 | 220 | 233 | 127 | 224 | 116 | 226 | 237 | 1.72E-06 | 0.67142857 | 0.72289157 |
| 156 | 212 | 204 | 259 | 214 | 237 | 240 | 191 | 1.05E-07 | 0.65714286 | 0.77108434 |
| 259 | 204 | 213 | 228 | 180 | 218 | 242 | 193 | 1.72E-06 | 0.67142857 | 0.72289157 |
| 218 | 250 | 227 | 211 | 171 | 185 | 251 | 133 | 1.05E-07 | 0.65714286 | 0.77108434 |
| 176 | 202 | 185 | 187 | 277 | 248 | 233 | 189 | 1.72E-06 | 0.67142857 | 0.72289157 |
| 112 | 277 | 218 | 155 | 156 | 237 | 235 | 244 | 5.67E-10 | 0.67142857 | 0.81927711 |
| 187 | 252 | 240 | 116 | 175 | 184 | 239 | 242 | 2.84E-07 | 0.67142857 | 0.74698795 |
| 182 | 227 | 206 | 181 | 132 | 224 | 244 | 188 | 1.10E-07 | 0.67142857 | 0.75903614 |
| 239 | 238 | 214 | 223 | 242 | 218 | 186 | 192 | 1.66E-08 | 0.7 | 0.75903614 |
| 185 | 188 | 277 | 241 | 219 | 193 | 201 | 176 | 1.64E-06 | 0.65714286 | 0.73493976 |
| 116 | 233 | 199 | 247 | 183 | 238 | 214 | 180 | 4.11E-08 | 0.67142857 | 0.77108434 |
| 180 | 242 | 116 | 239 | 158 | 238 | 243 | 240 | 7.46E-07 | 0.68571429 | 0.72289157 |
| 234 | 237 | 193 | 235 | 224 | 179 | 190 | 233 | 3.92E-08 | 0.65714286 | 0.78313253 |

For k = 32, 99.9% of tested polymorphism genotype combinations yield a sensitivity and specificity of higher than 50% each in specificity and sensitivity, 98.9% of tested polymorphism genotype combinations yield a sensitivity and specificity of higher than 60% each, 72.8% of tested polymorphism genotype combinations yield a sensitivity and specificity of higher than 65% each, 15.6% of tested polymorphism genotype combinations yield a sensitivity and specificity of higher than 70% each in predicting a clinical response. Finally, some of the tested polymorphism genotype combinations (0.3%) even yield a sensitivity and specificity of higher than 75% each (data not shown).

As will be understood from the above explanations and data in Table 5, Table 6, and Table 7, even minimal subsets of polymorphism genotypes selected from the particularly useful set of polymorphism genotypes disclosed in Table 2 already allow for predictions of a clinical response significantly better than 50% ("coin-flip"). Therefore, while the present invention ideally aims at predicting the treatment response to SSR-125543 with sensitivity and specificity of at least 75% each, at least 80% each, at least 85% each, or even at least 90% each, methods of prediction using smaller subsets, e.g., of only one, two, four, or eight polymorphism genotypes selected from the group consisting of the polymorphism genotypes disclosed in Table 2 already provide a significant performance in predicting clinical responses. A subset of k = 32 polymorphism genotypes already comprises combinations yielding a sensitivity and specificity of at least 75% each in predicting a clinical response. The predictive performance can be further increased by including, e.g., 150 polymorphism genotypes, as has been done in Example 1, 200 polymorphism genotypes, 250 polymorphism genotypes or all polymorphism genotypes as disclosed in Table 2.

### Equivalents

The foregoing exemplary embodiments are to be considered illustrative of, and not limiting to, the invention disclosed herein. It will be apparent to those skilled in the art that various modifications may be made without departing from the scope of the invention. Therefore, it will be appreciated that the scope of the present invention is primarily defined by the appended claims, and is not limited by the specific embodiments which have been presented as examples. All changes which come within the meaning and range of equivalency of the claims are intended to be encompassed.

## Claims

1. The compound SSR-125543 or a pharmaceutically acceptable salt thereof for use in a method of treating a condition characterized, caused or accompanied by CRH overproduction or over-activity, comprising administering an effective amount of SSR-125543 or a pharmaceutically acceptable salt thereof to a subject in need of such a treatment, wherein the subject has been predicted to respond, or has an increased likelihood of responding, to a treatment with SSR-125543 or a pharmaceutically acceptable salt thereof, wherein the treatment response has been predicted by detecting the presence or absence of one or more polymorphism genotypes in a biological sample from the subject, wherein the one or more polymorphism genotypes comprise:
at least one polymorphism genotype selected from the group consisting of rs34169260 (A/G), rs796287 (A/C), rs56149945 (A/G), rs6190 (T/C), rs7179092 (T/C), rs7614867 (A/G), rs920640 (T/C), rs7167722 (T/C), rs920638 (T/C), rs7165629 (T/C), rs80049044 (T/A), rs16941058 (A/G), rs112015971 (A/G), rs10894873 (T/C), rs117455294 (T/G), rs1170303 (T/C), rs16940681 (C/G), rs968519 (T/C), rs28381866 (T/C), rs79320848 (T/G), rs114653646 (T/G), rs2589496 (T/C), rs10482650 (A/G), rs17614642 (A/G), rs73200317 (T/C), rs1380146 (T/A), rs735164 (T/C), rs730976 (T/G), rs55934524 (T/G), rs4570614 (A/G), rs4458044 (C/G), rs77850169 (A/G), rs35339359 (A/G), rs34800935 (T/C), rs72945439 (T/C), rs113959523 (A/G), rs116798177 (A/G), rs11247577 (T/G), rs75869266 (T/C), rs74372553 (T/C), rs11691508 (A/G), rs6493965 (A/G), rs4869476 (T/C), rs3730170 (T/C), rs2145288 (A/C), rs2935752 (A/C), rs146512400 (A/G), rs62057097 (T/C), rs115061314 (T/C), rs34113594 (T/G), rs61751173 (A/G), rs74338736 (A/C), rs10851726 (T/C), rs4610906 (T/C), rs59485211 (T/C), rs7060015 (T/G), rs75710780 (T/G), rs6520908 (T/C), rs487011 (T/G), rs1383699 (A/C), rs67516871 (A/G), rs114106519 (T/C), rs7220091 (A/G), rs12489026 (A/G), rs876270 (T/C), rs4968161 (T/C), rs62056907 (A/G), rs2235013 (T/C), rs16878812 (A/G), rs6549407 (A/G), rs28381848 (A/G), rs79723704 (A/C), rs72814052 (A/G), rs10152908 (T/C), rs172769 (A/C), rs78596668 (T/C), rs73307922 (T/C), rs3842 (A/G), rs7210584 (A/C), rs62402121 (T/C), rs55709291 (A/G), rs72747088 (A/G), rs929610 (G/C), rs6766242 (T/C), rs1468552 (G/C), rs78838114 (T/C), rs62489862 (T/C), rs894342 (A/G), rs58882373 (T/C), rs3811939 (A/G), rs6984688 (T/G), rs1018160 (T/C), rs76602912 (A/G), rs80067508 (A/G), rs74888440 (T/C), rs12481583 (T/C), rs66794218 (A/G), rs16946701 (A/G), rs75726724 (A/G), rs67959715 (T/A), rs11871392 (T/G), rs2044070 (A/G), rs77612799 (T/C), rs6743702 (T/C), rs616870 (T/C), rs79590198 (A/G), rs75715199 (A/G), rs13087555 (T/C), rs4869618 (T/C), rs117397046 (A/G), rs8042817 (A/G), rs2258097 (T/C), rs2260882 (C/G), rs532996 (A/G), rs11747040 (T/C), rs10034039 (T/G), rs116909369 (A/G), rs79134986 (A/G), rs117615688 (T/C), rs8032253 (T/C), rs12818653 (T/A), rs4587884 (A/C), rs77122853 (T/C), rs117615061 (T/C), rs74682905 (A/G), rs2257468 (T/C), rs2032582 (T/G), rs2235015 (T/G), rs2729794 (T/C), rs77549514 (A/G), rs74790420 (A/C), rs73129579 (T/C), rs12913346 (A/C), rs117560908 (T/C), rs72747091 (A/G), rs2935751 (A/G), rs4331446 (A/G), rs7523266 (T/C), rs7648662 (T/C), rs117034065 (A/G), rs4836256 (T/C), rs80238698 (T/C), rs3730173 (T/C), rs11687884 (T/C), rs72693005 (T/C), rs2589476 (T/C), rs9813396 (T/C), rs10482667 (A/G), rs72784444 (A/G), rs75074511 (T/C), rs7951003 (A/G), rs79584784 (A/G), rs2214102 (T/C), rs28811003 (A/G), rs6100261 (A/T), rs77152456 (A/G), rs66624622 (T/G), rs140302965 (A/G), rs11653269 (T/C), rs74405057 (A/G), rs7121 (A/G), rs16977818 (A/C), rs12490095 (T/C), rs118003903 (A/G), rs62377761 (T/C), P1_M_061510_6_34_M (-/CACTTACCTTCTTTGTGCCACAGTTTCCCTATCTAAAACACAAGGTTATCAGTTATCAACATCTCTTGGGATTGTGAGGACTAAAGTAATGCACATAAAG), rs375115639 (-/AAATTACCCTGTTAGGTTTCAATGAAACACCTTTTCTCTTGTAACAAACATCTCC TCCAAGCTAGAATTTCAAAACAG), rs1002204 (A/C), rs10062367 (A/G), rs10482642 (A/G), rs10482658 (A/G), rs1053989 (A/C), rs10851628 (T/C), rs10947562 (T/C), rs11069612 (A/G), rs11071351 (T/C), rs11091175 (A/G), rs11638450 (T/C), rs11715827 (T/G), rs11745958 (T/C), rs11834041 (A/G), rs1202180 (T/C), rs12054781 (A/G), rs12539395 (A/G), rs12720066 (T/G), rs1279754 (A/C), rs12872047 (T/C), rs12876742 (A/C), rs12917505 (A/G), rs13066950 (T/G), rs13229143 (C/G), rs1383707 (T/C), rs1441824 (T/C), rs1652311 (A/G), rs17064 (T/A), rs17100236 (A/G), rs17149699 (A/G), rs1724386 (A/G), rs17250255 (A/G), rs17327624 (T/G), rs17616338 (A/G), rs17687796 (A/G), rs17740874 (T/C), rs17763104 (T/C), rs1880748 (T/C), rs1882478 (A/G), rs1944887 (T/C), rs2028629 (A/G), rs2143404 (A/G), rs2173530 (T/C), rs220806 (T/C), rs2235047 (A/C), rs2242071 (A/G), rs2257474 (T/C), rs2295583 (A/T), rs234629 (T/C), rs234630 (A/G), rs2436401 (A/G), rs258750 (T/C), rs2589487 (T/C), rs28364018 (T/G), rs28381774 (T/C), rs28381784 (A/G), rs2963155 (A/G), rs3133622 (T/G), rs32897 (T/C), rs33388 (A/T), rs3730168 (T/C), rs3735833 (T/G), rs3777747 (A/G), rs3786066 (T/C), rs3798346 (T/C), rs3822736 (A/G), rs389035 (T/C), rs3924144 (A/G), rs4148737 (T/C), rs4148749 (G/C), rs417968 (T/C), rs4458144 (T/C), rs4515335 (T/C), rs4728699 (A/G), rs4758040 (A/G), rs4812040 (A/G), rs4912650 (T/G), rs4957891 (T/C), rs5906392 (A/G), rs6026561 (T/C), rs6026565 (T/A), rs6026567 (A/G), rs6026593 (A/G), rs6092704 (T/G), rs6100260 (A/G), rs6128461 (T/C), rs6415328 (T/C), rs6610868 (T/C), rs6686061 (A/C), rs6730350 (T/G), rs6746197 (T/C), rs6963426 (T/C), rs7121326 (T/C), rs7721799 (A/G), rs7787082 (T/C), rs7799592 (A/C), rs796245 (T/C), rs809482 (A/C), rs8125112 (T/C), rs919196 (A/G), rs920750 (T/C), rs9332385 (A/G), rs930473 (T/G), rs9324921 (A/C), rs9348979 (A/G), rs9571939 (A/C), and rs9892359 (T/C).

2. The compound of claim 1 for the use according to claim 1, wherein the predicting step comprises:
(a) determining whether the subject will respond, or has an increased likelihood of responding to the treatment with SSR-125543 or a pharmaceutically acceptable salt thereof; and/or
(b) determining whether the subject will not respond, or has a decreased likelihood of responding to the treatment with SSR-125543 or a pharmaceutically acceptable salt thereof.

3. The compound of claim 1 for the use according to claim 2, wherein the determining step comprises one or more statistical analysis method selected from the group consisting of artificial neural network learning, decision tree learning, decision tree forest learning, linear discriminant analysis, non-linear discriminant analysis, genetic expression programming, relevance vector machines, linear models, generalized linear models, generalized estimating equations, generalized linear mixed models, the elastic net, the lasso support vector machine learning, Bayesian network learning, probabilistic neural network learning, clustering, and regression analysis, optionally wherein the statistical analysis method is computer-implemented.

4. The compound of claim 1 for the use according to any one of claims 1-3, wherein the one or more polymorphism genotypes comprise:
(a) at least two;
(b) at least four;
(c) at least eight;
(d) at least sixteen;
(e) at least thirty-two; or
(f) all
of the polymorphism genotypes as defined in claim 1.

5. The compound of claim 1 for the use according to of any one of claims 1-3, wherein the one or more polymorphism genotypes comprise:
(a) at least two polymorphism genotypes selected from the combinations of polymorphism genotypes disclosed in Table 5;
(b) at least four polymorphism genotypes selected from the combinations of polymorphism genotypes disclosed in Table 6;
(c) at least eight polymorphism genotypes selected from the combinations of polymorphism genotypes disclosed in Table 7;
(d) all of the polymorphism genotypes disclosed in Table 2.

6. The compound of claim 1 for use according to of any one of claims 1 to 5, wherein the treatment response to treatment with SSR-125543 or a pharmaceutically acceptable salt thereof is a clinical response.

7. The compound of claim 1 for the use according to of any one of claims 1 to 6, wherein the subject has depressive symptoms, anxiety symptoms or both depressive symptoms and anxiety symptoms or a sleep disorder; and/or
wherein the treatment is a treatment of depressive symptoms, anxiety symptoms or both depressive symptoms and anxiety symptoms or a sleep disorder.

8. The compound of claim 1 for the use according to claim 7, wherein the treatment response to treatment with SSR-125543 or a pharmaceutically acceptable salt thereof is a clinical response, and wherein the clinical response is a prevention, alteration, alleviation or complete remission of depressive symptoms and/or anxiety symptoms or a sleep disorder.

9. The compound of claim 1 for the use according to claim 8, wherein the clinical response is a prevention, alteration, alleviation or complete remission of depressive symptoms and/or anxiety symptoms as determined using a scale selected from the group consisting of HAM-D, BDI, MADRS, GDS, ZSRDS, HAM-A and STAI.

10. The compound of claim 1 for the use according to any one of claims 1-9, wherein the biological sample is a buccal or a blood sample.

11. The compound of claim 1 for the use according to any one of claims 1-10, wherein detecting comprises the use of one or more polynucleotides capable of specifically hybridizing to at least one nucleic acid comprising the one or more polymorphism genotypes.

12. The compound of claim 1 for the use according to any one of claims 1-11, wherein after the subject has been administered SSR-125543 or a pharmaceutically acceptable salt thereof, further comprising comparing the prediction that the subject will respond to a treatment with SSR-125543 or a pharmaceutically acceptable salt thereof with the treatment response of the subject to administration of SSR-125543 or a pharmaceutically acceptable salt thereof.

13. The compound of claim 1 for the use according to any one of claims 1-12, wherein the prediction that the subject will respond to a treatment with SSR-125543 or a pharmaceutically acceptable salt thereof has a sensitivity of higher than 50% and a specificity of higher than 50%.

14. A method of predicting the likelihood that a given subject will or will not respond to treatment with SSR-125543 or a pharmaceutically acceptable salt thereof, comprising
detecting the presence or absence of one or more polymorphism genotypes in a biological sample from the subject, wherein the one or more polymorphism genotypes comprise:
at least one polymorphism genotype selected from the group consisting of rs34169260 (A/G), rs796287 (A/C), rs56149945 (A/G), rs6190 (T/C), rs7179092 (T/C), rs7614867 (A/G), rs920640 (T/C), rs7167722 (T/C), rs920638 (T/C), rs7165629 (T/C), rs80049044 (T/A), rs16941058 (A/G), rs112015971 (A/G), rs10894873 (T/C), rs117455294 (T/G), rs1170303 (T/C), rs16940681 (C/G), rs968519 (T/C), rs28381866 (T/C), rs79320848 (T/G), rs114653646 (T/G), rs2589496 (T/C), rs10482650 (A/G), rs17614642 (A/G), rs73200317 (T/C), rs1380146 (T/A), rs735164 (T/C), rs730976 (T/G), rs55934524 (T/G), rs4570614 (A/G), rs4458044 (C/G), rs77850169 (A/G), rs35339359 (A/G), rs34800935 (T/C), rs72945439 (T/C), rs113959523 (A/G), rs116798177 (A/G), rs11247577 (T/G), rs75869266 (T/C), rs74372553 (T/C), rs11691508 (A/G), rs6493965 (A/G), rs4869476 (T/C), rs3730170 (T/C), rs2145288 (A/C), rs2935752 (A/C), rs146512400 (A/G), rs62057097 (T/C), rs115061314 (T/C), rs34113594 (T/G), rs61751173 (A/G), rs74338736 (A/C), rs10851726 (T/C), rs4610906 (T/C), rs59485211 (T/C), rs7060015 (T/G), rs75710780 (T/G), rs6520908 (T/C), rs487011 (T/G), rs1383699 (A/C), rs67516871 (A/G), rs114106519 (T/C), rs7220091 (A/G), rs12489026 (A/G), rs876270 (T/C), rs4968161 (T/C), rs62056907 (A/G), rs2235013 (T/C), rs16878812 (A/G), rs6549407 (A/G), rs28381848 (A/G), rs79723704 (A/C), rs72814052 (A/G), rs10152908 (T/C), rs172769 (A/C), rs78596668 (T/C), rs73307922 (T/C), rs3842 (A/G), rs7210584 (A/C), rs62402121 (T/C), rs55709291 (A/G), rs72747088 (A/G), rs929610 (G/C), rs6766242 (T/C), rs1468552 (G/C), rs78838114 (T/C), rs62489862 (T/C), rs894342 (A/G), rs58882373 (T/C), rs3811939 (A/G), rs6984688 (T/G), rs1018160 (T/C), rs76602912 (A/G), rs80067508 (A/G), rs74888440 (T/C), rs12481583 (T/C), rs66794218 (A/G), rs16946701 (A/G), rs75726724 (A/G), rs67959715 (T/A), rs11871392 (T/G), rs2044070 (A/G), rs77612799 (T/C), rs6743702 (T/C), rs616870 (T/C), rs79590198 (A/G), rs75715199 (A/G), rs13087555 (T/C), rs4869618 (T/C), rs117397046 (A/G), rs8042817 (A/G), rs2258097 (T/C), rs2260882 (C/G), rs532996 (A/G), rs11747040 (T/C), rs10034039 (T/G), rs116909369 (A/G), rs79134986 (A/G), rs117615688 (T/C), rs8032253 (T/C), rs12818653 (T/A), rs4587884 (A/C), rs77122853 (T/C), rs117615061 (T/C), rs74682905 (A/G), rs2257468 (T/C), rs2032582 (T/G), rs2235015 (T/G), rs2729794 (T/C), rs77549514 (A/G), rs74790420 (A/C), rs73129579 (T/C), rs12913346 (A/C), rs117560908 (T/C), rs72747091 (A/G), rs2935751 (A/G), rs4331446 (A/G), rs7523266 (T/C), rs7648662 (T/C), rs117034065 (A/G), rs4836256 (T/C), rs80238698 (T/C), rs3730173 (T/C), rs11687884 (T/C), rs72693005 (T/C), rs2589476 (T/C), rs9813396 (T/C), rs10482667 (A/G), rs72784444 (A/G), rs75074511 (T/C), rs7951003 (A/G), rs79584784 (A/G), rs2214102 (T/C), rs28811003 (A/G), rs6100261 (A/T), rs77152456 (A/G), rs66624622 (T/G), rs140302965 (A/G), rs11653269 (T/C), rs74405057 (A/G), rs7121 (A/G), rs16977818 (A/C), rs12490095 (T/C), rs118003903 (A/G), rs62377761 (T/C), P1_M_061510_6_34_M (-/CACTTACCTTCTTTGTGCCACAGTTTCCCTATCTAAAACACAAGGTTATCAGTTATCAACATCTCTTGGGATTGTGAGGACTAAAGTAATGCACATAAAG), rs375115639 (-/AAATTACCCTGTTAGGTTTCAATGAAACACCTTTTCTCTTGTAACAAACATCTCC TCCAAGCTAGAATTTCAAAACAG), rs1002204 (A/C), rs10062367 (A/G), rs10482642 (A/G), rs10482658 (A/G), rs1053989 (A/C), rs10851628 (T/C), rs10947562 (T/C), rs11069612 (A/G), rs11071351 (T/C), rs11091175 (A/G), rs11638450 (T/C), rs11715827 (T/G), rs11745958 (T/C), rs11834041 (A/G), rs1202180 (T/C), rs12054781 (A/G), rs12539395 (A/G), rs12720066 (T/G), rs1279754 (A/C), rs12872047 (T/C), rs12876742 (A/C), rs12917505 (A/G), rs13066950 (T/G), rs13229143 (C/G), rs1383707 (T/C), rs1441824 (T/C), rs1652311 (A/G), rs17064 (T/A), rs17100236 (A/G), rs17149699 (A/G), rs1724386 (A/G), rs17250255 (A/G), rs17327624 (T/G), rs17616338 (A/G), rs17687796 (A/G), rs17740874 (T/C), rs17763104 (T/C), rs1880748 (T/C), rs1882478 (A/G), rs1944887 (T/C), rs2028629 (A/G), rs2143404 (A/G), rs2173530 (T/C), rs220806 (T/C), rs2235047 (A/C), rs2242071 (A/G), rs2257474 (T/C), rs2295583 (A/T), rs234629 (T/C), rs234630 (A/G), rs2436401 (A/G), rs258750 (T/C), rs2589487 (T/C), rs28364018 (T/G), rs28381774 (T/C), rs28381784 (A/G), rs2963155 (A/G), rs3133622 (T/G), rs32897 (T/C), rs33388 (A/T), rs3730168 (T/C), rs3735833 (T/G), rs3777747 (A/G), rs3786066 (T/C), rs3798346 (T/C), rs3822736 (A/G), rs389035 (T/C), rs3924144 (A/G), rs4148737 (T/C), rs4148749 (G/C), rs417968 (T/C), rs4458144 (T/C), rs4515335 (T/C), rs4728699 (A/G), rs4758040 (A/G), rs4812040 (A/G), rs4912650 (T/G), rs4957891 (T/C), rs5906392 (A/G), rs6026561 (T/C), rs6026565 (T/A), rs6026567 (A/G), rs6026593 (A/G), rs6092704 (T/G), rs6100260 (A/G), rs6128461 (T/C), rs6415328 (T/C), rs6610868 (T/C), rs6686061 (A/C), rs6730350 (T/G), rs6746197 (T/C), rs6963426 (T/C), rs7121326 (T/C), rs7721799 (A/G), rs7787082 (T/C), rs7799592 (A/C), rs796245 (T/C), rs809482 (A/C), rs8125112 (T/C), rs919196 (A/G), rs920750 (T/C), rs9332385 (A/G), rs930473 (T/G), rs9324921 (A/C), rs9348979 (A/G), rs9571939 (A/C), and rs9892359 (T/C).

## Patentansprüche

1. Verbindung SSR-125543 oder ein pharmazeutisch annehmbares Salz davon zur Verwendung in einem Verfahren zur Behandlung eines Zustandes, **gekennzeichnet durch**, verursacht oder begleitet von CRH-Überproduktion oder Überaktivität, umfassend Verabreichen einer effektiven Menge von SSR-125543 oder eines pharmazeutisch annehmbaren Salzes davon an ein Individuum, das solch eine Behandlung benötigt, wobei von dem Individuum vorhergesagt wurde, dass es auf eine Behandlung mit SSR-125543 oder einem pharmazeutisch annehmbaren Salz davon anspricht oder eine erhöhte Wahrscheinlichkeit aufweist, darauf anzusprechen, wobei die Behandlungsantwort durch Nachweisen des Vorhandenseins oder der Abwesenheit eines oder mehrerer Polymorphismus-Genotypen in einer biologischen Probe aus dem Individuum vorhergesagt wurde, wobei der eine oder die mehreren Polymorphismus-Genotypen umfassen:
mindestens einen Polymorphismus-Genotyp, ausgewählt aus der Gruppe bestehend aus rs34169260 (A/G), rs796287 (A/C), rs56149945 (A/G), rs6190 (T/C), rs7179092 (T/C), rs7614867 (A/G), rs920640 (T/C), rs7167722 (T/C), rs920638 (T/C), rs7165629 (T/C), rs80049044 (T/A), rs16941058 (A/G), rs112015971 (A/G), rs10894873 (T/C), rs117455294 (T/G), rs1170303 (T/C), rs16940681 (C/G), rs968519 (T/C), rs28381866 (T/C), rs79320848 (T/G), rs114653646 (T/G), rs2589496 (T/C), rs10482650 (A/G), rs17614642 (A/G), rs73200317 (T/C), rs1380146 (T/A), rs735164 (T/C), rs730976 (T/G), rs55934524 (T/G), rs4570614 (A/G), rs4458044 (C/G), rs77850169 (A/G), rs35339359 (A/G), rs34800935 (T/C), rs72945439 (T/C), rs113959523 (A/G), rs116798177 (A)/G), rs11247577 (T/G), rs75869266 (T/C), rs74372553 (T/C), rs11691508 (A/G), rs6493965 (A/G), rs4869476 (T/C), rs3730170 (T/C), rs2145288 (A/C), rs2935752 (A/C), rs146512400 (A/G), rs62057097 (T/C), rs115061314 (T/C), rs34113594 (T/G) rs61751173 (A/G), rs74338736 (A/C), rs10851726 (T/C), rs4610906 (T/C), rs59485211 (T/C), rs7060015 (T/G), rs75710780 (T/G), rs6520908 (T/C), rs487011 (T/G), rs1383699 (A/C), rs67516871 (A/G), rs114106519 (T/C), rs7220091 (A/G), rs12489026 (A/G), rs876270 (T/C), rs4968161 (T/C), rs62056907 (A/G), rs2235013 (T/C), rs16878812 (A/G), rs6549407 (A/G), rs28381848 (A/G)), rs79723704 (A/C), rs72814052 (A/G), rs10152908 (T/C), rs172769 (A/C), rs78596668 (T/C), rs73307922 (T/C), rs3842 (A/G) rs7210584 (A/C), rs62402121 (T/C), rs55709291 (A/G), rs72747088 (A/G), rs929610 (G/C), rs6766242 (T/C), rs1468552 (G/C), rs78838114 (T/C), rs62489862 (T/C), rs894342 (A/G), rs58882373 (T/C), rs3811939 (A/G), rs6984688 (T/G), rs1018160 (T/C) rs76602912 (A/G), rs80067508 (A/G), rs74888440 (T/C), rs12481583 (T/C), rs66794218 (A/G), rs16946701 (A/G), rs75726724 (A/G), rs67959715 (T/A), rs11871392 (T/G), rs2044070 (A/G), rs77612799 (T/C), rs6743702 (T/C), rs616870 (T/C), rs79590198 (A/G), rs75715199 (A/G), rs13087555 (T/C), rs4869618 (T/C), rs117397046 (A/G), rs8042817 (A/G), rs2258097 (T/C), rs2260882 (C/G), rs532996 (A/G), rs11747040 (T/C), rs10034039 (T/G), rs116909369 (A/G), rs79134986 (A/G), rs117615688 (T/C), rs8032253 (T/C), rs12818653 (T/A)), rs4587884 (A/C), rs77122853 (T/C), rs117615061 (T/C), rs74682905 (A/G), rs2257468 (T/C), rs2032582 (T/G), rs2235015 (T/G), rs2729794 (T/C), rs77549514 (A/G), rs74790420 (A/C), rs73129579 (T/C), rs12913346 (A/C), rs117560908 (T/C), rs72747091 (A/G), rs2935751 (A/G), rs4331446 (A/G), rs7523266 (T/C), rs7648662 (T/C), rs117034065 (A/G), rs4836256 (T/C), rs80238698 (T/C)), rs3730173 (T/C), rs11687884 (T/C), rs72693005 (T/C), rs2589476 (T/C), rs9813396 (T/C), rs10482667 (A/G), rs72784444 (A/G)), rs75074511 (T/C), rs7951003 (A/G), rs79584784 (A/G), rs2214102 (T/C), rs28811003 (A/G), rs6100261 (A/T), rs77152456 (A/G), rs66624622 (T/G), rs140302965 (A/G), rs11653269 (T/C), rs74405057 (A/G), rs7121 (A/G), rs16977818 (A/C), rs12490095 (T/C), RS118003903 (A/G), rs62377761 (T/C), P1_M_061510_6_34_M (-/CACTTACCTTCTTTGTGCCACAGTTTCCCTATCTAAAACACAAGGTTATCAGTTATCAACATCTCTTGGGATTGTGAGGACTAAAGTAATGCACATAAAG), rs375115639 (-/AAATTACCCTGTTAGGTTTCAATGAAACACCTTTTCTCTTGTAACAAACATCTCCTCCAAGCTAGAATTTCAAAACAG), rs1002204 (A/C), rs10062367 (A/G), rs10482642 (A/G), rs10482658 (A/G), rs1053989 (A/C), rs10851628 (T/C), rs10947562 (T/C), rs11069612 (A/G), rs11071351 (T/C), rs11091175 (A/G), rs11638450 (T/C), rs11715827 (T/G), rs11745958 (T/C), rs11834041 (A/G), rs1202180 (T/C), rs12054781 (A/G), rs12539395 (A/G), rs12720066 (T/G), rs1279754 (A/C), rs12872047 (T/C), rs12876742 (A/C), rs12917505 (A/G), rs13066950 (T/G), rs13229143 (C/G), rs1383707 (T/C), rs1441824 (T/C), rs1652311 (A/G), rs17064 (T/A), rs17100236 (A/G), rs17149699 (A/G), rs1724386 (A/G), rs17250255 (A/G), rs17327624 (T/G), rs17616338 (A/G), rs17687796 (A/G), rs17740874 (T/C), rs17763104 (T/C), rs1880748 (T/C), rs1882478 (A/G), rs1944887 (T/C), rs2028629 (A/G), rs2143404 (A/G), rs2173530 (T/C), rs220806 (T/C), rs2235047 (A/C), rs2242071 (A/G), rs2257474 (T/C), rs2295583 (A/T), rs234629 (T/C), rs234630 (A/G), rs2436401 (A/G), rs258750 (T/C), rs2589487 (T/C), rs28364018 (T/G), rs28381774 (T/C), rs28381784 (A/G), rs2963155 (A/G), rs3133622 (T/G), rs32897 (T/C), rs33388 (A/T), rs3730168 (T/C), rs3735833 (T/G), rs3777747 (A/G), rs3786066 (T/C), rs3798346 (T/C), rs3822736 (AG), rs389035 (T/C), rs3924144 (A/G), rs4148737 (T/C), rs4148749 (G/C), rs417968 (T/C), rs4458144 (T/C), rs4515335 (T/C), rs4728699 (A/G), rs4758040 (A/G), rs4812040 (A/G), rs4912650 (T/G), rs4957891 (T/C), rs5906392 (A/G), rs6026561 (T/C), rs6026565 (T/A), rs6026567 (A/G), rs6026593 (A/G), rs6092704 (T/G), rs6100260 (A/G), rs6128461 (T/C), rs6415328 (T/C), rs6610868 (T/C), rs6686061 (A/C), rs6730350 (T/G), rs6746197 (T/C), rs6963426 (T/C), rs7121326 (T/C), rs7721799 (A/G), rs7787082 (T/C), rs7799592 (A/C), rs796245 (T/C), rs809482 (A/C), rs8125112 (T/C), rs919196 (A/G), rs920750 (T/C), rs9332385 (A/G), rs930473 (T/G), rs9324921 (A/C), rs9348979 (A/G), rs9571939 (A/C) und rs9892359 (T/C).

2. Verbindung nach Anspruch 1 zur Verwendung nach Anspruch 1, wobei der Vorhersageschritt umfasst:
(a) Bestimmen, ob das Individuum auf die Behandlung mit SSR-125543 oder einem pharmazeutisch annehmbaren Salz davon ansprechen wird oder eine erhöhte Wahrscheinlichkeit aufweist, darauf anzusprechen; und/oder
(b) Bestimmen, ob das Individuum nicht auf die Behandlung mit SSR-125543 oder einem pharmazeutisch annehmbaren Salz davon ansprechen wird oder eine verringerte Wahrscheinlichkeit aufweist, darauf anzusprechen.

3. Verbindung nach Anspruch 1 zur Verwendung nach Anspruch 2, wobei der Bestimmungsschritt ein oder mehrere statistische Analyseverfahren umfasst, ausgewählt aus der Gruppe bestehend aus künstlichem neuronalen Netz-Lernen, Entscheidungsbaum-Lernen, Entscheidungsbaumwald-Lernen, linearer Diskriminanzanalyse, nichtlinearer Diskriminanzanalyse, genetischer Expressionsprogrammierung, Relevanzvektormaschinen, linearen Modellen, generalisierten linearen Modellen, generalisierten Schätzungsgleichungen, generalisierten linearen Mischmodellen, dem elastischen Netz, dem Lasso Support Vector Machine-Lernen, Bayes'schem Netz-Lernen, probabilistischem neuronalen Netz-Lernen, Clustering und Regressionsanalyse, gegebenenfalls wobei das statistische Analyseverfahren computerimplementiert ist.

4. Verbindung nach Anspruch 1 zur Verwendung nach einem der Ansprüche 1 - 3, wobei der eine oder die mehreren Polymorphismus-Genotypen umfassen:
(a) mindestens zwei;
(b) mindestens vier;
(c) mindestens acht;
(d) mindestens sechzehn;
(e) mindestens zweiunddreißig; oder
(f) alle
der Polymorphismus-Genotypen wie in Anspruch 1 definiert.

5. Verbindung nach Anspruch 1 zur Verwendung nach einem der Ansprüche 1 - 3, wobei der eine oder die mehreren Polymorphismus-Genotypen umfassen:
(a) mindestens zwei Polymorphismus-Genotypen, ausgewählt aus den Kombinationen der in Tabelle 5 offenbarten Polymorphismus-Genotypen;
(b) mindestens vier Polymorphismus-Genotypen, ausgewählt aus den Kombinationen der in Tabelle 6 offenbarten Polymorphismus-Genotypen;
(c) mindestens acht Polymorphismus-Genotypen, ausgewählt aus den Kombinationen der in Tabelle 7 offenbarten Polymorphismus-Genotypen;
(d) alle in Tabelle 2 offenbarten Polymorphismus-Genotypen.

6. Verbindung nach Anspruch 1 zur Verwendung nach einem der Ansprüche 1 bis 5, wobei die Behandlungsantwort auf die Behandlung mit SSR-125543 oder einem pharmazeutisch annehmbaren Salz davon eine klinische Antwort ist.

7. Verbindung nach Anspruch 1 zur Verwendung nach einem der Ansprüche 1 bis 6, wobei das Individuum depressive Symptome, Angstsymptome oder sowohl depressive Symptome als auch Angstsymptome oder eine Schlafstörung aufweist; und/oder
wobei die Behandlung eine Behandlung von depressiven Symptomen, Angstsymptomen oder sowohl depressiven Symptomen als auch Angstsymptomen oder einer Schlafstörung ist.

8. Verbindung nach Anspruch 1 zur Verwendung nach Anspruch 7, wobei die Behandlungsantwort auf die Behandlung mit SSR-125543 oder einem pharmazeutisch annehmbaren Salz davon eine klinische Antwort ist und wobei die klinische Antwort eine Prävention, Veränderung, Linderung oder vollständige Remission von depressiven Symptomen und/oder Angstsymptomen oder einer Schlafstörung ist.

9. Verbindung nach Anspruch 1 zur Verwendung nach Anspruch 8, wobei die klinische Antwort eine Prävention, Veränderung, Linderung oder vollständige Remission von depressiven Symptomen und/oder Angstsymptomen ist, bestimmt unter Verwendung einer Skala ausgewählt aus der Gruppe bestehend aus HAM-D, BDI, MADRS, GDS, ZSRDS, HAM-A und STAI.

10. Verbindung nach Anspruch 1 zur Verwendung nach einem der Ansprüche 1 - 9, wobei die biologische Probe eine bukkale oder eine Blutprobe ist.

11. Verbindung nach Anspruch 1 zur Verwendung nach einem der Ansprüche 1 - 10, wobei Nachweisen die Verwendung eines oder mehrerer Polynukleotide umfasst, die in der Lage sind, spezifisch an mindestens eine Nukleinsäure zu hybridisieren, umfassend den einen oder die mehreren Polymorphismus-Genotypen.

12. Verbindung nach Anspruch 1 zur Verwendung nach einem der Ansprüche 1 - 11, wobei nachdem dem Individuum SSR-125543 oder ein pharmazeutisch annehmbares Salz davon verabreicht wurde, weiterhin umfassend Vergleichen der Vorhersage, dass das Individuum auf eine Behandlung mit SSR-125543 oder einem pharmazeutisch annehmbaren Salz davon ansprechen wird mit der Behandlungsantwort des Individuums auf die Verabreichung von SSR-125543 oder einem pharmazeutisch annehmbaren Salz davon.

13. Verbindung nach Anspruch 1 zur Verwendung nach einem der Ansprüche 1 - 12, wobei die Vorhersage, dass das Individuum auf eine Behandlung mit SSR-125543 oder einem pharmazeutisch annehmbaren Salz davon ansprechen wird, eine Empfindlichkeit von mehr als 50% und eine Spezifität von mehr als 50% aufweist.

14. Verfahren zur Vorhersage der Wahrscheinlichkeit, dass ein bestimmtes Individuum auf eine Behandlung mit SSR-125543 oder einem pharmazeutisch annehmbaren Salz davon ansprechen wird oder nicht, umfassend:
Nachweisen des Vorhandenseins oder der Abwesenheit von einem oder mehreren Polymorphismus-Genotypen in einer biologischen Probe aus dem Individuum, wobei der eine oder die mehreren Polymorphismus-Genotypen umfassen:
mindestens einen Polymorphismus-Genotypen ausgewählt aus der Gruppe bestehend aus rs34169260 (A/G), rs796287 (A/C), rs56149945 (A/G), rs6190 (T/C), rs7179092 (T/C), rs7614867 (A/G), rs920640 (T/C), rs7167722 (T/C), rs920638 (T/C), rs7165629 (T/C), rs80049044 (T/A), rs16941058 (A/G), rs112015971 (A/G), rs10894873 (T/C), rs117455294 (T/G), rs1170303 (T/C), rs16940681 (C/G), rs968519 (T/C), rs28381866 (T/C), rs79320848 (T/G), rs114653646 (T/G), rs2589496 (T/C), rs10482650 (A/G), rs17614642 (A/G), rs73200317 (T/C), rs1380146 (T/A), rs735164 (T/C), rs730976 (T/G), rs55934524 (T/G), rs4570614 (A/G), rs4458044 (C/G), rs77850169 (A/G), rs35339359 (A/G), rs34800935 (T/C), rs72945439 (T/C), rs113959523 (A/G), rs116798177 (A)/G), rs11247577 (T/G), rs75869266 (T/C), rs74372553 (T/C), rs11691508 (A/G), rs6493965 (A/G), rs4869476 (T/C), rs3730170 (T/C), rs2145288 (A/C), rs2935752 (A/C), rs146512400 (A/G), rs62057097 (T/C), rs115061314 (T/C), rs34113594 (T/G) rs61751173 (A/G), rs74338736 (A/C), rs10851726 (T/C), rs4610906 (T/C), rs59485211 (T/C), rs7060015 (T/G), rs75710780 (T/G), rs6520908 (T/C), rs487011 (T/G), rs1383699 (A/C), rs67516871 (A/G), rs114106519 (T/C), rs7220091 (A/G), rs12489026 (A/G), rs876270 (T/C), rs4968161 (T/C), rs62056907 (A/G), rs2235013 (T/C), rs16878812 (A/G), rs6549407 (A/G), rs28381848 (A/G)), rs79723704 (A/C), rs72814052 (A/G), rs10152908 (T/C), rs172769 (A/C), rs78596668 (T/C), rs73307922 (T/C), rs3842 (A/G) rs7210584 (A/C), rs62402121 (T/C), rs55709291 (A/G), rs72747088 (A/G), rs929610 (G/C), rs6766242 (T/C), rs1468552 (G/C), rs78838114 (T/C), rs62489862 (T/C), rs894342 (A/G), rs58882373 (T/C), rs3811939 (A/G), rs6984688 (T/G), rs1018160 (T/C) rs76602912 (A/G), rs80067508 (A/G), rs74888440 (T/C), rs12481583 (T/C), rs66794218 (A/G), rs16946701 (A/G), rs75726724 (A/G), rs67959715 (T/A), rs11871392 (T/G), rs2044070 (A/G), rs77612799 (T/C), rs6743702 (T/C), rs616870 (T/C), rs79590198 (A/G), rs75715199 (A/G), rs13087555 (T/C), rs4869618 (T/C), rs117397046 (A/G), rs8042817 (A/G), rs2258097 (T/C), rs2260882 (C/G), rs532996 (A/G), rs11747040 (T/C), rs10034039 (T/G), rs116909369 (A/G), rs79134986 (A/G), rs117615688 (T/C), rs8032253 (T/C), rs12818653 (T/A)), rs4587884 (A/C), rs77122853 (T/C), rs117615061 (T/C), rs74682905 (A/G), rs2257468 (T/C), rs2032582 (T/G), rs2235015 (T/G), rs2729794 (T/C), rs77549514 (A/G), rs74790420 (A/C), rs73129579 (T/C), rs12913346 (A/C), rs117560908 (T/C), rs72747091 (A/G), rs2935751 (A/G), rs4331446 (A/G), rs7523266 (T/C), rs7648662 (T/C), rs117034065 (A/G), rs4836256 (T/C), rs80238698 (T/C)), rs3730173 (T/C), rs11687884 (T/C), rs72693005 (T/C), rs2589476 (T/C), rs9813396 (T/C), rs10482667 (A/G), rs72784444 (A/G)), rs75074511 (T/C), rs7951003 (A/G), rs79584784 (A/G), rs2214102 (T/C), rs28811003 (A/G), rs6100261 (A/T), rs77152456 (A/G), rs66624622 (T/G), rs140302965 (A/G), rs11653269 (T/C), rs74405057 (A/G), rs7121 (A/G), rs16977818 (A/C), rs12490095 (T/C), RS118003903 (A/G), rs62377761 (T/C),P1_M_061510_6_34_M (-/CACTTACCTTCTTTGTGCCACAGTTTCCCTATCTAAAACACAAGGTTATCAGTTATCAACATCTCTTGGGATTGTGAGGACTAAAGTAATGCACATAAAG), rs375115639 (-/AAATTACCCTGTTAGGTTTCAATGAAACACCTTTTCTCTTGTAACAAACATCTCCTCCAAGCTAGAATTTCAAAACAG), rs1002204 (A/C), rs10062367 (A/G), rs10482642 (A/G), rs10482658 (A/G), rs1053989 (A/C), rs10851628 (T/C), rs10947562 (T/C), rs11069612 (A/G), rs11071351 (T/C), rs11091175 (A/G), rs11638450 (T/C), rs11715827 (T/G), rs11745958 (T/C), rs11834041 (A/G), rs1202180 (T/C), rs12054781 (A/G), rs12539395 (A/G), rs12720066 (T/G), rs1279754 (A/C), rs12872047 (T/C), rs12876742 (A/C), rs12917505 (A/G), rs13066950 (T/G), rs13229143 (C/G), rs1383707 (T/C), rs1441824 (T/C), rs1652311 (A/G), rs17064 (T/A), rs17100236 (A/G), rs17149699 (A/G), rs1724386 (A/G), rs17250255 (A/G), rs17327624 (T/G), rs17616338 (A/G), rs17687796 (A/G), rs17740874 (T/C), rs17763104 (T/C), rs1880748 (T/C), rs1882478 (A/G), rs1944887 (T/C), rs2028629 (A/G), rs2143404 (A/G), rs2173530 (T/C), rs220806 (T/C), rs2235047 (A/C), rs2242071 (A/G), rs2257474 (T/C), rs2295583 (A/T), rs234629 (T/C), rs234630 (A/G), rs2436401 (A/G), rs258750 (T/C), rs2589487 (T/C), rs28364018 (T/G), rs28381774 (T/C), rs28381784 (A/G), rs2963155 (A/G), rs3133622 (T/G), rs32897 (T/C), rs33388 (A/T), rs3730168 (T/C), rs3735833 (T/G), rs3777747 (A/G), rs3786066 (T/C), rs3798346 (T/C), rs3822736 (AG), rs389035 (T/C), rs3924144 (A/G), rs4148737 (T/C), rs4148749 (G/C), rs417968 (T/C), rs4458144 (T/C), rs4515335 (T/C), rs4728699 (A/G), rs4758040 (A/G), rs4812040 (A/G), rs4912650 (T/G), rs4957891 (T/C), rs5906392 (A/G), rs6026561 (T/C), rs6026565 (T/A), rs6026567 (A/G), rs6026593 (A/G), rs6092704 (T/G), rs6100260 (A/G), rs6128461 (T/C), rs6415328 (T/C), rs6610868 (T/C), rs6686061 (A/C), rs6730350 (T/G), rs6746197 (T/C), rs6963426 (T/C), rs7121326 (T/C), rs7721799 (A/G), rs7787082 (T/C), rs7799592 (A/C), rs796245 (T/C), rs809482 (A/C), rs8125112 (T/C), rs919196 (A/G), rs920750 (T/C), rs9332385 (A/G), rs930473 (T/G), rs9324921 (A/C), rs9348979 (A/G), rs9571939 (A/C) und rs9892359 (T/C).

## Revendications

1. Composé SSR-125543 ou sel pharmaceutiquement acceptable de celui-ci pour une utilisation dans un procédé de traitement d'une affection **caractérisée**, causée ou accompagnée par la surproduction ou la suractivité de CRH, comprenant l'administration d'une quantité efficace de SSR-125543 ou d'un sel pharmaceutiquement acceptable de celui-ci à un sujet qui a besoin d'un tel traitement, où le sujet s'est vu prédire qu'il réagirait, ou qu'il a une probabilité accrue de réagir, à un traitement avec SSR-125543 ou un sel pharmaceutiquement acceptable de celui-ci, où la réponse au traitement a été prédite par détection de la présence ou de l'absence d'un ou plusieurs génotypes à polymorphisme dans un échantillon biologique provenant du sujet, où les un ou plusieurs génotypes à polymorphisme comprennent :
au moins un génotype à polymorphisme choisi dans le groupe consistant en rs34169260 (A/G), rs796287 (A/C), rs56149945 (A/G), rs6190 (T/C), rs7179092 (T/C), rs7614867 (A/G), rs920640 (T/C), rs7167722 (T/C), rs920638 (T/C), rs7165629 (T/C), rs80049044 (T/A), rs16941058 (A/G), rs112015971 (A/G), rs10894873 (T/C), rs117455294 (T/G), rs1170303 (T/C), rs16940681 (C/G), rs968519 (T/C), rs28381866 (T/C), rs79320848 (T/G), rs114653646 (T/G), rs2589496 (T/C), rs10482650 (A/G), rs17614642 (A/G), rs73200317 (T/C), rs1380146 (T/A), rs735164 (T/C), rs730976 (T/G), rs55934524 (T/G), rs4570614 (A/G), rs4458044 (C/G), rs77850169 (A/G), rs35339359 (A/G), rs34800935 (T/C), rs72945439 (T/C), rs113959523 (A/G), rs116798177 (A/G), rs11247577 (T/G), rs75869266 (T/C), rs74372553 (T/C), rs11691508 (A/G), rs6493965 (A/G), rs4869476 (T/C), rs3730170 (T/C), rs2145288 (A/C), rs2935752 (A/C), rs146512400 (A/G), rs62057097 (T/C), rs115061314 (T/C), rs34113594 (T/G), rs61751173 (A/G), rs74338736 (A/C), rs10851726 (T/C), rs4610906 (T/C), rs59485211 (T/C), rs7060015 (T/G), rs75710780 (T/G), rs6520908 (T/C), rs487011 (T/G), rs1383699 (A/C), rs67516871 (A/G), rs114106519 (T/C), rs7220091 (A/G), rs12489026 (A/G), rs876270 (T/C), rs4968161 (T/C), rs62056907 (A/G), rs2235013 (T/C), rs16878812 (A/G), rs6549407 (A/G), rs28381848 (A/G), rs79723704 (A/C), rs72814052 (A/G), rs10152908 (T/C), rs172769 (A/C), rs78596668 (T/C), rs73307922 (T/C), rs3842 (A/G), rs7210584 (A/C), rs62402121 (T/C), rs55709291 (A/G), rs72747088 (A/G), rs929610 (G/C), rs6766242 (T/C), rs1468552 (G/C), rs78838114 (T/C), rs62489862 (T/C), rs894342 (A/G), rs58882373 (T/C), rs3811939 (A/G), rs6984688 (T/G), rs1018160 (T/C), rs76602912 (A/G), rs80067508 (A/G), rs74888440 (T/C), rs12481583 (T/C), rs66794218 (A/G), rs16946701 (A/G), rs75726724 (A/G), rs67959715 (T/A), rs11871392 (T/G), rs2044070 (A/G), rs77612799 (T/C), rs6743702 (T/C), rs616870 (T/C), rs79590198 (A/G), rs75715199 (A/G), rs13087555 (T/C), rs4869618 (T/C), rs117397046 (A/G), rs8042817 (A/G), rs2258097 (T/C), rs2260882 (C/G), rs532996 (A/G), rs11747040 (T/C), rs10034039 (T/G), rs116909369 (A/G), rs79134986 (A/G), rs117615688 (T/C), rs8032253 (T/C), rs12818653 (T/A), rs4587884 (A/C), rs77122853 (T/C), rs117615061 (T/C), rs74682905 (A/G), rs2257468 (T/C), rs2032582 (T/G), rs2235015 (T/G), rs2729794 (T/C), rs77549514 (A/G), rs74790420 (A/C), rs73129579 (T/C), rs12913346 (A/C), rs117560908 (T/C), rs72747091 (A/G), rs2935751 (A/G), rs4331446 (A/G), rs7523266 (T/C), rs7648662 (T/C), rs117034065 (A/G), rs4836256 (T/C), rs80238698 (T/C), rs3730173 (T/C), rs11687884 (T/C), rs72693005 (T/C), rs2589476 (T/C), rs9813396 (T/C), rs10482667 (A/G), rs72784444 (A/G), rs75074511 (T/C), rs7951003 (A/G), rs79584784 (A/G), rs2214102 (T/C), rs28811003 (A/G), rs6100261 (A/T), rs77152456 (A/G), rs66624622 (T/G), rs140302965 (A/G), rs11653269 (T/C), rs74405057 (A/G), rs7121 (A/G), rs16977818 (A/C), rs12490095 (T/C), rs118003903 (A/G), rs62377761 (T/C), P1_M_061510_6_34_M (-/CACTTACCTTCTTTGTGCCACAGTTTCCCTATCTAAAACACAAGGTTATCAGTTATCAACATCTCTTGGGATTGTGAGGACTAAAGTAATGCACATAAAG), rs375115639 (-/AAATTACCCTGTTAGGTTTCAATGAAACACCTTTTCTCTTGTAACAAACATCTCC TCCAAGCTAGAATTTCAAAACAG), rs1002204 (A/C), rs10062367 (A/G), rs10482642 (A/G), rs10482658 (A/G), rs1053989 (A/C), rs10851628 (T/C), rs10947562 (T/C), rs11069612 (A/G), rs11071351 (T/C), rs11091175 (A/G), rs11638450 (T/C), rs11715827 (T/G), rs11745958 (T/C), rs11834041 (A/G), rs1202180 (T/C), rs12054781 (A/G), rs12539395 (A/G), rs12720066 (T/G), rs1279754 (A/C), rs12872047 (T/C), rs12876742 (A/C), rs12917505 (A/G), rs13066950 (T/G), rs13229143 (C/G), rs1383707 (T/C), rs1441824 (T/C), rs1652311 (A/G), rs17064 (T/A), rs17100236 (A/G), rs17149699 (A/G), rs1724386 (A/G), rs17250255 (A/G), rs17327624 (T/G), rs17616338 (A/G), rs17687796 (A/G), rs17740874 (T/C), rs17763104 (T/C), rs1880748 (T/C), rs1882478 (A/G), rs1944887 (T/C), rs2028629 (A/G), rs2143404 (A/G), rs2173530 (T/C), rs220806 (T/C), rs2235047 (A/C), rs2242071 (A/G), rs2257474 (T/C), rs2295583 (A/T), rs234629 (T/C), rs234630 (A/G), rs2436401 (A/G), rs258750 (T/C), rs2589487 (T/C), rs28364018 (T/G), rs28381774 (T/C), rs28381784 (A/G), rs2963155 (A/G), rs3133622 (T/G), rs32897 (T/C), rs33388 (A/T), rs3730168 (T/C), rs3735833 (T/G), rs3777747 (A/G), rs3786066 (T/C), rs3798346 (T/C), rs3822736 (A/G), rs389035 (T/C), rs3924144 (A/G), rs4148737 (T/C), rs4148749 (G/C), rs417968 (T/C), rs4458144 (T/C), rs4515335 (T/C), rs4728699 (A/G), rs4758040 (A/G), rs4812040 (A/G), rs4912650 (T/G), rs4957891 (T/C), rs5906392 (A/G), rs6026561 (T/C), rs6026565 (T/A), rs6026567 (A/G), rs6026593 (A/G), rs6092704 (T/G), rs6100260 (A/G), rs6128461 (T/C), rs6415328 (T/C), rs6610868 (T/C), rs6686061 (A/C), rs6730350 (T/G), rs6746197 (T/C), rs6963426 (T/C), rs7121326 (T/C), rs7721799 (A/G), rs7787082 (T/C), rs7799592 (A/C), rs796245 (T/C), rs809482 (A/C), rs8125112 (T/C), rs919196 (A/G), rs920750 (T/C), rs9332385 (A/G), rs930473 (T/G), rs9324921 (A/C), rs9348979 (A/G), rs9571939 (A/C) et rs9892359 (T/C).

2. Composé selon la revendication 1 pour l'utilisation selon la revendication 1, où l'étape de prédiction comprend :
(a) la détermination de ce que le sujet réagira, ou a une probabilité accrue de réagir au traitement avec SSR-125543 ou un sel pharmaceutiquement acceptable de celui-ci ; et/ou
(b) la détermination de ce que le sujet ne réagira pas ou a une probabilité réduite de réagir au traitement avec SSR-125543 ou un sel pharmaceutiquement acceptable de celui-ci.

3. Composé selon la revendication 1 pour l'utilisation selon la revendication 2, où l'étape de détermination comprend un ou plusieurs procédés d'analyse statistique choisis dans le groupe consistant en l'apprentissage à réseaux de neurones artificiels, l'apprentissage à arbres de décision, l'apprentissage à forêts d'arbres de décision, l'analyse discriminante linéaire, l'analyse discriminante non linéaire, la programmation d'expression génétique, les machines à vecteurs de pertinence, les modèles linaires, les modèles linéaires généralisés, les équations d'estimation généralisées, les modèles mixtes linéaires généralisés, le réseau élastique, l'apprentissage à machines à vecteurs de support lasso, l'apprentissage à réseaux bayésiens, l'apprentissage à réseaux de neurones probabilistes, le partitionnement de données et l'analyse de régression, éventuellement où le procédé d'analyse statistique est mis en oeuvre sur ordinateur.

4. Composé selon la revendication 1 pour l'utilisation selon l'une quelconque des revendications 1-3, où les un ou plusieurs génotypes à polymorphisme comprennent :
(a) au moins deux ;
(b) au moins quatre ;
(c) au moins huit ;
(d) au moins seize ;
(e) au moins trente-deux ; ou
(f) tous
les génotypes à polymorphisme tels que définis dans la revendication 1.

5. Composé selon la revendication 1 pour l'utilisation selon l'une quelconque des revendications 1-3, où les un ou plusieurs génotypes à polymorphisme comprennent :
(a) au moins deux génotypes à polymorphisme choisis parmi les combinaisons de génotypes à polymorphisme présentées dans le tableau 5 ;
(b) au moins quatre génotypes à polymorphisme choisis parmi les combinaisons de génotypes à polymorphisme présentées dans le tableau 6 ;
(c) au moins huit génotypes à polymorphisme choisis parmi les combinaisons de génotypes à polymorphisme présentées dans le tableau 7 ;
(d) tous les génotypes à polymorphisme présentés dans le tableau 2.

6. Composé selon la revendication 1 pour l'utilisation selon l'une quelconque des revendications 1 à 5, où la réponse au traitement à un traitement avec SSR-125543 ou un sel pharmaceutiquement acceptable de celui-ci est une réponse clinique.

7. Composé selon la revendication 1 pour l'utilisation selon l'une quelconque des revendications 1 à 6, où le sujet a des symptômes dépressifs, des symptômes d'anxiété ou des symptômes dépressifs et des symptômes d'anxiété ou un trouble du sommeil ; et/ou
où le traitement est un traitement de symptômes dépressifs, de symptômes d'anxiété ou de symptômes dépressifs et de symptômes d'anxiété ou d'un trouble du sommeil.

8. Composé selon la revendication 1 pour l'utilisation selon la revendication 7, où la réponse au traitement à un traitement avec SSR-125543 ou un sel pharmaceutiquement acceptable de celui-ci est une réponse clinique, et où la réponse clinique est une prévention, une altération, une atténuation ou une rémission complète de symptômes dépressifs et/ou de symptômes d'anxiété ou d'un trouble du sommeil.

9. Composé selon la revendication 1 pour l'utilisation selon la revendication 8, où la réponse clinique est une prévention, une altération, une atténuation ou une rémission complète de symptômes dépressifs et/ou de symptômes d'anxiété comme déterminé au moyen d'une échelle choisie dans le groupe consistant en HAM-D, BDI, MADRS, GDS, ZSRDS, HAM-A et STAI.

10. Composé selon la revendication 1 pour l'utilisation selon l'une quelconque des revendications 1-9, où l'échantillon biologique est un échantillon buccal ou sanguin.

11. Composé selon la revendication 1 pour l'utilisation selon l'une quelconque des revendications 1-10, où la détection comprend l'utilisation d'un ou plusieurs polynucléotides capables de s'hybrider spécifiquement à au moins un acide nucléique comprenant les un ou plusieurs génotypes à polymorphisme.

12. Composé selon la revendication 1 pour l'utilisation selon l'une quelconque des revendications 1-11, où après que le sujet s'est vu administrer SSR-125543 ou un sel pharmaceutiquement acceptable de celui-ci, comprenant en outre la comparaison de la prédiction que le sujet réagira à un traitement avec SSR-125543 ou un sel pharmaceutiquement acceptable de celui-ci avec la réponse au traitement du sujet à l'administration de SSR-125543 ou d'un sel pharmaceutiquement acceptable de celui-ci.

13. Composé selon la revendication 1 pour l'utilisation selon l'une quelconque des revendications 1-12, où la prédiction que le sujet réagira à un traitement avec SSR-125543 ou un sel pharmaceutiquement acceptable de celui-ci a une sensibilité supérieure à 50 % et une spécificité supérieure à 50 %.

14. Procédé de prédiction de la probabilité qu'un sujet donné réagira ou pas à un traitement avec SSR-125543 ou un sel pharmaceutiquement acceptable de celui-ci, comprenant
la détection de la présence ou de l'absence d'un ou plusieurs génotypes à polymorphisme dans un échantillon biologique provenant du sujet, où les un ou plusieurs génotypes à polymorphisme comprennent :
au moins un génotype à polymorphisme choisi dans le groupe consistant en rs34169260 (A/G), rs796287 (A/C), rs56149945 (A/G), rs6190 (T/C), rs7179092 (T/C), rs7614867 (A/G), rs920640 (T/C), rs7167722 (T/C), rs920638 (T/C), rs7165629 (T/C), rs80049044 (T/A), rs16941058 (A/G), rs112015971 (A/G), rs10894873 (T/C), rs117455294 (T/G), rs1170303 (T/C), rs16940681 (C/G), rs968519 (T/C), rs28381866 (T/C), rs79320848 (T/G), rs114653646 (T/G), rs2589496 (T/C), rs10482650 (A/G), rs17614642 (A/G), rs73200317 (T/C), rs1380146 (T/A), rs735164 (T/C), rs730976 (T/G), rs55934524 (T/G), rs4570614 (A/G), rs4458044 (C/G), rs77850169 (A/G), rs35339359 (A/G), rs34800935 (T/C), rs72945439 (T/C), rs113959523 (A/G), rs116798177 (A/G), rs11247577 (T/G), rs75869266 (T/C), rs74372553 (T/C), rs11691508 (A/G), rs6493965 (A/G), rs4869476 (T/C), rs3730170 (T/C), rs2145288 (A/C), rs2935752 (A/C), rs146512400 (A/G), rs62057097 (T/C), rs115061314 (T/C), rs34113594 (T/G), rs61751173 (A/G), rs74338736 (A/C), rs10851726 (T/C), rs4610906 (T/C), rs59485211 (T/C), rs7060015 (T/G), rs75710780 (T/G), rs6520908 (T/C), rs487011 (T/G), rs1383699 (A/C), rs67516871 (A/G), rs114106519 (T/C), rs7220091 (A/G), rs12489026 (A/G), rs876270 (T/C), rs4968161 (T/C), rs62056907 (A/G), rs2235013 (T/C), rs16878812 (A/G), rs6549407 (A/G), rs28381848 (A/G), rs79723704 (A/C), rs72814052 (A/G), rs10152908 (T/C), rs172769 (A/C), rs78596668 (T/C), rs73307922 (T/C), rs3842 (A/G), rs7210584 (A/C), rs62402121 (T/C), rs55709291 (A/G), rs72747088 (A/G), rs929610 (G/C), rs6766242 (T/C), rs1468552 (G/C), rs78838114 (T/C), rs62489862 (T/C), rs894342 (A/G), rs58882373 (T/C), rs3811939 (A/G), rs6984688 (T/G), rs1018160 (T/C), rs76602912 (A/G), rs80067508 (A/G), rs74888440 (T/C), rs12481583 (T/C), rs66794218 (A/G), rs16946701 (A/G), rs75726724 (A/G), rs67959715 (T/A), rs11871392 (T/G), rs2044070 (A/G), rs77612799 (T/C), rs6743702 (T/C), rs616870 (T/C), rs79590198 (A/G), rs75715199 (A/G), rs13087555 (T/C), rs4869618 (T/C), rs117397046 (A/G), rs8042817 (A/G), rs2258097 (T/C), rs2260882 (C/G), rs532996 (A/G), rs11747040 (T/C), rs10034039 (T/G), rs116909369 (A/G), rs79134986 (A/G), rs117615688 (T/C), rs8032253 (T/C), rs12818653 (T/A), rs4587884 (A/C), rs77122853 (T/C), rs117615061 (T/C), rs74682905 (A/G), rs2257468 (T/C), rs2032582 (T/G), rs2235015 (T/G), rs2729794 (T/C), rs77549514 (A/G), rs74790420 (A/C), rs73129579 (T/C), rs12913346 (A/C), rs117560908 (T/C), rs72747091 (A/G), rs2935751 (A/G), rs4331446 (A/G), rs7523266 (T/C), rs7648662 (T/C), rs117034065 (A/G), rs4836256 (T/C), rs80238698 (T/C), rs3730173 (T/C), rs11687884 (T/C), rs72693005 (T/C), rs2589476 (T/C), rs9813396 (T/C), rs10482667 (A/G), rs72784444 (A/G), rs75074511 (T/C), rs7951003 (A/G), rs79584784 (A/G), rs2214102 (T/C), rs28811003 (A/G), rs6100261 (A/T), rs77152456 (A/G), rs66624622 (T/G), rs140302965 (A/G), rs11653269 (T/C), rs74405057 (A/G), rs7121 (A/G), rs16977818 (A/C), rs12490095 (T/C), rs118003903 (A/G), rs62377761 (T/C), P1_M_061510_6_34_M (-/CACTTACCTTCTTTGTGCCACAGTTTCCCTATCTAAAACACAAGGTTATCAGTTATCAACATCTCTTGGGATTGTGAGGACTAAAGTAATGCACATAAAG), rs375115639 (-/AAATTACCCTGTTAGGTTTCAATGAAACACCTTTTCTCTTGTAACAAACATCTCC TCCAAGCTAGAATTTCAAAACAG), rs1002204 (A/C), rs10062367 (A/G), rs10482642 (A/G), rs10482658 (A/G), rs1053989 (A/C), rs10851628 (T/C), rs10947562 (T/C), rs11069612 (A/G), rs11071351 (T/C), rs11091175 (A/G), rs11638450 (T/C), rs11715827 (T/G), rs11745958 (T/C), rs11834041 (A/G), rs1202180 (T/C), rs12054781 (A/G), rs12539395 (A/G), rs12720066 (T/G), rs1279754 (A/C), rs12872047 (T/C), rs12876742 (A/C), rs12917505 (A/G), rs13066950 (T/G), rs13229143 (C/G), rs1383707 (T/C), rs1441824 (T/C), rs1652311 (A/G), rs17064 (T/A), rs17100236 (A/G), rs17149699 (A/G), rs1724386 (A/G), rs17250255 (A/G), rs17327624 (T/G), rs17616338 (A/G), rs17687796 (A/G), rs17740874 (T/C), rs17763104 (T/C), rs1880748 (T/C), rs1882478 (A/G), rs1944887 (T/C), rs2028629 (A/G), rs2143404 (A/G), rs2173530 (T/C), rs220806 (T/C), rs2235047 (A/C), rs2242071 (A/G), rs2257474 (T/C), rs2295583 (A/T), rs234629 (T/C), rs234630 (A/G), rs2436401 (A/G), rs258750 (T/C), rs2589487 (T/C), rs28364018 (T/G), rs28381774 (T/C), rs28381784 (A/G), rs2963155 (A/G), rs3133622 (T/G), rs32897 (T/C), rs33388 (A/T), rs3730168 (T/C), rs3735833 (T/G), rs3777747 (A/G), rs3786066 (T/C), rs3798346 (T/C), rs3822736 (A/G), rs389035 (T/C), rs3924144 (A/G), rs4148737 (T/C), rs4148749 (G/C), rs417968 (T/C), rs4458144 (T/C), rs4515335 (T/C), rs4728699 (A/G), rs4758040 (A/G), rs4812040 (A/G), rs4912650 (T/G), rs4957891 (T/C), rs5906392 (A/G), rs6026561 (T/C), rs6026565 (T/A), rs6026567 (A/G), rs6026593 (A/G), rs6092704 (T/G), rs6100260 (A/G), rs6128461 (T/C), rs6415328 (T/C), rs6610868 (T/C), rs6686061 (A/C), rs6730350 (T/G), rs6746197 (T/C), rs6963426 (T/C), rs7121326 (T/C), rs7721799 (A/G), rs7787082 (T/C), rs7799592 (A/C), rs796245 (T/C), rs809482 (A/C), rs8125112 (T/C), rs919196 (A/G), rs920750 (T/C), rs9332385 (A/G), rs930473 (T/G), rs9324921 (A/C), rs9348979 (A/G), rs9571939 (A/C) et rs9892359 (T/C).
